# EUROPEAN PATENT SPECIFICATION

(11) **EP 4 437 141 B1**
(45) Date of publication and mention of the grant of the patent: **11.11.2026**
(21) Application number: 22897865.6
(22) Date of filing: 24.11.2022
(51) Int. Cl.: C12Q 1/6886, C12Q 1/6883, C12Q 1/6869, C12Q 1/6809, G16B 20/10, G16H 50/30, C12Q 1/6806

(54) **MOLECULAR ANALYSES USING LONG CELL-FREE DNA MOLECULES FOR DISEASE CLASSIFICATION**
MOLEKULARE ANALYSEN UNTER VERWENDUNG VON LANGEN ZELLFREIEN DNA-MOLEKÜLEN ZUR KRANKHEITSKLASSIFIZIERUNG
ANALYSES MOLÉCULAIRES UTILISANT DE LONGUES MOLÉCULES D'ADN ACELLULAIRES POUR LA CLASSIFICATION DES MALADIES

(30) Priority: 24.11.2021 US 202163283190 P; 03.12.2021 US 202163285683 P
(43) Date of publication of application: 02.10.2024
(73) Proprietor: Centre for Novostics, Hong Kong (HK)
(72) Inventor: LO, Yuk-Ming Dennis, Hong Kong Science Park Shatin, New Territories Hong Kong (HK); CHIU, Rossa Wai Kwun, Hong Kong Science Park Shatin, New Territories Hong Kong (HK); CHAN, Kwan Chee, Hong Kong Science Park Shatin, New Territories Hong Kong (HK); JIANG, Peiyong, Hong Kong Science Park Shatin, New Territories Hong Kong (HK); CHOY, Lok Yee, Hong Kong Science Park Shatin, New Territories Hong Kong (HK)
(74) Representative: J A Kemp LLP
(86) International application number: PCT/CN2022/133878
(87) International publication number: WO 2023/093782

(56) References cited:
- WO-A1-2017/212428
- WO-A1-2018/099418
- WO-A1-2020/125709
- WO-A1-2020/125709
- WO-A1-2020/132148
- WO-A1-2021/139716
- WO-A1-2021/155831
- WO-A2-2020/154682
- PEIYONG JIANG ET AL: "Lengthening and shortening of plasma DNA in hepatocellular carcinoma patients", PROCEEDINGS OF THE NATIONAL ACADEMY OF SCIENCES (PNAS), vol. 112, no. 11, 2 February 2015 (2015-02-02), pages E1317 - E1325, XP055223840, ISSN: 0027-8424, DOI: 10.1073/pnas.1500076112
- SNYDER MATTHEW W ET AL: "Cell-free DNA Comprises an In Vivo Nucleosome Footprint that Informs Its Tissues-Of-Origin", CELL, ELSEVIER, AMSTERDAM NL, vol. 164, no. 1, 14 January 2016 (2016-01-14), pages 57 - 68, XP029385484, ISSN: 0092-8674, DOI: 10.1016/J.CELL.2015.11.050
- SHI JIPING ET AL: "Size profile of cell-free DNA: A beacon guiding the practice and innovation of clinical testing", vol. 10, no. 11, 26 March 2020 (2020-03-26), AU, pages 4737 - 4748, XP093017678, ISSN: 1838-7640, Retrieved from the Internet <URL:https://www.ncbi.nlm.nih.gov/pmc/articles/PMC7163439/pdf/thnov10p4737.pdf> DOI: 10.7150/thno.42565
- UDOMRUK SASIMOL ET AL: "Size distribution of cell-free DNA in oncology", CRITICAL REVIEWS IN ONCOLOGY/HEMATOLOGY, ELSEVIER, AMSTERDAM, NL, vol. 166, 28 August 2021 (2021-08-28), XP086812493, ISSN: 1040-8428, [retrieved on 20210828], DOI: 10.1016/J.CRITREVONC.2021.103455

## Description

### CROSS-REFERENCES TO RELATED APPLICATIONS

This application claims priority to U.S. Provisional Patent Application No. 63/283,190, entitled "Molecular Analyses Using Long Cell-Free DNA Molecules For Disease Classification," filed on November 24, 2021 and U.S. Provisional Patent Application No. 63/285,683, entitled "Molecular Analyses Using Long Cell-Free DNA Molecules For Disease Classification," filed on December 3, 2021

### BACKGROUND

Many previous research studies focused on genetic/epigenetic information of circulating cell-free DNA molecules below 400 bp in plasma of patients with cancers (Jiang et al. Proc Natl Acad Sci USA. 2015;112:E1317-25; Mouliere et al. PLoS One. 2011;6:e23418; Mouliere et al. Sci Transl Med. 2018; 10:eaat4921; Underhill et al. PLoS Genet. 2016; 12:e1006162; Chan et al. Proc Natl Acad Sci USA. 2013;110:18761-8). The diagnostic and commercial values regarding long DNA molecules, for example, but not limited to, ≥ 500 bp, ≥ 600 bp, ≥ 1 kb, ≥ 2 kb, ≥ 3 kb, ≥ 4 kb, ≥ 5 kb, ≥ 10 kb or other combinations, in patients with cancers or many other diseases such as autoimmune diseases remain unexplored.

Jahr et al. reported that high-molecular-weight DNA fragments were present in plasma for 3 out of 6 samples with cancers with the use of polyacrylamide gel electrophoresis (PAGE) (Jahr et al. Cancer Res. 2001;61:1659-65). Such high-molecular-weight DNA molecules being present in only 50% of the tested cancer samples of this study seem to suggest such long cell-free DNA molecules may not be consistently detectable among cancer patients. Even for the 3 samples showing the presence of high-molecular weight cell-free DNA molecules, it is not known how abundant these DNA molecules were. Furthermore, this study did not provide comparison against samples from individuals without cancer. These several factors do not seem to suggest long cell-free DNA molecules as a practical biomarker for cancer detection. Moreover, the polyacrylamide gel electrophoresis (PAGE) based analysis does not allow one to decode the actual genetic/epigenetic information in a sequence.

The prevalent genomic analytical tool includes short-read massively parallel sequencing. The short-read massively parallel sequencing is designed to analyze short DNA molecules, typically < 800 bp, or in fact, preferably < 600 bp. Compounded by literature such as Jahr et al. showing low detectability of long cell-free DNA molecules, the analysis of long cell-free DNA molecules remain unexplored. WO 2020/154682 discloses a method of determining a tissue of origin (TOO) of a cancer, comprising analysing the degree of methylation using sequencing in a sample comprising a plurality of cfDNA molecules. WO 2020/125709 discloses a method of classifying a level of pathology in a biological sample comprising cell-free DNA.

### SUMMARY

Techniques described herein can use various characteristics of cell-free DNA molecules to determine a property of a biological sample or a subject. Such characteristics can include size (e.g., where characteristic is of long cell-free DNA molecules), methylation, and end motifs. For example, some methods, apparatuses, and systems described herein can include using long cell-free DNA fragments to analyze a biological sample.

Various methods can include determining disease classification and/or predicting tissue of origin based on one or more characteristics of cell-free DNA molecules (e.g., long cell-free DNA molecules) in a biological sample (e.g., a plasma sample) of the subject. In some instances, the characteristics includes determining an amount of cell-free DNA molecules (e.g., within a size range with an upper-bound of 1000 bases), and the disease classification can be based on the determined amount. As further examples, the characteristics can also include identifying a methylation pattern of a cell-free DNA molecule, and then comparing the methylation pattern of the cell-free DNA molecule to a reference pattern to predict the tissue of origin. An origin of a variant on cell-free DNA molecule can be determined in this manner. As yet further examples, the characteristics can also include relative frequencies of sequences having one or more end motifs, at which the relative frequencies (e.g., a vector of relative frequencies) can be compared with reference frequencies to determine a disease classification.

In some instances, a methylation-pattern analysis can include using a trained machine-learning model. The methylation-pattern analysis can provide individual properties of cell-free DNA molecules, e.g., a methylation level determines from a set of sites on the molecule, such as a percentage of sites that are methylated. Such a single molecule methylation level can be used to determine a pathology.

In some embodiments, multiple characteristics of the cell-free DNA molecules (e.g., long cell-free DNA molecules) are combined for determining disease classification and/or predicting tissue of origin. For example, the methylation patterns of sequence reads that have a variant relative to a reference sequence can be determined, and such methylation patterns can be used to determine a disease classification. As another example, relative frequencies of end motifs can be selected from cell-free DNA molecules within a particular size range.

These and other embodiments of the disclosure are described in detail below. For example, other embodiments are directed to systems, devices, and computer readable media associated with methods described herein.

A better understanding of the nature and advantages of embodiments of the present disclosure may be gained with reference to the following detailed description and the accompanying drawings. Reference to the remaining portions of the specification, including the drawings and claims, will realize other features and advantages of the present disclosure. Further features and advantages of the present disclosure, as well as the structure and operation of various embodiments of the present disclosure, are described in detail below with respect to the accompanying drawings. In the drawings, like reference numbers can indicate identical or functionally similar elements.

### BRIEF DESCRIPTION OF DRAWINGS

FIG. 1 shows a schematic diagram that illustrates an example overview of analyzing long cell-free DNA molecules, according to some embodiments.
FIG. 2 shows an example of molecules carrying methylated and/or unmethylated CpG sites that were sequenced by single molecule, real-time sequencing.
FIG. 3 shows a schematic diagram illustrating an example process for determining kinetic features of cell-free DNA molecules, according to some embodiments.
FIG. 4 shows a schematic diagram illustrating another example process for determining kinetic features of cell-free DNA molecules, according to some embodiments.
FIG. 5 shows a graph that identifies proportions of plasma DNA fragments having a length greater than 500 bp across different sequencing techniques, according to some embodiments.
FIG. 6 shows a line graph that illustrates size distribution of one HCC subject and one HBV carrier.
FIG. 7 shows a bar graph that identifies percentages of cfDNA fragments above a given size for HCC patients with vascular invasion and HCC patients without vascular invasion.
FIG. 8 shows a boxplot that identifies percentage of long DNA fragments >200 bp in HCC patients with and without vascular invasion.
FIG. 9 shows a boxplot that identifies size ratios of HCC patients with and without vascular invasion.
FIG. 10 shows a flowchart depicting an example process for analyzing a biological sample of a subject based on frequencies of long cell-free DNA molecules, according to some embodiments.
FIG. 11 shows a heat map generated based on a hierarchical clustering analysis of 256 4-mer end motifs of plasma DNA molecules, according to some embodiments.
FIG. 12 shows a heatmap generated using a hierarchical clustering analysis of 4-mer end motifs of short plasma DNA (<200 bp), according to some embodiments.
FIG. 13 shows a heatmap generated using a hierarchical clustering analysis of 4-mer end motifs of long plasma DNA (>1 kb), according to some embodiments.
FIG. 14 shows a heatmap generated using a hierarchical clustering analysis of 4-mer end motifs of both short (<200 bp) and long plasma DNA (>1 kb), according to some embodiments.
FIG. 15 shows a heatmap generated using a hierarchical clustering analysis of 4-mer end motifs ratios, according to some embodiment.
FIG. 16 shows a flowchart illustrating an example process for analyzing a biological sample of a subject based on relative frequencies of sequences having one or more end motifs, according to some embodiments.
FIG. 17 shows a set of graphs that identify relationships of motif rankings between short plasma DNA molecules (< 600 bp) and long plasma DNA molecules (> 1 kb).
FIG. 18 shows a boxplot that identifies end-motif frequency of CCCA in plasma DNA molecules <200 bp in HCC and non-HCC subjects.
FIG. 19 shows a set of boxplots that identify motif frequencies of CCCA in plasma DNA molecules.
FIG. 20 shows ROC curve that identifies performance of motif frequency of CCCA in distinguishing between HCC and non-HCC subjects in short and long DNA molecules.
FIG. 21 shows a boxplot that identifies CCCA ratios in HCC patients, HBV carriers, and healthy subjects.
FIG. 22 shows an ROC curve that identifies performance of CCCA ratio in distinguishing subjects with and without HCC.
FIG. 23 shows a boxplot that identifies end-motif frequency of CCCA in plasma DNA molecules <200 bp in CRC patients and healthy subjects.
FIG. 24 shows a boxplot that identifies motif frequencies of CCCA in plasma DNA molecules longer than 1 kb in CRC patients and healthy subjects.
FIG. 25 shows a boxplot that identifies CCCA ratios in CRC patients and healthy subjects in SMRT-sequencing.
FIG. 26 shows a boxplot that identifies end-motif frequency of CCCA in plasma DNA molecules <200 bp in HCC patients and HBV carriers.
FIG. 27 shows a set of boxplots that identify motif frequencies of CCCA in plasma DNA molecules.
FIG. 28 shows a boxplot that identifies CCCA ratios in HCC patients and HBV carriers in nanopore sequencing. The CCCA ratio was calculated by dividing the CCCA motif frequency of long DNA molecules (>1 kb) by that of short DNA molecules (<200 bp) in HCC patients and HBV carriers.
FIG. 29 shows a boxplot that identifies results generated by logistic regression analysis of end motif features in short DNA molecules having sizes less than 200 bp.
FIG. 30 shows an ROC curve that identifies performance of logistic regression with the use of end motif features in short DNA molecules (<200 bp) in distinguishing subjects with and without HCC.
FIG. 31 shows a boxplot that identifies results generated from logistic regression analysis of end motif features in long DNA molecules with sizes greater than 1 kb.
FIG. 32 shows an ROC curve that identifies performance of logistic regression with the use of end motif features in long DNA molecules (>1 kb) in distinguishing subjects with and without HCC.
FIG. 33 shows a boxplot that identifies logistic regression analysis with the use of end motif features in both long DNA molecules > 1 kb and short DNA molecules <200 bp.
FIG. 34 shows an ROC curve that identifies performance of logistic regression with the combined use of end motif features derived from both long DNA molecules (>1 kb) and short DNA molecules (<200 bp) in distinguishing subjects with and without HCC.
FIG. 35 shows a boxplot that identifies results generated by logistic regression analysis with the use of motif ratio.
FIG. 36 shows an ROC curve that identifies performance of logistic regression with the use of motif ratios in distinguishing subjects with and without HCC.
FIG. 37 shows an ROC curve that identifies performance of SVM with the use of end-motif ratio in distinguishing subjects with and without HCC.
FIG. 38 shows an ROC curve that identifies performance of random forest analysis with the use of motif ratio in distinguishing subjects with and without HCC.
FIG. 39 shows an ROC curve that identifies performance of LDA analysis with the use of motif ratio in distinguishing subjects with and without HCC.
FIG. 40 shows a flowchart illustrating an example process for analyzing a biological sample of a subject based on relative frequencies of sequences having one or more end motifs, according to some embodiments.
FIG. 41 shows an example illustration of comparing methylation pattern of a long cell-free DNA molecule with methylation patterns of reference tissues, according to some embodiments.
FIG. 42 illustrates a technique for analyzing methylation patterns in long cell-free DNA molecules that include at least one methylation mismatch, according to some embodiments.
FIG. 43 shows a comparison of the pervasiveness of CpG sites and cancer-derived single nucleotide variants (SNVs) across the genome at 1-kb resolution.
FIG. 44 shows a comparison of the pervasiveness of CpG sites and cancer-derived SNVs across the genome at 3-kb resolution.
FIG. 45 shows a comparison of the pervasiveness of CpG sites and cancer-derived SNVs across the genome at 200 bp resolution.
FIG. 46 shows a schematic diagram that illustrates an example process for predicting whether a cell-free DNA molecule corresponds to tumor DNA, according to its methylation haplotype information.
FIG. 47 shows a boxplot that identifies percentage of DNA molecules determined to be of liver origin in HCC patients of different stages, on the basis of the methylation haplotype analysis according to embodiments of the present disclosure.
FIG. 48 shows a boxplot that identifies cancer methylation scores in HCC patients across different stages, according to some embodiments.
FIG. 49 shows a set of survival curves that identify survival analysis in HCC patients, according to some embodiments.
FIG. 50 shows a boxplot that identify HCC methylation scores for HBV carriers and HCC patients calculated using data from SMRT-seq and nanopore sequencing.
FIG. 51 shows a graph that identifies the percentages of liver-derived cfDNA determined by the single-molecule tissue-of-origin analysis in plasma samples from HBV carriers and HCC patients using data from SMRT-seq and nanopore sequencing.
FIG. 52 shows a boxplot that identifies the percentage of plasma DNA molecules being classified as colon origin based on embodiments presented in this disclosure in 15 healthy subjects, 45 HCC patients and 4 CRC patients.
FIG. 53 shows a set of bar plots that identify percentages of DNA molecules determined to be of HCC tumor origin between HCC patients with and without vascular invasion, on the basis of the methylation haplotype analysis according to some embodiments.
FIG. 54 shows a set of bar plots that identify a percentage of DNA molecules determined to be of HCC tumor origin, according to some embodiments.
FIG. 55 shows a set of ROC curves that identify cancer-detection accuracy of an analysis of single molecule methylation sequence data of long cell-free DNA and cancer-detection accuracy of other analyses that use methylation sequence data of short cell-free DNA.
FIG. 56 shows a set of ROC curves that identify HCC-detection accuracy of a methylation haplotype-based analysis using long DNA (> 1 kb) and HCC-detection accuracy of a plasma DNA tissue mapping analysis using short-read bisulfite sequencing of short plasma DNA molecules (< 600 bp).
FIG. 57 shows a flowchart illustrating an example process for analyzing a biological sample of a subject based on methylation patterns of the long cell-free DNA molecules, according to some embodiments.
FIG. 58 shows a boxplot that identifies single-molecule methylation levels in different groups of individuals in single-molecule real-time sequencing (SMRT-Seq), according to some embodiments.
FIG. 59 shows a boxplot that identifies single-molecule methylation levels in DNA molecules with sizes >500 bp, containing at least 3 CpG sites and with methylation level ≤60% in SMRT-Seq.
FIG. 60 shows ROC curves that identify performance of single-molecule methylation levels in distinguishing between HCC and non-HCC subjects in SMRT-Seq and short-read sequencing (e.g., Illumina sequencing), according to some embodiments.
FIG. 61 shows a boxplot that identify single-molecule methylation levels in HCC patients of different Barcelona Clinic Liver Cancer (BCLC) stages.
FIG. 62 shows a flowchart illustrating an example process for determining a disease classification using single-molecule methylation levels in DNA molecules, according to some embodiments.
FIG. 63 shows an illustrative diagram for pattern recognition of methylation haplotypes using machine-learning models, according to some embodiments.
FIG. 64 shows a set of bar graphs that identify performance of the machine-learning model for differentiating between tumoral and non-tumoral DNA in plasma across different sequencing depths used in the training process.
FIG. 65 shows a set of bar graphs that identify performance of the machine-learning model for differentiating between tumoral and non-tumoral DNA in plasma, in which the machine-learning was trained using differentially methylated regions across different sequencing depths.
FIG. 66 shows a table that identifies performance of a machine-learning model differentiating between tumoral and non-tumoral DNA in plasma of cancer patients, with different lengths of plasma DNA molecules.
FIG. 67 shows a flowchart illustrating an example process for using machine-learning models to determine a tissue-type property based on methylation patterns of long cell-free DNA molecules, according to some embodiments.
FIG. 68 shows a schematic diagram that illustrates an example of combined analysis using SNV and CpG methylation haplotype information, according to some embodiments.
FIG. 69 shows characteristics of a first group of plasma DNA molecules carrying wildtype alleles and a second group of plasma DNA molecules carry mutations.
FIG. 70 shows a table identifying distributions of the number of CpG sites in a 200 bp or 1kb region surrounding a somatic mutation.
FIG. 71 shows a schematic diagram of DNA molecules having relative haplotype imbalance with skewed allelic ratio and skewed methylation level informs the presence or absence of cancer.
FIG. 72 shows a flowchart illustrating an example process for analyzing a biological sample of using variants and methylation patterns to determine a tissue of origin based on methylation patterns of long cell-free DNA molecules, according to some embodiments.
FIG. 73 shows a flowchart illustrating an example process for analyzing a biological sample of using variants and methylation patterns to determine a cancer classification based on methylation patterns of long cell-free DNA molecules, according to some embodiments.
FIG. 74 shows a schematic diagram illustrating an example process for training a machine-learning model for differentiating patients with and without cancers, based on, sequence context, genomic locations, fragmentomic and epigenetic information present in plasma DNA molecules.
FIG. 75 shows a schematic diagram illustrating an example process for applying the trained model to cancer detection using fragmentomic and epigenetic information present in plasma DNA molecules.
FIG. 76 shows a flowchart illustrating an example process for analyzing a biological sample of a subject using machine-learning models to determine a disease classification based on multiple characteristics of long cell-free DNA molecules, according to some embodiments.
FIG. 77 shows an example set of microsatellite sequences in DNA molecules.
FIG. 78 illustrates an example overview of detecting tumor-derived DNA based on a cancer-specific microsatellite marker.
FIG. 79 illustrates a measurement system according to an embodiment of the present invention.
FIG. 80 shows a block diagram of an example computer system usable with system and methods according to embodiments of the present invention.

### TERMS

A *"tissue"* corresponds to a group of cells that group together as a functional unit. More than one type of cells can be found in a single tissue. Different types of tissue may consist of different types of cells (e.g., hepatocytes, alveolar cells or blood cells), but also may correspond to tissue from different organisms (host vs. virus) or to healthy cells vs. tumor cells. The term "tissue" can generally refer to any group of cells found in the human body (e.g., heart tissue, lung tissue, kidney tissue, nasopharyngeal tissue, oropharyngeal tissue). In some aspects, the term "tissue" or "tissue type" may be used to refer to a tissue from which a cell-free nucleic acid originates. In one example, viral nucleic acid fragments may be derived from blood tissue, e.g., for Epstein- Barr Virus (EBV). In another example, viral nucleic acid fragments may be derived from tumor tissue, e.g., EBV or Human papillomavirus infection (HPV).

The term "*sample*", "*biological sample*" or "*patient sample*" is meant to include any tissue or material derived from a living or dead subject. A biological sample may be a cell-free sample, which may include a mixture of nucleic acid molecules from the subject and potentially nucleic acid molecules from a pathogen, e.g., a virus. A biological sample generally comprises a nucleic acid (e.g., DNA or RNA) or a fragment thereof. The term "nucleic acid" may generally refer to deoxyribonucleic acid (DNA), ribonucleic acid (RNA) or any hybrid or fragment thereof. The nucleic acid in the sample may be a cell-free nucleic acid. A sample may be a liquid sample or a solid sample (e.g., a cell or tissue sample). The biological sample can be a bodily fluid, such as blood, plasma, serum, urine, vaginal fluid, fluid from a hydrocele (e.g., of the testis), vaginal flushing fluids, pleural fluid, ascitic fluid, cerebrospinal fluid, saliva, sweat, tears, sputum, bronchoalveolar lavage fluid, discharge fluid from the nipple, aspiration fluid from different parts of the body (e.g., thyroid, breast), etc. Stool samples can also be used. In various embodiments, the majority of DNA in a biological sample that has been enriched for cell-free DNA (e.g., a plasma sample obtained via a centrifugation protocol) can be cell-free (e.g., greater than 50%, 60%, 70%, 80%, 90%, 95%, or 99% of the DNA can be cell-free). In some embodiments, at least 1,000 cell-free DNA molecules are analyzed. In other embodiments, at least 10,000 or 50,000 or 100,000 or 500,000 or 1,000,000 or 5,000,000 cell-free DNA molecules, or more, can be analyzed. At least a same number of sequence reads can be analyzed. The biological sample may be treated to physically disrupt tissue or cell structure (e.g., centrifugation and/or cell lysis), thus releasing intracellular components into a solution which may further contain enzymes, buffers, salts, detergents, and the like which are used to prepare the sample for analysis.

The term "*constitutional genome*" (also referred to a CG) is composed of the consensus nucleotides at loci within the genome, and thus can be considered a consensus sequence. The CG can cover the entire genome of the subject (e.g., the human genome), or just parts of the genome. The constitutional genome (CG) can be obtained from DNA of cells as well as cell-free DNA (e.g., as can be found in plasma). Ideally, the consensus nucleotides should indicate that a locus is homozygous for one allele or heterozygous for two alleles. A heterozygous locus typically contains two alleles which are members of a genetic polymorphism. As an example, the criteria for determining whether a locus is heterozygous can be a threshold of two alleles each appearing in at least a predetermined percentage (e.g., 30% or 40%) of reads aligned to the locus. If one nucleotide appears at a sufficient percentage (e.g., 70% or greater) then the locus can be determined to be homozygous in the CG. Although the genome of one healthy cell can differ from the genome of another healthy cell due to random mutations spontaneously occurring during cell division, the CG should not vary when such a consensus is used. Some cells can have genomes with genomic rearrangements, e.g., B and T lymphocytes, such as involving antibody and T cell receptor genes. Such large scale differences would still be a relatively small population of the total nucleated cell population in blood, and thus such rearrangements would not affect the determination of the constitutional genome with sufficient sampling (e.g., sequencing depth) of blood cells. Other cell types, including buccal cells, skin cells, hair follicles, or biopsies of various normal body tissues, can also serve as sources of CG.

The term "*constitutional DNA*" refers to any source of DNA that is reflective of the genetic makeup with which a subject is born. For a subject, examples of "constitutional samples", from which constitutional DNA can be obtained, include healthy blood cell DNA, buccal cell DNA and hair root DNA. The DNA from these healthy cells defines the CG of the subject. The cells can be identified as healthy in a variety of ways, e.g., when a person is known to not have cancer or the sample can be obtained from tissue that is not likely to contain cancerous or premalignant cells (e.g., hair root DNA when liver cancer is suspected). As another example, a plasma sample may be obtained when a patient is cancer-free, and the determined constitutional DNA compared against results from a subsequent plasma sample (e.g., a year or more later). In another embodiment, a single biologic sample containing <50% of tumor DNA can be used for deducing the constitutional genome and the tumor-associated genetic alterations. In such a sample, the concentrations of tumor-associated single nucleotide mutations would be lower than those of each allele of heterozygous SNPs in the CG. Such a sample can be the same as the biological sample used to determine a sample genome, described below.

*A* "*sequence read*" refers to a string of nucleotides sequenced from any part or all of a nucleic acid molecule. For example, a sequence read may be a short string of nucleotides (e.g., 20-150 nucleotides) sequenced from a nucleic acid fragment, a short string of nucleotides at one or both ends of a nucleic acid fragment, or the sequencing of the entire nucleic acid fragment that exists in the biological sample. A sequence read may be obtained in a variety of ways, e.g., using sequencing techniques or using probes, e.g., in hybridization arrays or capture probes as may be used in microarrays, or amplification techniques, such as the polymerase chain reaction (PCR) or linear amplification using a single primer or isothermal amplification. As part of an analysis of a biological sample, at least 1,000 sequence reads can be analyzed. As other examples, at least 10,000 or 50,000 or 100,000 or 500,000 or 1,000,000 or 5,000,000 sequence reads, or more, can be analyzed. Such example numbers of DNA molecules may be analyzed as part of massively parallel sequencing.

A sequence read can include an "*ending sequence*" associated with an end of a fragment. The ending sequence can correspond to the outermost N bases of the fragment, e.g., 1-30 bases at the end of the fragment. If a sequence read corresponds to an entire fragment, then the sequence read can include two ending sequences. When paired-end sequencing provides two sequence reads that correspond to the ends of the fragments, each sequence read can include one ending sequence.

A "*sequence motif*" may refer to a short, recurring pattern of bases in DNA fragments (e.g., cell-free DNA fragments). A sequence motif can occur at an end of a fragment, and thus be part of or include an ending sequence. An "*end motif*" can refer to a sequence motif for an ending sequence that preferentially occurs at ends of DNA fragments, potentially for a particular type of tissue. An end motif may also occur just before or just after ends of a fragment, thereby still corresponding to an ending sequence. A nuclease can have a specific cutting preference for a particular end motif, as well as a second most preferred cutting preference for a second end motif.

An "*ending position*" or "*end position*" (or just "*end*) can refer to the genomic coordinate or genomic identity or nucleotide identity of the outermost base, i.e. at the extremities, of a cell-free DNA molecule, e.g. plasma DNA molecule. The end position can correspond to either end of a DNA molecule. In this manner, if one refers to a start and end of a DNA molecule, both would correspond to an ending position. In practice, one end position is the genomic coordinate or the nucleotide identity of the outermost base on one extremity of a cell-free DNA molecule that is detected or determined by an analytical method, such as but not limited to massively parallel sequencing or next-generation sequencing, single molecule sequencing, double- or single-stranded DNA sequencing library preparation protocols, polymerase chain reaction (PCR), or microarray. Such *in vitro* techniques may alter the true *in vivo* physical end(s) of the cell-free DNA molecules. Thus, each detectable end may represent the biologically true end or the end is one or more nucleotides inwards or one or more nucleotides extended from the original end of the molecule e.g. 5' blunting and 3' filling of overhangs of non-blunt-ended double stranded DNA molecules by the Klenow fragment. The genomic identity or genomic coordinate of the end position could be derived from results of alignment of sequence reads to a human reference genome, e.g. hg19. It could be derived from a catalog of indices or codes that represent the original coordinates of the human genome. It could refer to a position or nucleotide identity on a cell-free DNA molecule that is read by but not limited to target-specific probes, mini-sequencing, DNA amplification.

A "*preferred end*" (or "*recurrent ending position*") refers to an end that is more highly represented or prevalent (e.g., as measured by a rate) in a biological sample having a physiological (e.g. pregnancy) or pathological (disease) state (e.g. cancer) than a biological sample not having such a state or than at different time points or stages of the same pathological or physiological state, e.g., before or after treatment. A preferred end therefore has an increased likelihood or probability for being detected in the relevant physiological or pathological state relative to other states. The increased probability can be compared between the pathological state and a non-pathological state, for example in patients with and without a cancer and quantified as likelihood ratio or relative probability. The likelihood ratio can be determined based on the probability of detecting at least a threshold number of preferred ends in the tested sample or based on the probability of detecting the preferred ends in patients with such a condition than patients without such a condition. Examples for the thresholds of likelihood ratios include but not limited to 1.1, 1.2, 1.3, 1.4, 1.5, 1.6, 1.8, 2.0, 2.5, 3.0, 3.5, 4.0, 4.5, 5, 6, 8, 10, 20, 40, 60, 80 and 100. Such likelihood ratios can be measured by comparing relative abundance values of samples with and without the relevant state. Because the probability of detecting a preferred end in a relevant physiological or disease state is higher, such preferred ending positions would be seen in more than one individual with that same physiological or disease state. With the increased probability, more than one cell-free DNA molecule can be detected as ending on a same preferred ending position, even when the number of cell-free DNA molecules analyzed is far less than the size of the genome. Thus, the preferred or recurrent ending positions are also referred to as the "frequent ending positions." In some embodiments, a quantitative threshold may be used to require that ends be detected at least multiple times (e.g., 3, 4, 5, 6, 7, 8, 9, 10, 15, 20, or 50) within the same sample or same sample aliquot to be considered as a preferred end. A relevant physiological state may include a state when a person is healthy, disease-free, or free from a disease of interest. Similarly, a "*preferred ending window*" corresponds to a contiguous set of preferred ending positions.

A "*rate*" of DNA molecules ending on a position relates to how frequently a DNA molecule ends on the position. Such a rate can be referred to as an "*end density.*" The rate may be based on a number of DNA molecules that end on the position normalized against a number of DNA molecules analyzed. The normalization can also be based on the average, median, or total number of ends in the surrounding region. The surrounding region used for normalization may include, but is not limited to, 500, 1000, 3000, 5000, etc. bp upstream and/or downstream of the position.

The term "*alleles*" refers to alternative DNA sequences at the same physical genomic locus, which may or may not result in different phenotypic traits. In any particular diploid organism, with two copies of each chromosome (except the sex chromosomes in a male human subject), the genotype for each gene comprises the pair of alleles present at that locus, which are the same in homozygotes and different in heterozygotes. A population or species of organisms typically include multiple alleles at each locus among various individuals. A genomic locus where more than one allele is found in the population is termed a polymorphic site. Allelic variation at a locus is measurable as the number of alleles (i.e., the degree of polymorphism) present, or the proportion of heterozygotes (i.e., the heterozygosity rate) in the population. As used herein, the term "*polymorphism*" refers to any inter-individual variation in the human genome, regardless of its frequency. Examples of such variations include, but are not limited to, single nucleotide polymorphism, simple tandem repeat polymorphisms, insertion-deletion polymorphisms, mutations (which may be disease causing) and copy number variations. The term "*haplotype*" as used herein refers to a combination of alleles at multiple loci that are transmitted together on the same chromosome or chromosomal region. A haplotype may refer to as few as one pair of loci or to a chromosomal region, or to an entire chromosome or chromosome arm.

A "*relative frequency*" (also referred to just as "frequency") may refer to a proportion (e.g., a percentage, fraction, or concentration). In particular, a relative frequency of a particular end motif (e.g., CCGA or just a single base) can provide a proportion of cell-free DNA fragments in a sample that are associated with the end motif CCGA, e.g., by having an ending sequence of CCGA. As another example, a relative frequency can be a ranking of the occurrences if each motif among each other. Such a ranking can use proportions or the raw counts, as the denominator would be the same.

A "*subread*" is a sequence generated from all bases in one strand of a circularized DNA template that has been copied in one contiguous strand by a DNA polymerase. For example, a subread can correspond to one strand of circularized DNA template DNA. In such an example, after circularization, one double-stranded DNA molecule would have two subreads: one for each sequencing pass. In some embodiments, the sequence generated may include a subset of all the bases in one strand, e.g., because of the existence of sequencing errors.

A "*site*" (also called a "*genomic site*") corresponds to a single site, which may be a single base position or a group of correlated base positions, e.g., a CpG site or larger group of correlated base positions. A "locus" may correspond to a region that includes multiple sites. A locus can include just one site, which would make the locus equivalent to a site in that context.

A "*methylation status*" refers to the state of methylation at a given site. For example, a site may be either methylated, unmethylated, or in some cases, undetermined. A sequence read can include one or more sites at which a methylation status of the corresponding cell-free DNA molecule can be determined. Each site of one or more sites can be associated with a methylation status. For example, one or more sites can be CpG sites, and each site can be a CpG site at which a particular methylation status is determined. In some instances, the one or more sites for each of the sequence reads include at least an N number of sites. For example, a given sequence read can include at least 3 CpG sites. Other numbers can be contemplated, including but are not limited to at least 2, 3, 4, 5, 6, 7, 8, 9, 10, 15, 20, 25, 30, 35, 40, 45, 50, or greater than 50 sites. Additionally or alternatively, a sequence read can correspond to long cell-free DNA molecules having sizes within a first size range (e.g., greater than 500 base pairs (bps)) and include at least an *N* number of sites (e.g., 3 CpG sites). As used herein, a "*set of sites*" can correspond to the N number of sites.

The "*methylation index*" for each genomic site (e.g., a CpG site) can refer to the proportion of DNA fragments (e.g., as determined from sequence reads or probes) showing methylation at the site over the total number of reads covering that site. A "read" can correspond to information (e.g., methylation status at a site) obtained from a DNA fragment. A read can be obtained using reagents (e.g., primers or probes) that preferentially hybridize to DNA fragments of a particular methylation status at one or more sites. Typically, such reagents are applied after treatment with a process that differentially modifies or differentially recognizes DNA molecules depending on their methylation status, e.g. bisulfite conversion, or methylation-sensitive restriction enzyme, or methylation binding proteins, or anti-methylcytosine antibodies, or single molecule sequencing techniques (e.g. single molecule, real-time sequencing and nanopore sequencing (e.g. from Oxford Nanopore Technologies)) that recognize methylcytosines and hydroxymethylcytosines. The methylation index can be transformed into a binary value (0 or 1). For example, the methylation index can be recoded as 0 when the actual methylation index ≤ 0.5, and the methylation index can be recoded as 1 when the actual methylation index > 0.5. The methylation index is a binary value when one refers to the methylation across individual CpG sites in a single DNA molecule.

The "*methylation density*" of a region can refer to the number of reads at sites within the region showing methylation divided by the total number of reads covering the sites in the region. The sites may have specific characteristics, e.g., being CpG sites. Thus, the "CpG methylation density" of a region can refer to the number of reads showing CpG methylation divided by the total number of reads covering CpG sites in the region (e.g., a particular CpG site, CpG sites within a CpG island, or a larger region). For example, the methylation density for each 100-kb bin in the human genome can be determined from the total number of cytosines not converted after bisulfite treatment (which corresponds to methylated cytosine) at CpG sites as a proportion of all CpG sites covered by sequence reads mapped to the 100-kb region. This analysis can also be performed for other bin sizes, e.g., 500 bp, 5 kb, 10 kb, 50-kb or 1-Mb, etc. A region could be the entire genome or a chromosome or part of a chromosome (e.g., a chromosomal arm). The methylation index of a CpG site is the same as the methylation density for a region when the region only includes that CpG site. The "proportion of methylated cytosines" can refer the number of cytosine sites, "C's", that are shown to be methylated (for example unconverted after bisulfite conversion) over the total number of analyzed cytosine residues, i.e., including cytosines outside of the CpG context, in the region. The methylation index, methylation density, count of molecules methylated at one or more sites, and proportion of molecules methylated (e.g., cytosines) at one or more sites are examples of "*methylation levels.*" Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al. Proc Natl Acad Sci 2013; 110: 18910-18915) and by single molecule, real-time sequencing (e.g. that from Pacific Biosciences) (Flusberg et al. Nat Methods 2010; 7: 461-465)).

A "*methylome*" provides a measure of an amount of DNA methylation at a plurality of sites or loci in a genome. The methylome may correspond to all of the genome, a substantial part of the genome, or relatively small portion(s) of the genome.

A "*methylation profile*" includes information related to DNA or RNA methylation for multiple sites or regions. Information related to DNA methylation can include, but not limited to, a methylation index of a CpG site, a methylation density (MD for short) of CpG sites in a region, a distribution of CpG sites over a contiguous region, a pattern or level of methylation for each individual CpG site within a region that contains more than one CpG site, and non-CpG methylation. In some embodiments, the methylation profile can include the pattern of methylation or non-methylation of more than one type of base (e.g. cytosine or adenine). A methylation profile of a substantial part of the genome can be considered equivalent to the methylome. "DNA methylation" in mammalian genomes typically refers to the addition of a methyl group to the 5' carbon of cytosine residues (i.e. 5-methylcytosines) among CpG dinucleotides. DNA methylation may occur in cytosines in other contexts, for example CHG and CHH, where H is adenine, cytosine or thymine. Cytosine methylation may also be in the form of 5-hydroxymethylcytosine. Non-cytosine methylation, such as N⁶-methyladenine, has also been reported.

A "*methylation pattern*" refers to the order of methylated and non-methylated bases. For example, the methylation pattern can be the order of methylated bases on a single DNA strand, a single double-stranded DNA molecule, or another type of nucleic acid molecule. As an example, three consecutive CpG sites may have any of the following methylation patterns: UUU, MMM, UMM, UMU, UUM, MUM, MUU, or MMU, where "U" indicates an unmethylated site and "M" indicates a methylated site. When one extends this concept to base modifications that include, but not restricted to methylation, one would use the term "*modification pattern*," which refers to the order of modified and non-modified bases. For example, the modification pattern can be the order of modified bases on a single DNA strand, a single double-stranded DNA molecule, or another type of nucleic acid molecule. As an example, three consecutive potentially modifiable sites may have any of the following modification patterns: UUU, MMM, UMM, UMU, UUM, MUM, MUU, or MMU, where "U" indicates an unmodified site and "M" indicates a modified site. One example of base modification that is not based on methylation is oxidation changes, such as in 8-oxo-guanine.

The terms "*hypermethylated*" and "*hypomethylated*" may refer to the methylation density of a single DNA molecule as measured by its single molecule methylation level, e.g., the number of methylated bases or nucleotides within the molecule divided by the total number of methylatable bases or nucleotides within that molecule. A hypermethylated molecule is one in which the single molecule methylation level is at or above a threshold, which may be defined from application to application. The threshold may be 5%, 10%, 20%, 30%, 40%, 50%, 60%, 70%, 80%, 90%, or 95%. A hypomethylated molecule is one in which the single molecule methylation level is at or below a threshold, which may be defined from application to application, and which may change from application to application. The threshold may be 5%, 10%, 20%, 30%, 40%, 50%, 60%, 70%, 80%, 90%, or 95%.

The terms "hypermethylated" and "hypomethylated" may also refer to the methylation level of a population of DNA molecules as measured by the multiple molecule methylation levels of these molecules. A hypermethylated population of molecules is one in which the multiple molecule methylation level is at or above a threshold which may be defined from application to application, and which may change from application to application. The threshold may be 5%, 10%, 20%, 30%, 40%, 50%, 60%, 70%, 80%, 90%, or 95%. A hypomethylated population of molecules is one in which the multiple molecule methylation level is at or below a threshold which may be defined from application to application. The threshold may be 5%, 10%, 20%, 30%, 40%, 50%, 60%, 70%, 80%, 90%, and 95%. In some embodiments, the population of molecules may be aligned to one or more selected genomic regions. In some embodiments, the selected genomic region(s) may be related to a disease such as a genetic disorder, an imprinting disorder, a metabolic disorder, or a neurological disorder. The selected genomic region(s) can have a length of 50 nucleotides (nt), 100 nt, 200 nt, 300 nt, 500 nt, 1000 nt, 2 knt, 5 knt, 10 knt, 20 knt, 30 knt, 40 knt, 50 knt, 60 knt, 70 knt, 80 knt, 90 knt, 100 knt, 200 knt, 300 knt, 400 knt, 500 knt, or 1 Mnt.

*"Methylation-aware sequencing"* refers to any sequencing method that allows one to ascertain the methylation status of a DNA molecule during a sequencing process, including, but not limited to bisulfite sequencing, or sequencing preceded by methylation-sensitive restriction enzyme digestion, immunoprecipitation using anti-methylcytosine antibody or methylation binding protein, or single molecule sequencing that allows elucidation of the methylation status (e.g., without bisulfite sequencing). Any such sequencing described herein may be massively parallel sequencing. A "*methylation-aware assay*" or "*methylation-sensitive assay*" can include both sequencing and non-sequencing based methods, such as MSP, probe based interrogation, hybridization, restriction enzyme digestion followed by density measurements, anti-methylcytosine immunoassays, mass spectrometry interrogation of proportion of methylated cytosines or hydroxymethylcytosines, immunoprecipitation not followed by sequencing, etc.

The term "*sequencing depth*" refers to the number of times a locus is covered by a sequence read aligned to the locus. The locus could be as small as a nucleotide, or as large as a chromosome arm, or as large as the entire genome. Sequencing depth can be expressed as 50x, 100x, etc., where "x" refers to the number of times a locus is covered with a sequence read. Sequencing depth can also be applied to multiple loci, or the whole genome, in which case x can refer to the mean number of times the loci or the haploid genome, or the whole genome, respectively, is sequenced. Ultra-deep sequencing can refer to at least 100x in sequencing depth.

The term "*level of cancer*" can refer to whether cancer exists, a stage of a cancer, a size of tumor, whether there is metastasis, the total tumor burden of the body, and/or other measure of a severity of a cancer. The level of cancer could be a number or other indicia, such as symbols, alphabet letters, and colors. The level could be zero. The level of cancer also includes premalignant or precancerous conditions (states) associated with mutations or a number of mutations. The level of cancer can be used in various ways. For example, screening can check if cancer is present in someone who is not known previously to have cancer. Assessment can investigate someone who has been diagnosed with cancer to monitor the progress of cancer over time, study the effectiveness of therapies or to determine the prognosis. In some embodiments, the prognosis can be expressed as the chance of a patient dying of cancer, or the chance of the cancer progressing after a specific duration or time, or the chance of cancer metastasizing. Detection can mean 'screening' or can mean checking if someone, with suggestive features of cancer (e.g. symptoms or other positive tests), has cancer.

A *"level of pathology"* (or level of disorder) can refer to the amount, degree, or severity of pathology associated with an organism, where the level can be as described above for cancer. Another example of pathology is a rejection of a transplanted organ. Other example pathologies can include gene imprinting disorders, autoimmune attack (e.g., lupus nephritis damaging the kidney or multiple sclerosis), inflammatory diseases (e.g., hepatitis), fibrotic processes (e.g. cirrhosis), fatty infiltration (e.g. fatty liver diseases), degenerative processes (e.g. Alzheimer's disease), and ischemic tissue damage (e.g., myocardial infarction or stroke). A heathy state of a subject can be considered a classification of no pathology.

A "*separation value*" corresponds to a difference or a ratio involving two values, e.g., two fractional contributions or two methylation levels. The separation value could be a simple difference or ratio. The separation value can include other factors, e.g., multiplicative factors. As other examples, a difference or ratio of functions of the values can be used, e.g., a difference of the natural logarithms (ln) of the two values.

A "*separation value*" and an "*aggregate value*" (e.g., of relative frequencies) are two examples of a parameter (also called a metric) that provides a measure of a sample that varies between different classifications (states), and thus can be used to determine different classifications. An aggregate value can be a separation value, e.g., when a difference is taken between a set of relative frequencies of a sample and a reference set of relative frequencies, as may be done in clustering.

A "*relative abundance*" is a type of separation value that relates an amount (one value) of cell-free DNA molecules ending within one window of genomic position to an amount (other value) of cell-free DNA molecules ending within another window of genomic positions. The two windows may overlap, but would be of different sizes. In other implementations, the two windows would not overlap. Further, the windows may be of a width of one nucleotide, and therefore be equivalent to one genomic position. An end density is a type of relative abundance.

The term "*classification*" as used herein refers to any number(s) or other characters(s) that are associated with a particular property of a sample. For example, a "+" symbol (or the word "positive") could signify that a sample is classified as having deletions or amplifications. The classification can be binary (e.g., positive or negative) or have more levels of classification (e.g., a scale from 1 to 10 or 0 to 1). The term "*cutoff*" and "*threshold*" refer to a predetermined number used in an operation. For example, a cutoff size can refer to a size above which fragments are excluded. A threshold value may be a value above or below which a particular classification applies. Either of these terms can be used in either of these contexts.

The term "*parameter*" as used herein means a numerical value that characterizes a quantitative data set and/or a numerical relationship between quantitative data sets. For example, a ratio (or function of a ratio) between a first amount of a first nucleic acid sequence and a second amount of a second nucleic acid sequence is a parameter.

The term "*size profile*" generally relates to the sizes of DNA fragments in a biological sample. A size profile may be a histogram that provides a distribution of an amount of DNA fragments at a variety of sizes. Various statistical parameters (also referred to as size parameters or just parameter) can be used to distinguish one size profile to another. One parameter is the percentage of DNA fragment of a particular size or range of sizes relative to all DNA fragments or relative to DNA fragments of another size or range.

The terms "*cutoff*" and "*threshold*" refer to predetermined numbers used in an operation. For example, a cutoff size can refer to a size above which fragments are excluded. A threshold value may be a value above or below which a particular classification applies. Either of these terms can be used in either of these contexts. A cutoff or threshold may be "a reference value" or derived from a reference value that is representative of a particular classification or discriminates between two or more classifications. Such a reference value can be determined in various ways, as will be appreciated by the skilled person. For example, metrics can be determined for two different cohorts of subjects with different known classifications, and a reference value can be selected as representative of one classification (e.g., a mean) or a value that is between two clusters of the metrics (e.g., chosen to obtain a desired sensitivity and specificity). As another example, a reference value can be determined based on statistical analyses or simulations of samples. A particular value for a cutoff, threshold, reference, etc. can be determined based on a desired accuracy (e.g., a sensitivity and specificity).

The abbreviation "*bp*" refers to base pairs. In some instances, "bp" may be used to denote a length of a DNA fragment, even though the DNA fragment may be single stranded and does not include a base pair. In the context of single-stranded DNA, "bp" may be interpreted as providing the length in nucleotides.

The abbreviation "*nt*" refers to nucleotides. In some instances, "nt" may be used to denote a length of a single-stranded DNA in a base unit. Also, "nt" may be used to denote the relative positions such as upstream or downstream of the locus being analyzed. For a double-stranded DNA, "nt" may still refer to the length of a single strand rather than the total number of nucleotides in the two strands, unless context clearly dictates otherwise. In some contexts concerning technological conceptualization, data presentation, processing and analysis, "nt" and "bp" may be used interchangeably.

The term "*kinetic features*" can refer to features derived from sequencing, including from single molecule, real-time sequencing. Such features can be used for base modification analysis. Example kinetic features include upstream and downstream sequence context, strand information, interpulse duration, pulse widths, and pulse strength. In single molecule, real-time sequencing, one is continuously monitoring the effects of activities of a polymerase on a DNA template. Hence, measurements generated from such a sequencing can be regarded as kinetic features, e.g., nucleotide sequences.

The term "*machine learning models*" may include models based on using sample data (e.g., training data) to make predictions on test data, and thus may include supervised learning. Machine learning models often are developed using a computer or a processor. Machine learning models may include statistical models.

The term "*data analysis framework*" may include algorithms and/or models that can take data as an input and then output a predicted result. Examples of "data analysis frameworks" include statistical models, mathematical models, machine learning models, other artificial intelligence models, and combinations thereof.

The term "*real-time sequencing*" may refer to a technique that involves data collection or monitoring during progress of a reaction involved in sequencing. For example, real-time sequencing may involve optical monitoring or filming the DNA polymerase incorporating a new base.

The term "*about*" or "*approximately*" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term "about" or "approximately" can mean within an order of magnitude, within 5-fold, and more preferably within 2-fold, of a value. Where particular values are described in the application and claims, unless otherwise stated the term "about" meaning within an acceptable error range for the particular value should be assumed. The term "about" can have the meaning as commonly understood by one of ordinary skill in the art. The term "about" can refer to ±10%. The term "about" can refer to ±5%.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within embodiments of the present disclosure. The upper and lower limits of these smaller ranges may independently be included or excluded in the range, and each range where either, neither, or both limits are included in the smaller ranges is also encompassed within the present disclosure, subject to any specifically excluded limit in the stated range. Where the stated range includes one or both of the limits, ranges excluding either or both of those included limits are also included in the present disclosure.

Standard abbreviations may be used, e.g., bp, base pair(s); kb, kilobase(s); pi, picoliter(s); s or sec, second(s); min, minute(s); h or hr, hour(s); aa, amino acid(s); nt, nucleotide(s); and the like.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the embodiments of the present disclosure, some potential and exemplary methods and materials may now be described.

### DETAILED DESCRIPTION

The present techniques include analyzing the presence, abundance and sequence characteristics of long cell-free DNA molecules in plasma samples of subjects with cancer and subjects without cancer. These characteristics can then be used to determine a disease classification for a subject. Using these long cell-free DNA molecules allows for analysis not contemplated or not possible with shorter cell-free DNA fragments. For example, the status of methylated CpG sites and single nucleotide polymorphisms (SNPs) is often used to analyze DNA fragments of a biological sample. A CpG site and a SNP are typically separated from the nearest CpG site or SNP by hundreds or thousands of base pairs. The length of most of the cell-free DNA fragments in a biological sample is usually less than 200 bp. As a result, finding two or more consecutive CpG sites or SNPs on most cell-free DNA fragments is improbable or impossible. Cell-free DNA fragments longer than 200 bp, including those longer than 600 bp or 1 kb, may include multiple CpG sites and/or SNPs. The presence of multiple CpG sites and/or SNPs on long cell-free DNA fragments may allow for more efficient and/or accurate analysis than with short cell-free DNA fragments alone.

In some embodiments, methylation patterns of cell-free DNA molecules are used to determine a classification of a disease of a subject. A methylation pattern of a cell-free DNA molecule can include methylation statuses of a set of sites (e.g., at least three CpG sites). The methylation status can indicate whether a corresponding site is methylated or unmethylated. To determine the methylation patterns, a biological sample can be sequenced using methylation-aware sequencing (e.g., single-molecule sequencing, nanopore sequencing) to obtain sequence reads, in which each of the sequence reads include the respective methylation patterns. Long cell-free DNA molecules (e.g., sizes greater than 600 bp) can be used, as they can include relatively more CpG sites (e.g., at least three CpG sites).

For each of the sequence reads, the methylation pattern of the sequence read is compared to one or more reference methylation patterns. Each of the one or more reference methylation patterns can be associated with a tissue type of a plurality of tissue types. In some instances, a reference methylation pattern of the one or more reference methylation patterns is associated with a known classification of the disease. For example, the comparison can include: (i) determining, for each site of the set of sites, a similarity metric between the methylation status of a CpG site of the sequence read and a methylation index of a reference methylation pattern for a corresponding CpG site, and (ii) generating an aggregate value (e.g., a sum) of the sequence read based on the similarity metrics. Based on the comparison, a tissue classification (e.g., liver) of the sequence read can then determined based on a reference methylation pattern that most closely matches the methylation pattern of the sequence read. Continuing with the example, the reference methylation pattern that most closely matches the methylation pattern can be determined if the aggregate value of the reference methylation pattern is greater than one or more other aggregate values of other reference methylation patterns. The tissue classification process can be repeated for each sequence read, until the tissue classifications are determined for the sequence reads. The disease classification can then be determined based on the tissue classifications. For example, the disease classification can be determined based on an amount of sequence reads being classified as having a particular tissue classification (e.g., liver, lung, colon).

In some instances, the methylation pattern of each sequence reads is inputted to a machine-learning model to generate an output indicative of a tissue classification of the sequence read. The classifications can be used to determine a property of a tissue type (e.g., an amount of sequence reads classified as being derived from the tissue type). The property of the tissue type can also identify a disease state of a disease associated with the tissue type.

In some instances, the methylation pattern and one or more variants (e.g., a polymorphism) detected in the cell-free DNA molecule is used to determine a tissue of origin. For example, a number of plasma DNA molecules can carry mutations not present in white blood cells. But, it can be determined that these plasma DNA molecules are associated with liver tissue based on their respective methylation patterns. In some instances, the variant and the methylation pattern is inputted to the machine-learning model to generate an output, at which the output is used to determine the tissue of origin for the cell-free DNA molecule.

The methylation pattern and one or more variants (e.g., a polymorphism) detected in the cell-free DNA molecule can be used together to determine a classification of cancer. For example, the variants of the plasma DNA molecules (e.g., single-nucleotide variant) and their respective methylation patterns (e.g., a large number of unmethylated statuses) of sequences surrounding the variants can be used in tandem to determine that a classification of hepatocellular carcinoma (HCC).

In some embodiments, an amount of long cell-free DNA molecules is used to determine a classification of cancer of a subject. For example, a size of each cell-free DNA molecule is measured. An amount of cell-free DNA molecules having a size within a size range (e.g., sizes greater than 1000 bp) can be determined. A normalized parameter can be determined from the determined amount of cell-free DNA molecules. For example, the normalized parameter can be determined by normalizing the first amount with a second amount of cell-free DNA molecules in a second size range (e.g., sizes less than 150 bp). In some instances, the normalized parameter is a ratio value between the first amount and the second amount. The normalized parameter can then be used to determine the level of cancer.

In some embodiments, frequencies of end motifs of cell-free DNA molecules are used to determine a classification of the disease. A biological sample is sequenced to obtain sequence reads. For each of the sequence reads, a sequence motif (e.g., CCCA) for each of ending sequences of the sequence read can be determined. Then, for each sequence motif of a set of N sequence motifs, a relative frequency can be determined. For example, the relative frequency for the sequence motif can be determined based on a proportion of cell-free DNA molecules that have ending sequences corresponding to the sequence motif relative to a number of cell-free DNA molecules that have ending sequence corresponding to other sequence motifs of the set of N sequence motifs.

A vector of N frequencies can be determined using the relative frequencies of the set of N motifs, in which each of N frequencies is normalized to each other or to other frequencies of the sequence motif in a group of reference samples. The vector can be compared to a plurality of reference vectors. The comparisons can include determining a distance between the vector and a reference vector of the plurality of reference vectors. Each of the plurality of reference vectors is determined using a reference sample of known classification of the disease. Based on the comparisons, the classification of the disease can be determined for a subject. For example, the classification can include selecting a disease classification of a particular reference vector determined to have the shortest distance to the vector of N frequencies.

In some embodiments, end-motif frequencies of cell-free DNA molecules having different size ranges are used to determine a classification of the disease. For one or more sequence motifs (e.g., CCCA), a first motif frequency can be determined for cell-free DNA molecules in first size range (e.g., sizes greater than 1-kb), and a second motif frequency can be determined for cell-free DNA molecules in a second size range (e.g., sizes less than 200 bp). A separation value (e.g., a ratio value) can be determined based on the first motif frequency and the second motif frequency. The separation value can be used to determine the classification of the disease. For example, the separation value can be compared to a cutoff value determined using a reference sample with known classification of the disease. In another example, the separation value can be processed using a machine-learning model to determine the disease classification, in which the machine-learning model (e.g., logistic regression, support vector machine) was trained using training samples with know classifications of the disease.

In some embodiments, a machine-learning model is trained using various features of a training dataset to differentiate reads from first tissue and other tissues. Based on the differentiation, cancer classification can be determined. Sequence reads can be obtained from a plasma DNA sample. In some instances, at least some of the sequence reads have a length greater than a threshold size (e.g., 600 bp). For each sequence read, one or more features are determined. The one or more features can include, for the sequence read, a location of end in a reference genome, sequence context, size, sequence motif at one or more ends, or a DNA methylation pattern. The features can be inputted into the trained machine-learning model. The machine-learning model can generate an output, which can be used to determine a classification for the sequence read. The classification can identify whether the sequence read is derived from a first tissue type or another tissue type. The classifications for the sequence reads can then be used to determine a classification of the disease. For example, an amount of sequence reads classified as being derived from the first tissue type can be determine, and the amount can be used to determine the disease classification.

In some embodiments, single molecule methylation level of cell-free DNA molecules are used to determined a level of pathology for a subject. For example, a percentage of methylated sites is determined for each DNA molecule of a plurality of cell-free DNA molecules. In some instances, the plurality of cell-free DNA molecules have sizes above a threshold (e.g., 500 bp). The determined percentages of methylated sites for the plurality of cell-free DNA molecules can be used to determine a statistical value (e.g., an average, a media). The statistical value can be compared to a reference to determine a pathology.

The analysis of long cell-free DNA molecules can provide added value to cancer detection and assessment that has previously been unexplored. The methylation pattern, profile or haplotype of longer cell-free DNA molecules can be more specific than short molecules due to the presence of higher numbers of CpG sites. Hence, the permutations in the order of methylated and unmethylated sites would be much greater. This would allow improved identification of DNA molecules originating from any particular tissues, aka tissue of origin analysis.

Such a tissue-of-origin analysis would distinguish from previously known techniques that use short-read sequencing to analyze short cell-free DNA molecules. Due to the limited number of CpG sites on short cell-free DNA molecules, previous methods used population statistics on a population/plurality of short cell-free DNA molecules to assemble the methylation profile of the cell-free DNA content in the plasma sample. This approach only allowed one to deduce the relative contributions of cell-free DNA molecules originating from a range of tissues or organs. With the DNA methylation pattern specificity conferred by the higher number of CpG sites on long cell-free DNA molecules, we believe determination of the tissue of origin of such an individual long cell-free DNA molecule would be feasible. In other words, individual molecules could be assigned to a tissue or organ of origin.

Another projected advantage of analyzing long cell-free DNA molecules would be the potential ability to link a sequence variant on the molecule with the adjacent CpG methylation information on the same molecule. Indeed, the analysis of long cell-free DNA molecules would allow one to analyze two or more molecular (e.g. genetic or epigenetic) characteristics on such molecules. Examples include (i) two or more sequence variations (e.g. point mutations, microsatellite variations, etc), (ii) two or more epigenetic variations (e.g. two or more hyper- or hypo-methylated CpG sites) and (iii) different combinations of genetic and epigenetic changes. In addition, because malignant tumors are known to have higher cell death rate, the abundance of long cell-free DNA molecules released from tumors may be different from non-tumor tissues. A number of approaches for analyzing long cell-free DNA fragments have been invented in this disclosure for enabling the detection and monitoring of cancers and many other diseases, including but not limited to autoimmune diseases, organ transplant rejection, trauma, ischemia, necrosis, etc. In some embodiments, the approaches present in this disclosure could be used for prognosis, risk stratification, treatment guidance, etc.

### I. TECHNIQUES FOR ANALYZING LONG CELL-FREE DNA MOLECULES

Cell-free DNA were obtained from the plasma samples of patients with cancer and subjects without cancer. Such cell-free DNA was subjected to single molecule sequencing for various analyses, including but not limited to methylation haplotype analysis, tissue of origin of individual plasma DNA molecule, fragment size profiling, plasma DNA end analysis, jagged end analysis, microsatellite instability, etc. Information about techniques for identifying various features of long cell-free DNA molecules (e.g., methylation status, jagged ends) are further described in U.S. Patent Application Serial No. 16/995,607,

### A. Detecting long cell-free DNA molecules in a biological sample

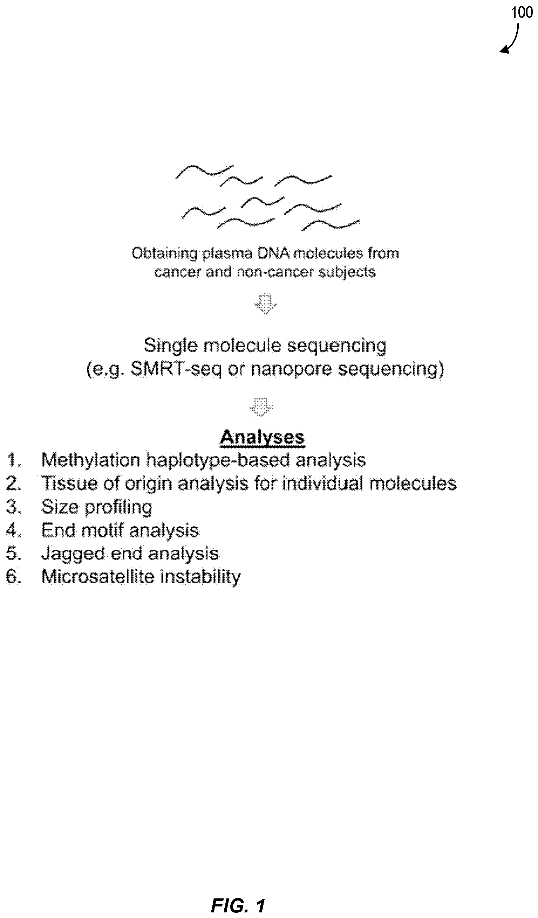

FIG. 1 shows a schematic diagram 100 that illustrates an example overview of analyzing long cell-free DNA molecules, according to some embodiments. In one example, the analysis may include sequencing, e.g., single module sequencing. Single molecule sequencing may include, but not limited to, single molecule real-time sequencing (i.e. SMRT-seq) (e.g. from Pacific Biosciences, PacBio SMRT-seq) and nanopore sequencing (e.g. from Oxford Nanopore Technologies). The nucleotides for each sequenced DNA molecule can be identified according to the electrical or optical signals produced during the sequencing processes. The identified nucleotides can be used for subsequent analysis of the corresponding long cell-free DNA molecules. Additionally or alternatively, other sequencing techniques can be used to detect the long cell-free DNA. For example, cluster-based sequencing can include sequencing each end (e.g., 200 bp or more) of a given fragment, thereby producing a sequence read of identified nucleotide sequences (e.g., 400 bp or more).

For example, the length of a plasma DNA could be determined by counting the number of nucleotides present in a sequence. The 4-mer end motifs of a plasma DNA could be determined by analyzing the 4 nucleotides at its ends. Similarly, in some embodiments, other types of end motifs could be used, including but not limited to, 1-mer, 2-mer, 3-mer, 5-mer, 6-mer, 7-mer, 8-mer, 9-mer, 10-mer, 15-mer, 20-mer, or other combinations.

In some embodiments, the analysis of plasma DNA molecules between cancer and non-cancer subjects could also involve jagged ends (i.e. the original double-stranded carrying a single-stranded protruding end(s)) and microsatellite instability. Microsatellite instability refers to a genomic alteration in which microsatellites, usually of one to six nucleotide repeats, accumulate mutations corresponding to deletions/insertions of one or more nucleotides.

In some embodiments, using PacBio SMRT-seq, the methylation status, across a series of CpG sites in a plasma DNA molecule, could be determined by analyzing the DNA polymerase kinetic signals in a measurement window according to, but not limited to, the previously published approach (Tse et al. Proc Natl Acad Sci USA. 2021;118: e2019768118). Additionally or alternatively, using nanopore sequencing, the methylation status, across a series of CpG sites in a plasma DNA molecule, could be determined by analyzing electrical signals depending on a DNA molecule passing through a nanopore according to, but not limited to, tools present in U.S. Application No. 63/173,728, the published approaches such as the open-source software Nanopolish (Simpson et al. Nat Methods. 2017; 14:407-410), DeepMod (Liu et al. Nat Commun. 2019;10:2449), Tombo (Stoiber et al. BioRxiv. 2017:p.094672), DeepSignal (Ni et al. Bioinformatics. 2019;35:4586-4595), Guppy (github.com/nanoporetech), Megalodon (github.com/nanoporetech/megalodon), etc. In some embodiments, the methylation patterns can be obtained with the treatment of chemical conversion (e.g. bisulfite) or enzymatic conversion (e.g. TET2 and APOBEC) followed by PacBio SMRT-seq and/or nanopore sequencing. The enzymatic conversion would convert the unmethylated cytosines to uracils, amplified and sequenced as thymines, whereas leaving the methylated cytosines unchanged. Thus, the methylation status could be determined by the detection of thymines (unmethylated signal) or cytosines (methylated signal) across the CpG sites in a reference genome.

In some embodiments, the sequenced reads are aligned to a human reference genome using Minimap2 (Li H. Bioinformatics. 2018;34(18):3094-3100). In some embodiments, BLASR (Mark J Chaisson et al. BMC Bioinformatics. 2012; 13: 238), BLAST (Altschul SF et al. J Mol Biol. 1990;215(3):403-410), BLAT (Kent WJ. Genome Res. 2002; 12(4):656-664), BWA (Li H et al. Bioinformatics. 2010;26(5):589-595), NGMLR (Sedlazeck FJ et al. Nat Methods. 2018; 15(6):461-468), and LAST (Kielbasa SM et al. Genome Res. 2011;21(3):487-493) are used for aligning sequenced reads to a reference genome. In some embodiments, the alignment of sequenced reads is not used.

### B. Determining methylation status of long cell-free DNA molecules

As described herein, the methylation status across CpG sites can be obtained by analyzing the kinetic features produced during SMRT sequencing. For example, using Pacific Biosciences SMRT sequencing as an example of single molecule, real-time sequencing for illustration purposes, a DNA polymerase molecule is positioned at the bottom of wells that serve as zero-mode waveguides (ZMW). The ZMW is a nanophotonic device for confining light to a small observation volume, which can be a hole whose diameter is very small and does not allow the propagation of light in the wavelength range used for detection such that only emission of optical signals from dye-labeled nucleotide incorporated by the immobilized polymerase are detectable against a low and constant background signal (Eid *et al*., 2009). The DNA polymerase catalyzes the incorporation of fluorescently labeled nucleotides into complementary nucleic acid strands.

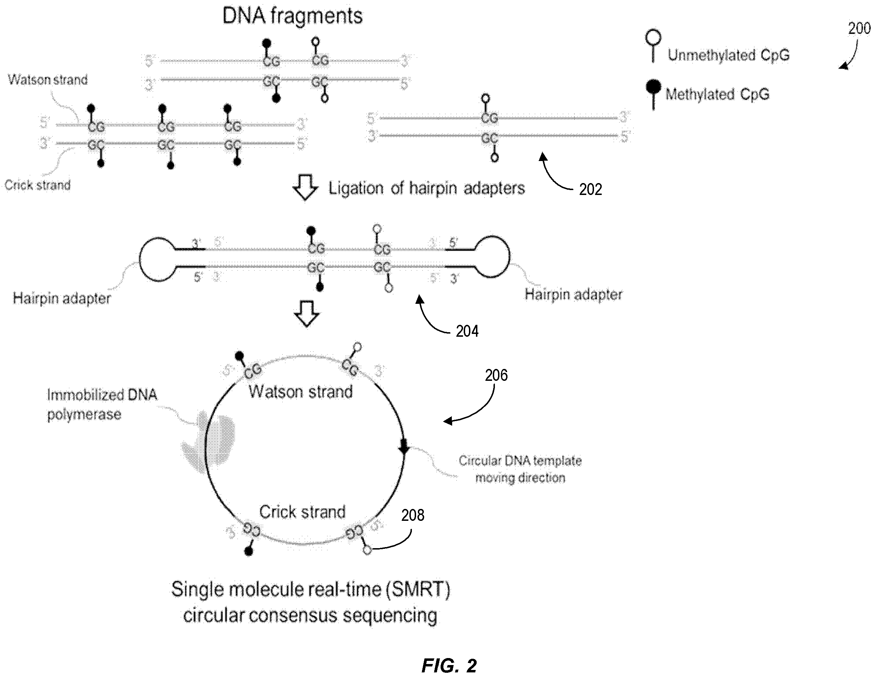

FIG. 2 shows an example of molecules 200 carrying methylated and/or unmethylated CpG sites that were sequenced by single molecule, real-time sequencing. DNA molecules were first ligated with hairpin adapters to form circularized molecules which would bind to immobilized DNA polymerase and to initiate the DNA synthesis. In FIG. 2, DNA molecule 202 is ligated with hairpin adapters to form ligated molecule 204. Ligated molecule 204 then forms circularized molecule 206. The molecules without CpG sites can also be sequenced. Circularized molecule 206 includes an unmethylated CpG site 208, which may still be sequenced.

Once the methylation status across CpG sites in a plasma DNA molecule has been determined (herein referred to as methylation haplotype), one could compare the methylation haplotype of plasma DNA molecules with the methylation haplotypes of different tissues to determine the tissue of origin of that plasma DNA molecule. In other words, the methylation haplotype was defined as the methylation patterns across one or more CpG sites in a single DNA molecule. For example, '-M-U-M-M-M-' represented a methylation haplotype, showing methylated CpG followed by unmethylated CpG followed by three consecutive methylated CpG sites. The methylation haplotype information of '-M-U-M-M-M-' and '-M-U-M-M-U-' were different. The aforementioned tissues could include, but not limited to, neutrophils, T cells, B cells, megakaryocytes, erythrocytes, monocytes, NK cells, liver, lungs, esophagus, heart, pancreas, colon, small intestines, adipose tissues, adrenal glands, brain, breast, kidney, bladder, thyroid, prostate, uterus, etc. The tissues could involve cancers, such as but not limited to, bladder cancer, breast cancer, colon and rectal cancer, endometrial cancer, kidney cancer, leukemia, liver cancer, lung cancer, melanoma, non-Hodgkin lymphoma, pancreatic cancer, prostate cancer, thyroid cancer, etc.

### 1. Kinetic features

Some embodiments of methods described in this disclosure are based on measuring and utilizing interpulse duration (IPD), pulse widths (PW), and sequence context for every base within the measurement window. We reasoned that if we can use a combination of multiple metrics, for example, concurrently making use of features including upstream and downstream sequence context, strand information, IPD, pulse widths as well as pulse strength, we might be able to achieve the accurate measurement of base modifications (e.g. mC detection) at single-base resolution. Sequence context refers to the base compositions (A, C, G, or T) and the base orders in a stretch of DNA. Such a stretch of DNA could be surrounding a base that is subjected to or the target of base modification analysis. In one embodiment, the stretch of DNA could be proximal to a base that is subjected to base modification analysis. In another embodiment, the stretch of DNA could be far away from a base that is subjected to base modification analysis. The stretch of DNA could be upstream and/or downstream of a base that is subjected to base modification analysis.

In one embodiment, the features of upstream and downstream sequence context, strand information, IPD, pulse widths as well as pulse strength, which are used for base modification analysis, are referred to as kinetic features.

Techniques to detect modifications in bases without enzymatic or chemically converting the modification and/or the base are desired. As described herein, modifications in a target base may be detected using kinetic feature data obtained from single molecule, real-time sequencing for bases surrounding the target base. Kinetic features may include interpulse duration, pulse width, and sequence context. These kinetic features may be obtained for a measurement window of a certain number of nucleotides upstream and downstream of the target base. These features (e.g., at particular locations in the measurement window) can be used to train a machine learning model. As an example of the sample preparation, the two strands of a DNA molecule may be connected by hairpin adapters, thereby forming a circular DNA molecule. The circular DNA molecule allows for kinetic features to be obtained for either or both of the Watson and Crick strands. A data analysis framework can be developed based on the kinetic features in the measurement windows. This data analysis framework may then be used to detect modifications, including methylation. The section describes various techniques for detecting modifications.

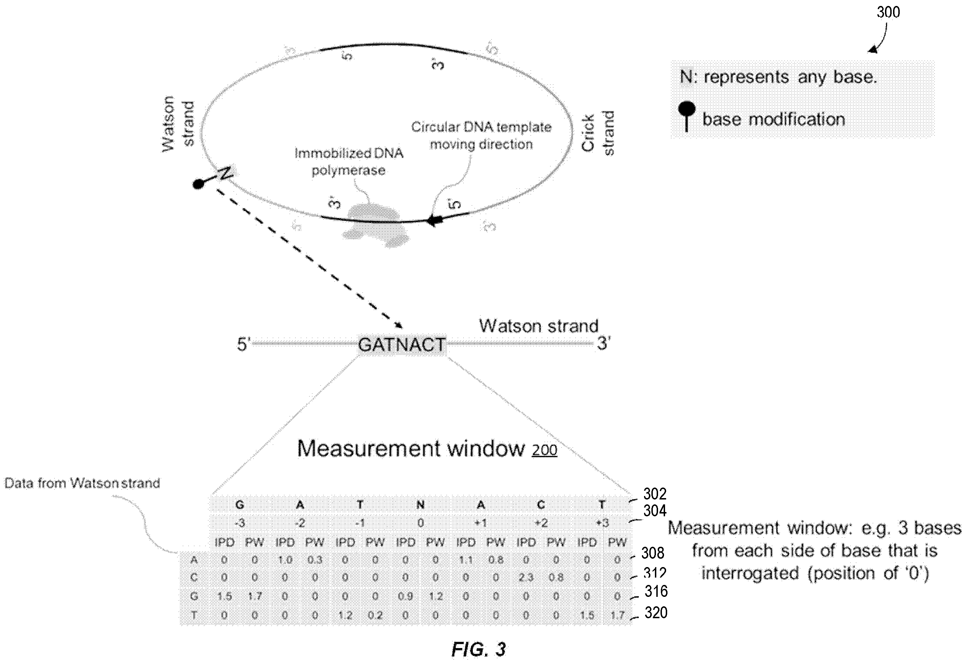

FIG. 3 shows a schematic diagram 300 illustrating an example process for determining kinetic features of cell-free DNA molecules, according to some embodiments. As shown in FIG. 3, as an example, we obtained the subreads of the Watson strand from Pacific Biosciences SMRT sequencing to analyze one particular base regarding the states of base modifications. In FIG. 3, the 3 bases from each side of a base that was subjected to base modification analysis would be defined as a measurement window 300. In one embodiment, sequence context, IPDs, and PWs for these 7 bases (i.e. 3-nucleotide (nt) upstream and downstream sequence and one nucleotide for base modification analysis) were compiled into a 2-dimensional (i.e. 2-D) matrix as a measurement window. In the example shown, the measurement window 300 is for one subread of the Watson strand. Other variations are described herein.

The first row 302 of the matrix indicated the sequence that was studied. In the second row 304 of the matrix, the position of 0 represented the base for base modification analysis. The relative positions of -1, -2, and -3 indicated the position 1-nt, 2-nt, and 3-nt, respectively, upstream of the base that was subjected to base modification analysis. The relative positions of +1, +2, and +3 indicated the position 1-nt, 2-nt and 3-nt, respectively, downstream of the base that was subjected to base modification analysis. Each position includes 2 columns, which contain the corresponding IPD and PW values. The following 4 rows (rows 308, 312, 316, and 320) corresponded to 4 types of nucleotides (A, C, G, and T) in the strand (e.g. Watson strand), respectively. The presence of IPD and PW values in the matrix depended on which corresponding nucleotide type was sequenced at a particular position. As shown in FIG. 3, at the relative position of 0, the IPD and PW values were shown in the row indicating 'G' in the Watson strand, suggesting that a guanine was called in the sequence result at that position. The other grids in a column that did not correspond to a sequenced base would be coded as '0'. As an example, the sequence information corresponding to the 2-D digital matrix (FIG. 3) would be 5'-GATGACT-3' for the Watson strand.

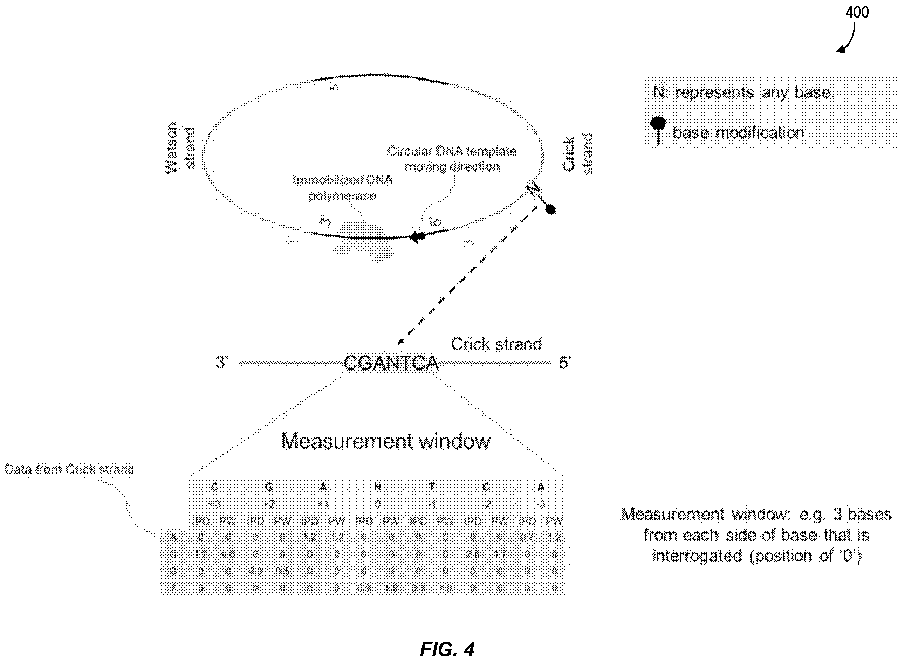

FIG. 4 shows a schematic diagram 400 illustrating another example process for determining kinetic features of cell-free DNA molecules, according to some embodiments. As shown in one embodiment depicted in FIG. 4, the measurement window could be applied to data from the Crick strand. We obtained the subreads of the Crick strand from single molecule, real-time sequencing to analyze one particular base regarding the states of base modifications. In FIG. 4, the 3 bases from each side of a base that was subjected to base modification analysis and the base subjected to base modification analysis would be defined as a measurement window. In one embodiment, sequence context, IPDs, PWs for these 7 bases (i.e. 3-nucleotide (nt) upstream and downstream sequence and one nucleotide for base modification analysis) were compiled into a 2-dimensional (i.e. 2-D) matrix as a measurement window.

The first row of the matrix indicated the sequence that was studied. In the second row of the matrix, the position of 0 represented the base for base modification analysis. The relative positions of -1, -2, and -3 indicated the position 1-nt, 2-nt and 3-nt, respectively, upstream of the base that was subjected to base modification analysis. The relative positions of +1, +2, and +3 indicated the position 1-nt, 2-nt and 3-nt, respectively, downstream of the base that was subjected to base modification analysis. Each position includes 2 columns, which contained the corresponding IPD and PW values. The following 4 rows corresponded to 4 types of nucleotides (A, C, G, and T) in this strand (e.g. the Crick strand). The presence of IPD and PW values in the matrix depended on which corresponding nucleotide type was sequenced at a particular position. As shown in FIG. 4, at the relative position of 0, the IPD and PW values were shown in the row indicating 'T' in the Crick strand, suggesting that a thymine was called in the sequence result at that position. The other grids in a column that did not correspond to a sequenced base would be coded as '0'. As an example, the sequence information corresponding to the 2-D digital matrix (FIG. 4) would be 5'-ACTTAGC-3' for the Crick strand.

### 2. Machine-learning model

For the machine learning model, input data structures of subreads can be used for the training. The input data structure may correspond to a window of nucleotides sequenced in a sample nucleic acid molecule. The training set can have sites with known methylation status. Each training sample can include one of the first plurality of first data structures and a label indicating the first state for the modification (e.g., methylation) of the nucleotide at the target position. The training is performed by optimizing parameters of the model based on outputs of the model matching or not matching corresponding labels of the first labels and optionally the second labels when the first plurality of first data structures and optionally the second plurality of second data structures are input to the model. An output of the model specifies whether the nucleotide at the target position in the respective window has the modification. In some embodiments, the output of the model may include a probability of being in each of a plurality of states. The state with the highest probability can be taken as the state.

The model may include a convolutional neural network (CNN). The CNN may include a set of convolutional filters configured to filter the first plurality of data structures and optionally the second plurality of data structures. The filter may be any filter described herein. The number of filters for each layer may be from 10 to 20, 20 to 30, 30 to 40, 40 to 50, 50 to 60, 60 to 70, 70 to 80, 80 to 90, 90 to 100, 100 to 150, 150 to 200, or more. The kernel size for the filters can be 2, 3, 4, 5, 6, 7, 8, 9, 10, 11, 12, 13, 14, 15, from 15 to 20, from 20 to 30, from 30 to 40, or more. The CNN may include an input layer configured to receive the filtered first plurality of data structures and optionally the filtered second plurality of data structures. The CNN may also include a plurality of hidden layers including a plurality of nodes. The first layer of the plurality of hidden layers coupled to the input layer. The CNN may further include an output layer coupled to a last layer of the plurality of hidden layers and configured to output an output data structure. The output data structure may include the properties.

The model may include a supervised learning model. Supervised learning models may include different approaches and algorithms including analytical learning, artificial neural network, backpropagation, boosting (meta-algorithm), Bayesian statistics, case-based reasoning, decision tree learning, inductive logic programming, Gaussian process regression, genetic programming, group method of data handling, kernel estimators, learning automata, learning classifier systems, minimum message length (decision trees, decision graphs, etc.), multilinear subspace learning, naive Bayes classifier, maximum entropy classifier, conditional random field, Nearest Neighbor Algorithm, probably approximately correct learning (PAC) learning, ripple down rules, a knowledge acquisition methodology, symbolic machine learning algorithms, subsymbolic machine learning algorithms, support vector machines, Minimum Complexity Machines (MCM), random forests, ensembles of classifiers, ordinal classification, data pre-processing, handling imbalanced datasets, statistical relational learning, or Proaftn, a multicriteria classification algorithm The model may linear regression, logistic regression, deep recurrent neural network (e.g., long short term memory, LSTM), Bayes classifier, hidden Markov model (HMM), linear discriminant analysis (LDA), k-means clustering, density-based spatial clustering of applications with noise (DBSCAN), random forest algorithm, support vector machine (SVM), or any model described herein.

### II. FREQUENCY-BASED ANALYSIS OF LONG CELL-FREE DNA MOLECULES

An amount of long cell-free DNA molecules present in plasma may depend on a disease state of a particular subject. For example, a first amount of long cell-free DNA molecules present in a biological sample of a subject with hepatocellular carcinoma (HCC) can be less than a second amount of long cell-free DNA molecules present in a biological sample of another subject who is a Hepatitis B virus (HBV) carrier. As such, long cell-free DNA molecules of HCC patients and HBV carriers can be sequenced using single molecule real-time sequencing (e.g., via PacBio sequencer) to identify these amount-based characteristics.

In some embodiments, a long DNA molecule is defined as a DNA molecule having a length equal to or greater than 500 bp, 600 bp, 1 kb, 2 kb, 3 kb, 4 kb, 5 kb, 10 kb, or above 10 kb. In some instances, the long DNA molecule is defined to have a size within a size range. The size range can include a lower bound and an upper bound. The lower bound identifies a minimum size of the cell-free DNA molecule to be considered as a long DNA molecule. For example, the lower bound of the size range includes at least 200 bps, at least 300 bps, at least 400 bps, at least 500 bps, at least 600 bps, at least 700 bps, at least 800 bps. Conversely, the upper bound identifies a maximum size of the cell-free DNA molecule to be considered as a long DNA molecule. For example, the upper bound of the size range includes at least 500 bp, 600 bp, 1 kb, 2 kb, 3 kb, 4 kb, 5 kb, 10 kb, or above 10 kb. In some instances, the size range only specifies the lower bound and does not specify the upper bound. The above lengths are non-limiting and other types of lengths can be considered.

### A. Comparison between frequency-based analyses using long cell-free DNA molecules and frequency-based analyses using short cell-free DNA molecules

Plasma DNA samples from 5 patients with chronic hepatitis B infection (HBV carriers) and 19 patients with HCC were subjected to single - molecule real - time (SMRT) sequencing template construction using a SMRTbell Express Template Prep Kit 2.0 (Pacific Biosciences). DNA was purified with 1.8 × AMPure PB beads, and library size was estimated using a TapeStation instrument (Agilent). Sequencing primer annealing and polymerase binding conditions were calculated with the SMRT Link v10.1 software (Pacific Biosciences). Briefly, sequencing primer v4 was annealed to the sequencing template, and then polymerase was bound to templates using a Sequel II Binding Kit 2.1 and Internal Control Kit 1.0 (Pacific Biosciences). Sequencing was performed on a SMRT Cell 8M. Sequencing movies were collected for 30 hours with a Sequel II Sequencing 2.0 Kit (Pacific Biosciences). We obtained a median of 314,477 sequenced reads (interquartile range (IQR): 128,791 - 561,018). The DNA methylation status across CpG sites in a plasma DNA molecule was determined according to the HK model (Tse et al. Proc Natl Acad Sci USA. 2021; 118; e2019768118). For comparison, short-read sequencing (e.g., Illumina sequencing) was performed on the same plasma DNA samples. Length of each sequence read was determined for sequence reads corresponding to the HCC samples. Size distribution of the sequence reads having a length above 500 bp was identified for each of the Illumina sequencer results and the SMRT sequence results.

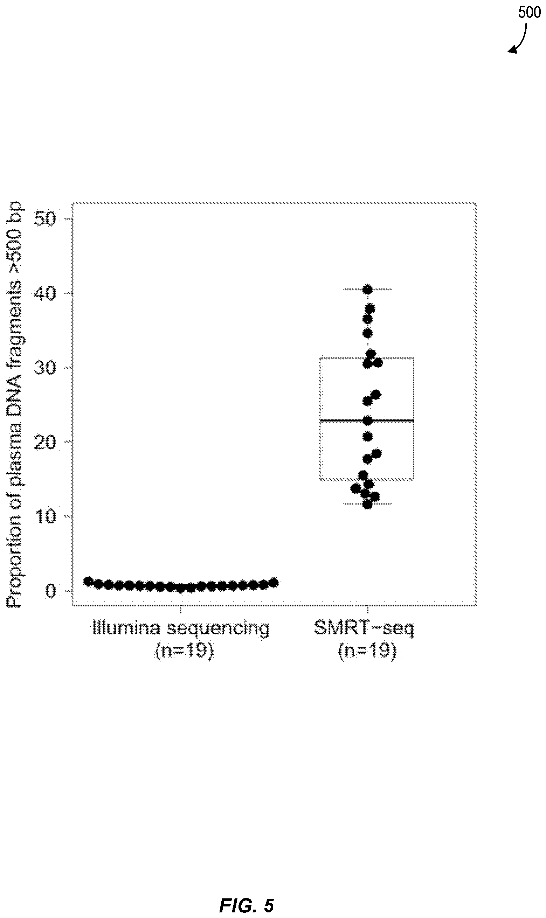

FIG. 5 shows a graph 500 that identifies proportions of plasma DNA fragments having a length greater than 500 bp across different sequencing techniques, according to some embodiments. FIG. 5 shows that the proportion of plasma DNA fragments > 500 bp in patients with HCC was much higher in single molecule real-time sequencing (SMRT-seq) results (median: 22.88%; range: 11.64% - 40.46%) than in Illumina sequencing results (median: 0.68%; range: 0.34% - 1.24%) (P value < 0.0001, Mann-Whitney U test). The data suggested that there were a substantial amount of long plasma DNA molecules that were not able to be explored based on Illumina sequencing.

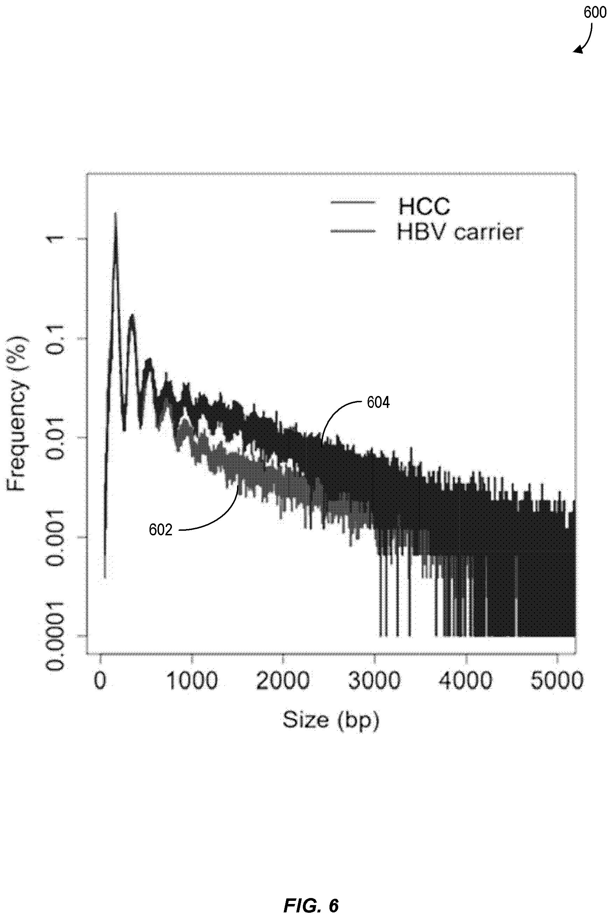

FIG. 6 shows a line graph 600 that illustrates size distribution of one HCC subject 602 and one HBV carrier 604. SMRT-seq was used to generate the sequence reads for each of the samples. Y-axis corresponds to frequency values shown on a logarithmic scale (e.g., a normalized parameter of size distribution). Both size profiles displayed multiple nucleosome-sized peaks, locating at 166 bp, 333 bp, 500 bp, 663 bp, 830 bp, 994 bp, etc. The long DNA frequencies longer than 1 kb appeared to decline faster in the patient with HCC than in the HBV carrier. In some embodiments, one could use the alterations in size profiles to differentiate patients with and without HCC.

The above results indicate that the size distribution of tumor samples can be used for disease classification. In some instances, a cutoff is determined for classifying whether a sample includes cancer. The cutoff can correspond to a normalized parameter that represents a particular amount or frequency of cell-free DNA molecules having equal or greater than a certain length (e.g., 600 bp).

### B. Predicting histological status of a disease based on frequencies of long DNA molecules

Vascular invasion in HCC is a prerequisite for systemic tumor dissemination and is the best predictor for tumor recurrence after transplantation or tumor resection (Thuluvath. J. Clin. Gastroenterol. 2009;43:101-2). While some studies suggested that circulating plasma DNA concentration is correlated with vascular invasion status (Huang et al. Pathol. Oncol. Res. 2012; 18: 271-276) and tumor-associated mutations (Oversoe et al. Scand. J. Gastroenterol. 2020;55:1433-1440; Liao et al. Oncotarget. 2016; 7:40481-40490), it is not known whether the size features of cfDNA are associated with vascular invasion.

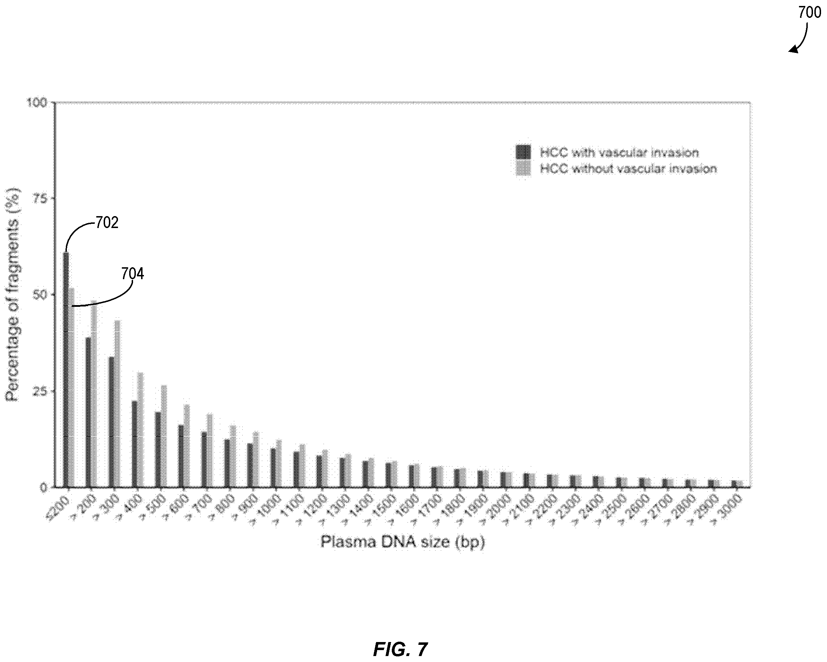

To explore the size features associated with vascular invasion in DNA molecules, we studied the plasma DNA in HCC patients using single-molecule real-time sequencing. In our cohort, 18 patients were with vascular invasion while 27 patients were without vascular invasion. FIG. 7 shows a bar graph 700 that identifies percentages of cfDNA fragments above a given size for HCC patients with vascular invasion 702 and HCC patients without vascular invasion 704. Red bars indicates HCC cases with vascular invasion and cyan bars indicates HCC cases without vascular invasion. In addition, the x-axis shows the percentage of DNA molecules longer than a given size cut-off (e.g., 200 bps, 500 bps, 2 kbps). As shown in FIG. 7, the plasma DNA of subjects with vascular invasion had a shorter size distribution than those without vascular invasion, and this difference is apparent up to 2 kb in size, which could not be revealed by previous sequencing methods such as Illumina sequencing.

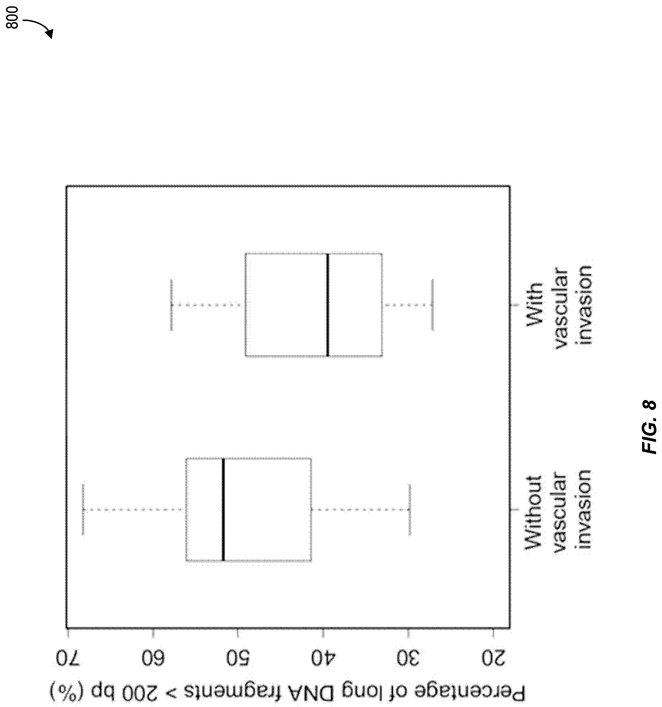

In some embodiments, the percentage of DNA fragments greater than a certain size (e.g., ≥200 bp, ≥ 500 bp, ≥ 600 bp, ≥ 1 kb, ≥ 2 kb, ≥ 3 kb, ≥ 4 kb, ≥ 5 kb, ≥ 10 kb, other combinations) could be used to predict the vascular invasion status of cancer patients non-invasively. FIG. 8 shows a boxplot 900 that identifies percentage of long DNA fragments >200 bp in HCC patients with and without vascular invasion. The long DNA molecules were identified using SMRT sequencing. As shown in FIG. 8, HCC patients with vascular invasion had a significantly lower percentage of long DNA fragments >200 bp (P value: 0.015, Mann-Whitney U-test), suggesting its potential use in predicting vascular invasion status of HCC patients. Further, the potential use in predicting vascular invasion status can enable assessment of recurrence risk and prognosis in a non-invasive manner.

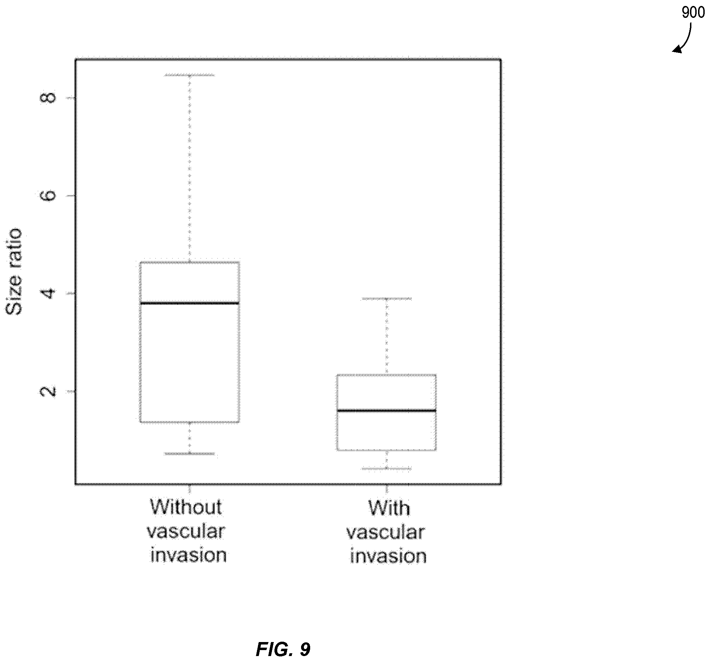

Additionally or alternatively, the size ratio between long and short DNA fragments could be used for vascular invasion status prediction in cancer patients non-invasively. FIG. 9 shows a boxplot 900 that identifies size ratios of HCC patients with and without vascular invasion. The size ratios of HCC patients with and without vascular invasion were calculated by dividing the proportion of long DNA fragments (>500 bp) by short DNA fragments (< 150 bp). As shown in FIG. 9, HCC patients with vascular invasion have a significantly lower size ratio than that of HCC patients without vascular invasion (P value: 0.004, Mann-Whitney U-test). The results of FIG. 9 show its potential use in predicting vascular invasion status of HCC patients and enabling assessment of recurrence risk and prognosis in a non-invasive manner.

### C. Methods for frequency-based analysis of long cell-free DNA molecules

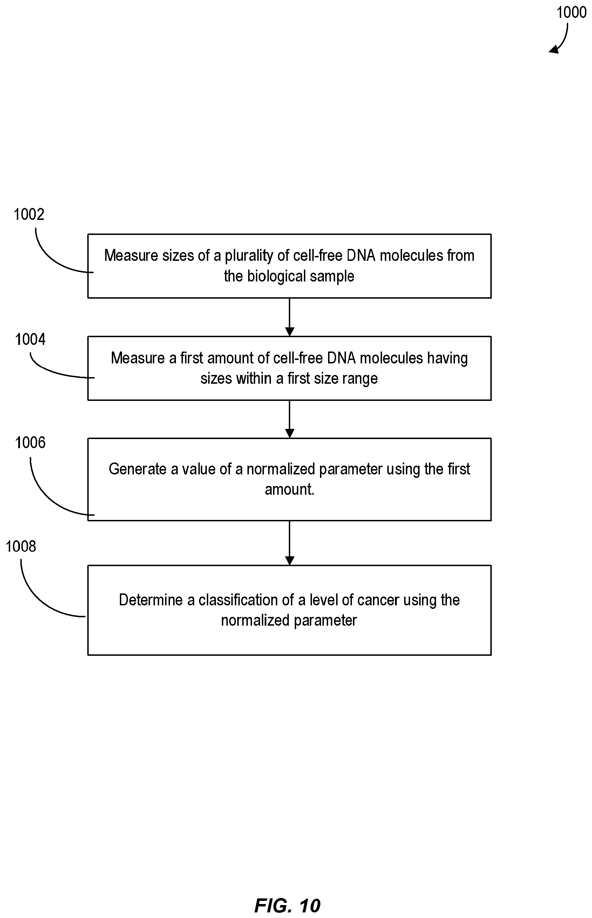

FIG. 10 shows a flowchart 1000 depicting an example process for analyzing a biological sample of a subject based on frequencies of long cell-free DNA molecules, according to some embodiments. The biological sample can include DNA originating from normal cells and potentially from cells associated with cancer. In addition, at least some of the DNA is cell-free in the biological sample.

At step 1002, sizes of a plurality of cell-free DNA molecules from the biological sample can be measured. For example, single molecule real-time sequencing (i.e. SMRT-seq) (e.g. from Pacific Biosciences, PacBio SMRT-seq) and nanopore sequencing (e.g. from Oxford Nanopore Technologies) can be used to identify and count the nucleotides of a cell-free DNA molecule. The number of nucleotides can be counted to determine the size of the cell-free DNA molecule. In other embodiments, sequence reads at each end of a DNA molecule can be sequenced, and the pair of reads can be aligned to a reference genome to determine the size of the DNA molecule.

At step 1004, a first amount of cell-free DNA molecules having sizes within a first size range can be measured. The first size range includes an upper bound of at least 1,000 bases, at least 3,000 bases, or above. In some instances, the first size range includes a lower bound that is greater than zero. The lower bound can be selected from one of at least 300 bases, at least 400 bases, at least 500 bases, at least 600 bases, or at least 800 bases. Accordingly, some example size ranges are: 300-1000 bp, 300-3000 bp, 300-800 bp, 400-800 bp, 400-1500 bp, and 500-3000 bp.

Additionally or alternatively, the first amount of cell-free DNA molecules can have ending sequences corresponding to one or more sequence motifs (e.g., CCCA). To determine the cell-free DNA molecules having the one or more sequence motifs, sequence reads are obtained from a sequencing of the plurality of cell-free DNA molecules from the biological sample. For each of the sequence reads, a sequence motif is determined for each of one or more ending sequences of a corresponding cell-free DNA molecule. A group of the plurality of cell-free DNA molecules that have at least one of a set of one or more sequence motifs in ending sequences can be determined. And, the first amount is of a subgroup of the group of the plurality of cell-free DNA molecules having the first size range.

At step 1006, a value of a normalized parameter can be generated using the first amount. The normalized parameter can be a frequency of the cell-free DNA molecules having sizes within the first size range in the biological sample. In some instances, the normalized parameter can be a frequency of the cell-free DNA molecules having sizes within the first size range in the biological sample that is normalized on a logarithmic scale. In other examples, a second amount of cell-free DNA molecules in a second size range can be used to normalize the first amount. The second size range can be different from the first size range. For example, the second size range can be less than the first size range (e.g., 1-150 bp).

At step 1008, a classification of a level of cancer can be determined using the normalized parameter. For example, the normalized parameter can be compared with a cutoff value. In some instances, the cutoff is determined for classifying whether a sample includes cancer. The cutoff can correspond to a normalized parameter that represents a particular amount or frequency of cell-free DNA molecules of a reference sample having equal or greater than a certain length (e.g., 600 bp), in which the reference sample is associated with a known classification of the level of cancer. The cutoff value or the comparison may be determined using machine learning with training data sets, e.g., using the training sample from FIG. 6.

Cutoff values and comparisons for other methods can also be determined using machine learning with training data sets. The comparison of the normalized parameter to the cutoff (reference) can involve a machine learning model, e.g., trained using supervised learning. In some instances, the cutoff values are determined using one or more training datasets comprising reference samples with known classifications of the levels of cancer. For example, the normalized parameter or separation value (and potentially other criteria, such as copy number, and methylation levels) and the known classifications of training subjects from whom training samples were obtained can form a training data set. Parameters of the machine learning model can be optimized based on the training set to provide an optimized accuracy in classifying the level of cancer. Example machine learning models include neural networks, decision trees, clustering, and support vector machines.

The level of cancer can include no cancer, early stage, intermediate stage, or advanced stage. The classification can then select one of the levels. Accordingly, the classification can be determined from a plurality of levels of cancer that include a plurality of stages of cancer. As examples, the cancer can be hepatocellular carcinoma, lung cancer, breast cancer, gastric cancer, glioblastoma multiforme, pancreatic cancer, colorectal cancer, nasopharyngeal carcinoma, and head and neck squamous cell carcinoma. Determining the disease classification can include a histological status of the cancer, e.g., whether vascular invasion exists.

### III. END-MOTIF ANALYSIS OF LONG AND SHORT CELL-FREE DNA MOLECULES

End motifs of cell-free DNA molecules of a biological sample can be identified and used for disease classification. In addition to the previous studies that it was feasible to use end motif signatures for cancer diagnosis in cfDNA molecules < 600 bp based on short-read sequencing (Illumina) (Jiang et al. Cancer Discov. 2020; 10:664-673), the end motif features in long cfDNA molecules can also be used for cancer diagnosis. In particular, analysis of end motifs, including but not limited to 1-mer, 2-mer, 3-mer, 5-mer, 6-mer, 7-mer, 8-mer, 9-mer, 10-mer, 15-mer, 20-mer, or other combinations, could be used to discriminate between subjects with and without cancer.

For each end motif of a set of end motifs, a relative frequency of sequences having the end motif of the biological sample can be determined. In some instances, the relative frequency of sequences having an end motif is determined based on other frequencies of the sequence motif in a group of reference samples. The relative frequencies of sequences for the set of end motifs can thereby form a vector of N frequencies for the biological sample, in which N corresponds to the number of end motifs in the set of end motifs. The vector of N frequencies of the biological sample can be compared to a plurality of reference vectors determined from the group of reference samples having a known classification of a disease (e.g., HCC). Based on the comparison, the classification of the disease can be determined for the biological sample.

### A. Cluster-based analysis

On the basis of hierarchical clustering analysis using 5' 4-mer end motif of plasma DNA molecules, patients with and without HCC tended to be grouped into different clusters. The plasma DNA molecules can be sequenced using single molecule sequencing (e.g., SMART-seq), such that the sequence reads include long cell-free DNA molecules. An end motif can be identified for each sequence read, and the relative frequencies of sequence reads can be determined for each type of end motif (e.g., CCGC). Biological samples corresponding to a disease classification share similar relative frequencies of sequence reads across different motifs, and can be grouped together to form a cluster. Such similar relative frequencies can suggest that the end motifs deduced from single molecule sequencing of plasma DNA could inform the presence or absence of cancer.

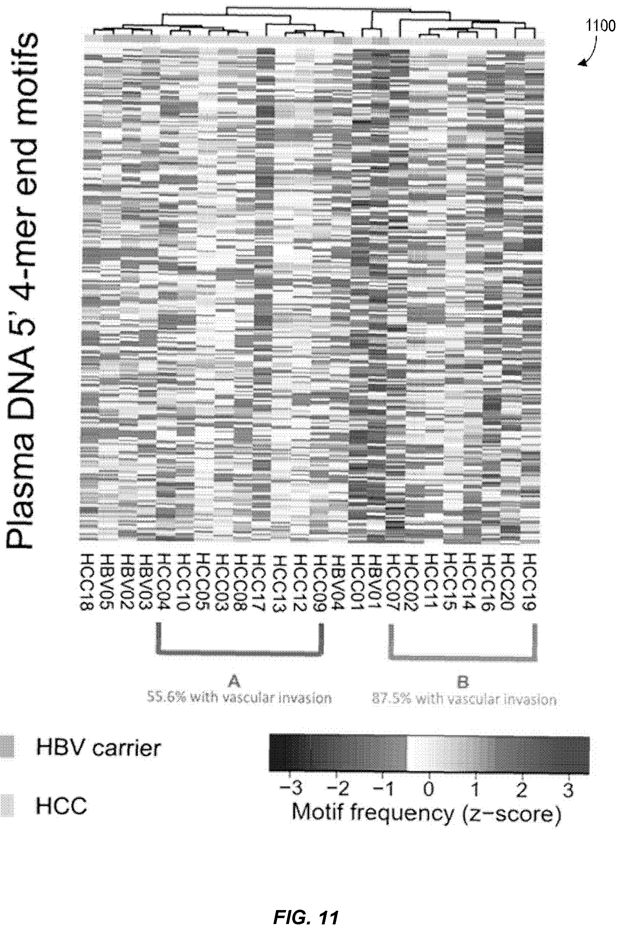

FIG. 11 shows a heat map 1100 generated based on a hierarchical clustering analysis of 256 4-mer end motifs of plasma DNA molecules, according to some embodiments. For example, a mean and standard deviation of frequencies of sequences across biological samples (e.g., the HCC samples, the HBV-carrier samples) can be determined for an end motif representing a row. Then, for a biological sample, a relative frequency of sequence reads having the end motif can be generated, in which the relative frequency can be based on the end-motif frequency of the sequence reads having the end motif being subtracted from the calculated mean then divided by the standard deviation. The result of the relative frequency of the end motif can then be indicated as a color-coded value on a corresponding row of the column representing the biological sample (e.g., HCC04) in the heat map 1100. The process can continue through other end motifs, such that an entire column of color-coded values of the heat map can be determined for the given sample.

In some instances, z-scores are used to indicate relative end-motif frequency of sequences from a sequencing of cell-free DNA molecules. A z-score can be a difference of the frequency for a particular end motif and a mean frequency (e.g., across samples for that given end motif) divided by a variation of the frequency (e.g., across samples for that given end motif). In FIG. 11, each row in the heatmap represented z-score values of frequency of a particular end motif across different training samples (e.g., HCC samples, HBV-carrier samples). The Z-score for a particular end motif can be calculated using the mean and standard deviation for the particular end motif among the training samples. The z-score can be used to virtualize the end motif frequency in different colors for more sharp comparisons.

The biological samples can be grouped based on their similarity in relative end-motif frequencies. As shown in FIG. 11, two clusters "A" and "B" can be formed. The subgroups A and B were associated with a low and high incidence of the histological status of vascular invasion. In particular, the "A" cluster identifies HCC samples in which 55.6% implicate vascular invasion, and the "B" cluster identifies HCC samples in which 87.5% implicate vascular invasion. Vascular invasion refers to a disease state in which tumor cells (e.g., ctDNA) are present in the lumen of blood and/or lymph vessel. Vascular invasion can also include extramural vascular invasion (EMVI), which involves direct invasion of a blood vessel (usually a vein) by a tumor. Vascular invasion can indicate a relatively more severe case of cancer. This was determined by examining the anatomical pathology reports.

Using these clusters as frequencies of reference samples, end-motif frequencies of a particular biological sample can be compared against the above reference samples to determine a disease classification.

These results suggested that the classification of histological status could be noninvasively enabled on the basis of the use of plasma DNA end motifs deduced by single molecule sequencing. Further, classification of vascular invasion can be clinically relevant for prognosis of patients, especially since vascular invasion involves more severe forms of the corresponding disease.

### B. Cluster-based analysis of end-motif frequencies of long DNA molecules

On the basis of hierarchical clustering analysis using 5' 4-mer end motifs, we analyzed the short DNA molecules (e.g., < 200 bp) and long DNA molecules (e.g., > 1kb) . In some embodiment, the combined analysis of short and long DNA molecules could concatenate the first vector containing frequencies of 256 motifs from long DNA molecules (e.g. > 1kb) and the second vector containing frequencies of 256 motifs from short DNA molecules (e.g.< 200 bp) into a new vector with a dimension of 512. Additionally or alternatively, the combined analysis of short and long DNA molecules could be a ratio of the first vector containing frequencies of 256 motifs from long DNA molecules (e.g. > 1kb) to the second vector containing frequencies of 256 motifs from short DNA molecules (e.g .< 200 bp). In some embodiments, the short and long DNA molecules are defined by different cut-offs. For example, the short DNA molecules can be defined by not limited to less than 50 bp, 60 bp, 70 bp, 80 bp, 90 bp, 100 bp, 110 bp, 120 bp, 130 bp, 140 bp, 150 bp, 160 bp, 170 bp, 180 bp, 190 bp, 200 bp, 250 bp, 300 bp, 400 bp, 500 bp, 600 bp, etc. The long DNA molecules can be defined by not limited to greater than 600 bp, 700 bp, 800 bp, 1 kb, 2 kb, 3 kb, 4 kb, 5 kb, 10 kb, 15 kb, 20 kb, 30 kb, 40 kb, 50 kb, etc.

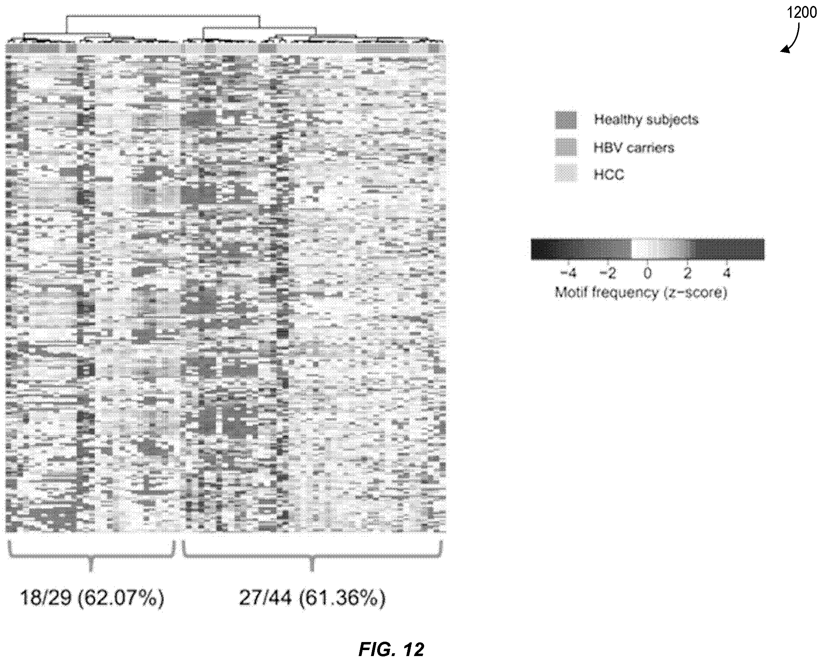

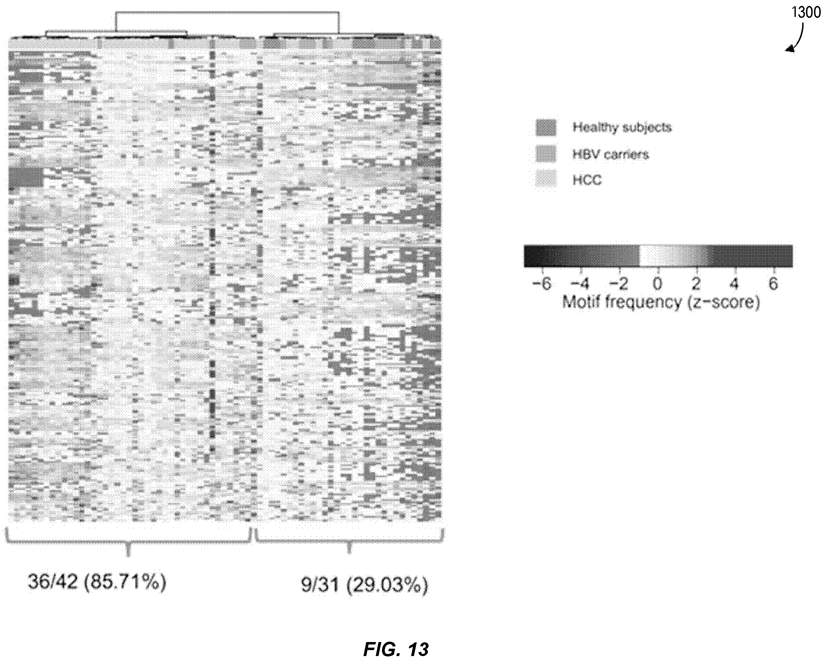

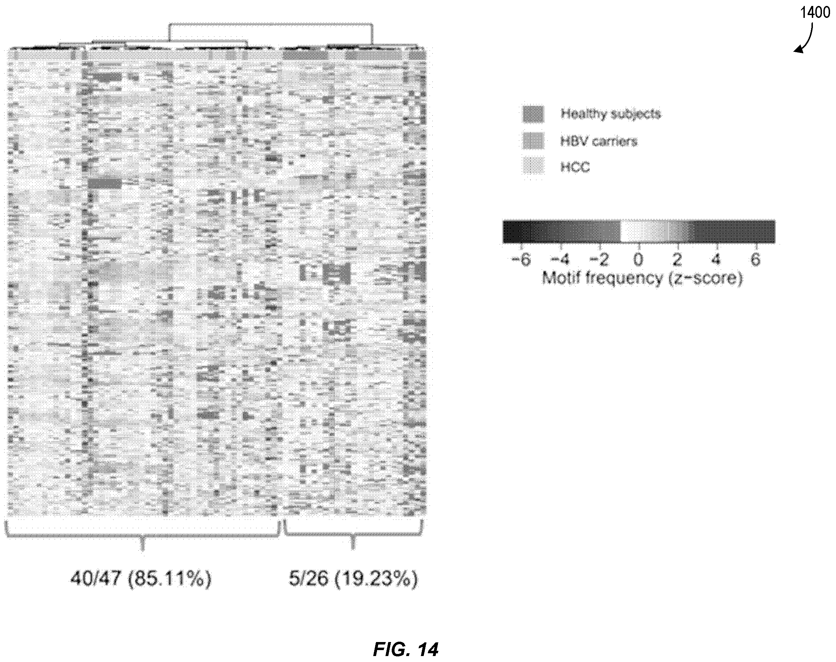

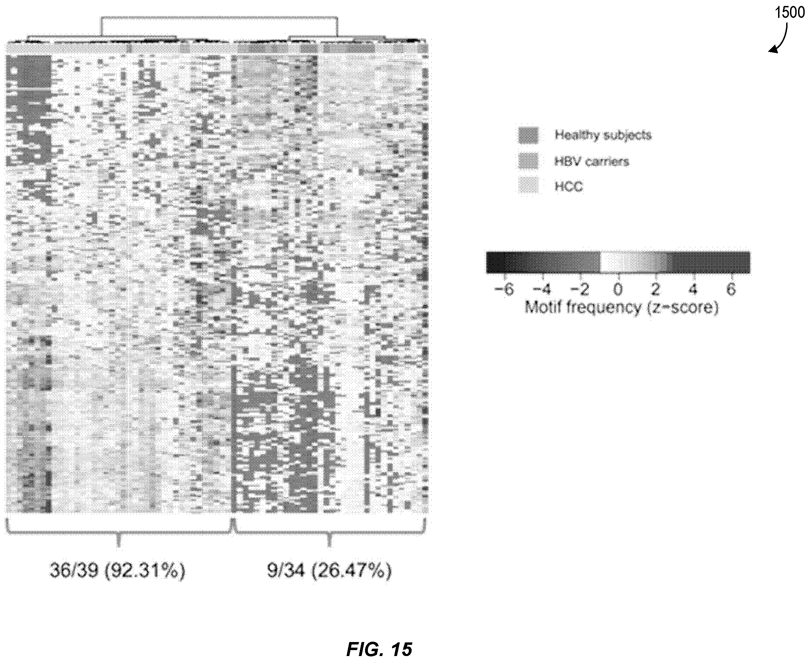

FIG. 12 shows a heatmap 1200 generated using a hierarchical clustering analysis of 4-mer end motifs of short plasma DNA (<200 bp), according to some embodiments. In addition, FIG. 13 shows a heatmap 1300 generated using a hierarchical clustering analysis of 4-mer end motifs of long plasma DNA (>1 kb), according to some embodiments. FIG. 14 shows a heatmap 1400 generated using a hierarchical clustering analysis of 4-mer end motifs of both short (<200 bp) and long plasma DNA (>1 kb), according to some embodiments. FIG. 15 shows a heatmap 1500 generated using a hierarchical clustering analysis of 4-mer end motifs ratios, according to some embodiment. As shown in FIGS. 12-15, each of the percentages shown on the bottom brackets indicates the percentage of HCC patients identified from the corresponding patient group.

Compared with the analysis based on short DNA molecules (FIG. 12), the differentiation power between HCC and non-HCC groups had been improved when using long DNA molecules (> 1 kb) (FIG. 13) and further enhanced in the combined analyses (FIGS. 14 and 15). The improvements can be demonstrated by the fact that clearer separations between patients with and without HCC were observed from FIGS. 13, 14, and 15 compared with FIG. 12. For example, as shown in FIG. 12, if one cut hierarchical clusters into two major groups, the percentages of HCC patients between groups were not able to be differentiated (62.07% vs. 61.36%) (P value: 1, Fisher's exact test) when using end motifs derived from short cfDNA molecules (e.g. < 200 bp). In contrast, as shown in FIG. 13, when using end motifs derived from long cfDNA molecules (e.g. > 1 kb), the percentages of HCC patients between groups became significantly different (85.71% vs. 29.03%) (P value: 1.577 x 10⁻⁶, Fisher's exact test). Further, when combining end motifs of short and long cfDNA molecules, the percentages of HCC patients between groups appeared more distinguishable as shown in FIG. 14 (85.11% vs. 19.23%) (P value: 3.51 x 10⁻⁸, Fisher's exact test) and FIG. 15 (92.31% vs. 26.47%) (P value: 5.121 x 10⁻⁹, Fisher's exact test).

### C. Methods for cluster-based analysis

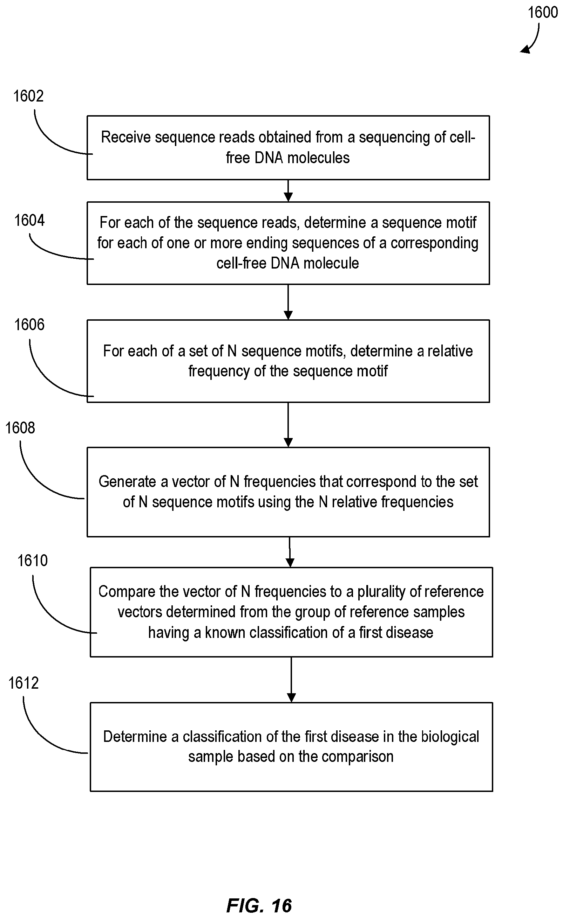

FIG. 16 shows a flowchart 1600 illustrating an example process for analyzing a biological sample of a subject based on relative frequencies of sequences having one or more end motifs, according to some embodiments. The biological sample can include DNA originating from normal cells and potentially from cells associated with a disease (e.g., a cancer). In addition, at least some of the DNA is cell-free in the biological sample.

At step 1602, sequence reads obtained from a sequencing of cell-free DNA molecules can be received. For example, single molecule real-time sequencing (i.e. SMRT-seq) (e.g. from Pacific Biosciences, PacBio SMRT-seq) and nanopore sequencing (e.g. from Oxford Nanopore Technologies) can be used to obtain the sequence reads from the biological sample. Other sequence techniques can be used, e.g., as described herein.

In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least 300 bases, at least 400 bases, at least 500 bases, at least 600 bases, or at least 800 bases.

Additionally or alternatively, a first set of the sequence reads can be selected from the sequence reads. The first set of the sequence reads can includes sizes within a first size range. Then, a second set of the cell-free DNA molecules can be selected from the sequence reads, in which the second set of sequence reads can include sizes within a second size range. In some instances, the second size range has an upper bound that is larger than the upper bound for the first size range. For example, the first size range can be less than 600 bp, and the second size range can be greater than 1000 bases. In some examples, the two size ranges can overlap, e.g., the first size range can be less than 800 bp and the second size range can be between 700 bp and 2000 bp.

At step 1604, for each of the sequence reads, a sequence motif for each of one or more ending sequences of a corresponding cell-free DNA molecule can be determined. For example, an 4-mer end motifs of the sequence read could be determined by analyzing the 4 nucleotides at its ends. Similarly, in some embodiments, other types of end motifs could be used, including but not limited to, 1-mer, 2-mer, 3-mer, 5-mer, 6-mer, 7-mer, 8-mer, 9-mer, 10-mer, 15-mer, 20-mer, or other combinations.

At step 1606, for each of a set of N sequence motifs, a relative frequency of the sequence motif can be determined. N relative frequencies can be determined. The relative frequency for an end motif can be the percentage of DNA molecules having that particular end motif. As another example, the relative frequency can be a ranking of the sequence motif, e.g., a ranking of the raw counts of DNA molecules (fragments) having that end motif. In some instances, the normalized frequency is a z-score, e.g., as described above for FIG. 11. As examples, N can be an integer equal to 2, 3, 4, 5, 8, 10, 15, 16, 20, 50, 64, 100, 128, 200, 256, or more, e.g., depending on the k-mer size of the end motif used.

In some instances, the relative frequency can be determined based on a proportion of cell-free DNA molecules that have ending sequences corresponding to the sequence motif relative to the cell-free DNA molecules from the biological sample. Alternatively, the relative frequency can be determined based on a proportion of cell-free DNA molecules that have ending sequences corresponding to the sequence motif relative to a number of cell-free DNA molecules that have ending sequence corresponding to other sequence motifs of the set of N sequence motifs.

At step 1608, a vector of N frequencies can be generated that correspond to the set of N sequence motifs using the N relative frequencies. Each of the N frequencies in the vector can be normalized to each other (e.g., as rankings) or to other frequencies of the sequence motif in a group of reference samples (e.g., as described above for the z-scores). The normalization of each frequency within a group for the reference samples can also be done using rankings. For example, the vector of N frequencies can be generated by normalizing the relative frequency of the sequence motif using the other frequencies of the sequence motif in the group of reference samples. In some instances, each frequency in the vector of N frequencies is determined by comparing the relative frequency to an average frequency for the sequence motif in the group of reference samples, e.g., to determine a z-score.

In some instances, the vector of N frequencies can be generated based on: (i) a first vector corresponding the N relative frequencies of the first set of sequence reads within the first size range; and (ii) a second vector corresponding the N relative frequencies of the sequence reads within the second sequence reads within second size range. Thus, the vector of N frequencies can be a value that identifies a correlation between short- (e.g., the first set of sequence reads) and long- (e.g., the second set of sequence reads) DNA molecules.

At step 1610, the vector of N frequencies can be compared to a plurality of reference vectors determined from the group of reference samples having a known classification of a disease. The comparison can include determining distances between the vector and the reference vectors. As examples, the reference vector can be of a particular reference sample or be representative of a group (cluster) of reference samples, e.g., a statistical value (such as an average, median, mean, or centroid) of the vectors of the group of reference samples.

At step 1612, a classification of the disease in the biological sample can be determined based on the comparison. In some instances, the classification can be determined using hierarchical clustering and/or heatmap clustering. Other machine learning techniques can also be used, e.g., neural networks, decision trees, and support vector machines.

In some instances, determining the classification of the disease includes identifying a classification associated with a cluster of reference vectors that are closest to the vector of N frequencies. For example, a first distance between the vector of N frequencies and a closest reference vector of a first cluster of reference vectors of the set of clusters can be determined. The first cluster of reference vectors represent a first subgroup of the group of reference samples classified as having the disease. A second distance between the vector of N frequencies and a closest reference vector of a second cluster of reference vectors of the set of clusters can also be determined. The second cluster of reference vectors represent a second subgroup of the group of reference samples classified as not having the disease. The first and second distance can then be compared. If the first distance is greater than the second distance, the subject can be determined as not having the disease. If the first distance is less than the second distance, the subject can be determined as having the disease.

### D. Rankings and separation values of short and long cell-free DNA molecules

In addition to using relative frequencies of various end motifs to determine a disease classification of a biological sample, a frequency of sequences having a particular end motif can be determined for each of size ranges of plasma DNA molecules of a biological sample. In some instances, a relative frequency of an end motif is determined based on a number of sequences having the end motif compared to numbers of sequences having other end motifs that can be found in the plasma DNA molecule. Additionally or alternatively, the relative frequency can be a percentage of sequences having the end motif relative to all sequences of the plasma DNA molecule. The frequency of sequences for each size range of DNA molecules can be used to determine a separation value. The separation value can then be used to determine a classification of a disease.

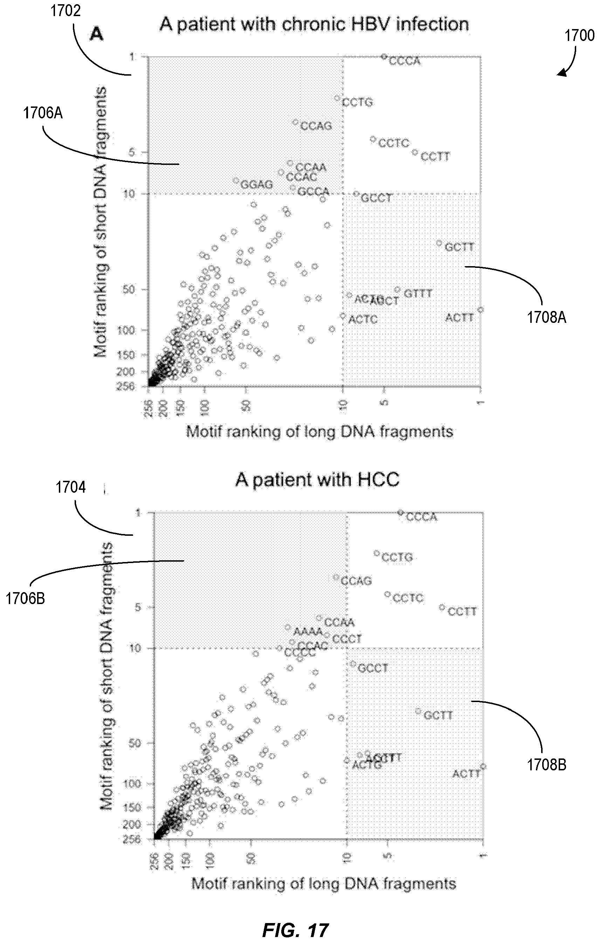

FIG. 17 shows a set of graphs 1700 that identify relationships of motif rankings between short plasma DNA molecules (< 600 bp) and long plasma DNA molecules (> 1 kb). In FIG. 17, each circle in the graphs represents a 4-mer end motif. The graph "A" identifies motif rankings of a subject with chronic HBV infection, and the graph "B" identifies motif rankings of a subject with HCC. In particular, for graph 1702, the rankings of 256 end motifs of plasma DNA molecules (< 600 bp) were plotted against counterparts of long plasma DNA molecules (> 1 kb) for a patient with chronic HBV infection. The pink area 1806A in the graph 1702 identify motifs that were ranked within the top 10 for plasma DNA molecules but ranked 11^{th} or lower for long plasma DNA molecules. Conversely, the yellow area 1808A highlighted motifs that were ranked within the top 10 for long plasma DNA molecules but ranked 11^{th} or lower for short plasma DNA molecules. The motif patterns between short and long DNA molecules were found to be different. For example, the rankings of GCTT, ACTT, and GTTT increased in the long plasma DNA relative to the short plasma DNA, while that of CCAG, CCTG, and CCAA decreased.

For a patient with HCC identified in graph 1704, the relative frequencies reflected in the rankings of 256 end motifs of plasma DNA were different from the relative frequencies of end motifs for patient with chronic HBV infection (graph 1702). In particular, the graphs 1702 and 1704 indicate end-motif frequencies of plasma DNA molecules. Similar to graph 1702, the pink area 1806B in the graph 1704 identify motifs that were ranked within the top 10 for plasma DNA molecules but ranked 11^{th} or lower for long plasma DNA molecules. Conversely, the yellow area 1808B highlighted motifs that were ranked within the top 10 for long plasma DNA molecules but ranked 11^{th} or lower for short plasma DNA molecules. For example, the motif rankings regarding CCAG, CCTG, and CCAA motifs was less obvious in plasma DNA in the patient with HCC were different from those of the patient with HBV infection. Thus, these data suggest that the analysis of end motifs between short and long plasma DNA molecules would be informative for cancer detection. For example, a separation value for CCAG of a biological sample can be compared with a cutoff value to determine a disease classification. However, such analysis involving plasma DNA molecules > 1 kb in size was not able to be obtained in short-read sequencing technologies (e.g. Illumina sequencing platforms), due to the lack of the ability to sequence the long DNA molecules, e.g. > 600 bp.

### E. End-motif frequencies of long DNA molecules

In some embodiments, the end motif analysis corresponds to an analysis of one particular 4-mer end motif. For example, the end motif frequency of CCCA was calculated in short plasma DNA molecules <200 bp, long plasma DNA molecules >600 bp, and long plasma DNA molecules >1 kb.

### 1. Hepatocellular carcinoma

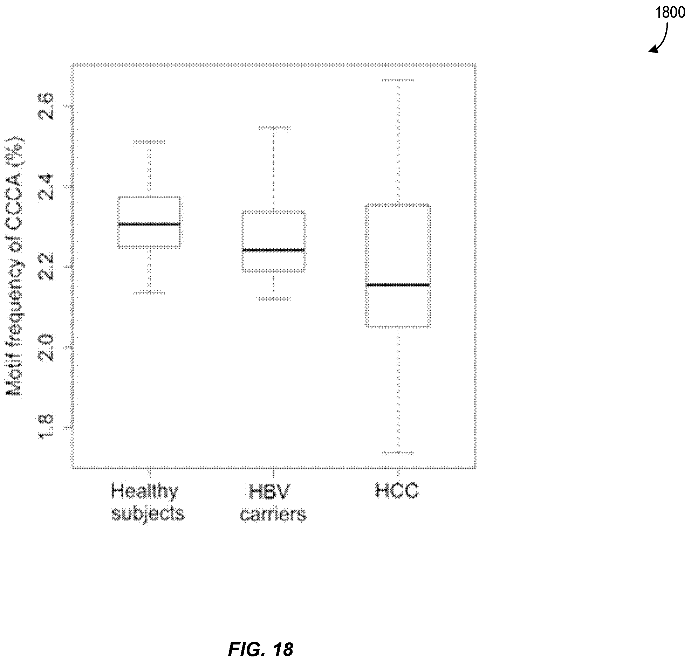

FIG. 18 shows a boxplot 1800 that identifies end-motif frequency of CCCA in plasma DNA molecules <200 bp in HCC and non-HCC subjects. As shown in FIG. 18, the decrease of CCCA end motif in HCC group for short cfDNA molecules was consistent with the previous finding revealed by Illumina platform (Jiang et al. Cancer Discov. 2020; 10:664-673), where a decrease in HCC was observed. The decrease in motif frequency of CCCA for HCC while statistically significant does have some overlap with the other classifications. We explored using long DNA fragments to see if better results could be obtained.

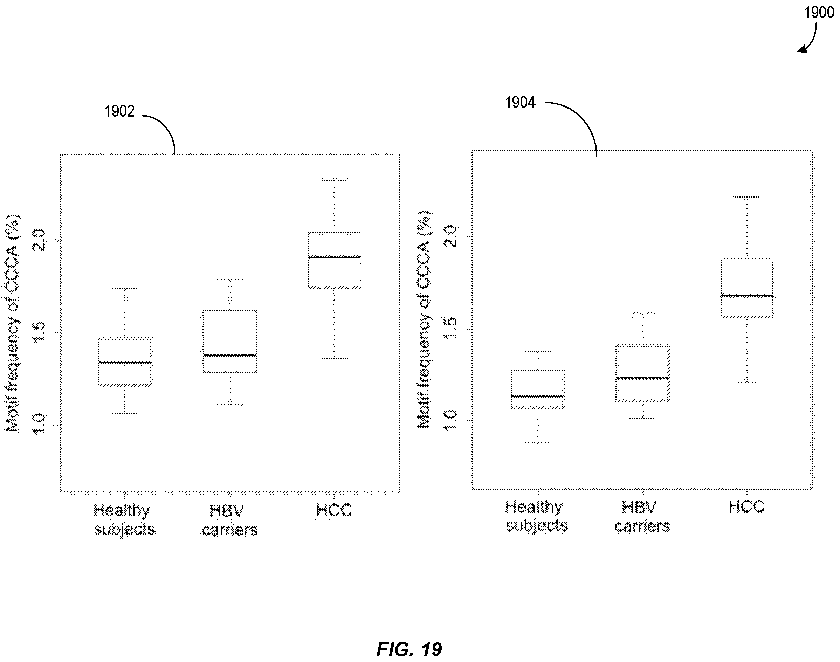

FIG. 19 shows a set of boxplots 1900 that identify motif frequencies of CCCA in plasma DNA molecules. Boxplot 1902 shows CCCA frequencies for plasma DNA molecules longer than 600 bp HCC and non-HCC subjects, and boxplot 1904 shows CCCA frequencies for plasma DNA molecules longer than > 1 kb in HCC and non-HCC subjects. In contrast to FIG. 18, when long DNA molecules were identified and analyzed with the use of SMRT sequencing in our cohort, it was surprisingly found that a higher (not lower) motif frequency of CCCA in long cfDNA molecules was observed in HCC patients compared to non-HCC subjects. Additionally, the separation between HCC and the other classifications was larger for long cfDNA molecules than for short cfDNA molecules.

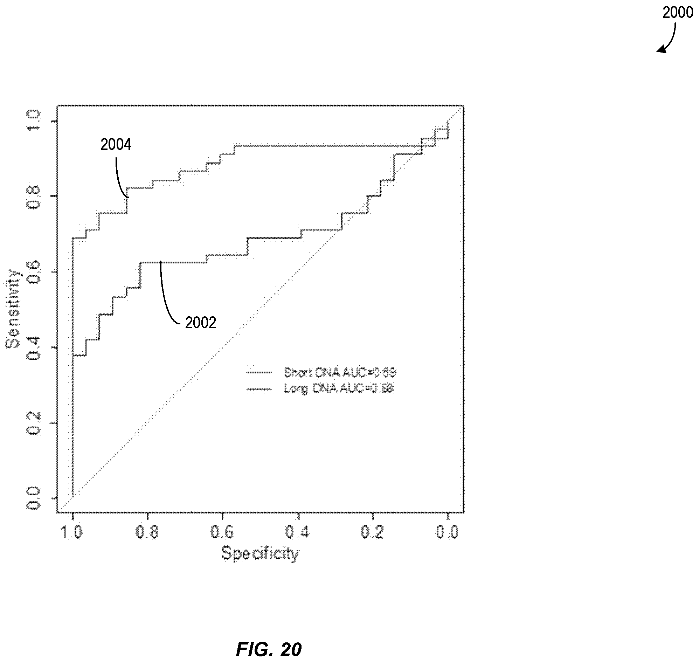

To assess performance of end-motif analysis using long DNA molecules, FIG. 20 shows ROC curve 2000 that identifies performance of motif frequency of CCCA in distinguishing between HCC and non-HCC subjects in short DNA molecules 2002 and long DNA molecules 2004. As shown in FIG. 20, AUC based on end motifs of short cfDNA was 0.69, as opposed to the AUC of long cfDNA 0.88 (P value: 0.0065, Bootstrap test). The use of end motif CCCA deduced from long cfDNA molecules led to an substantially higher power in differentiating HCC patients from non-HCC patients compared with the use of short cfDNA molecules.

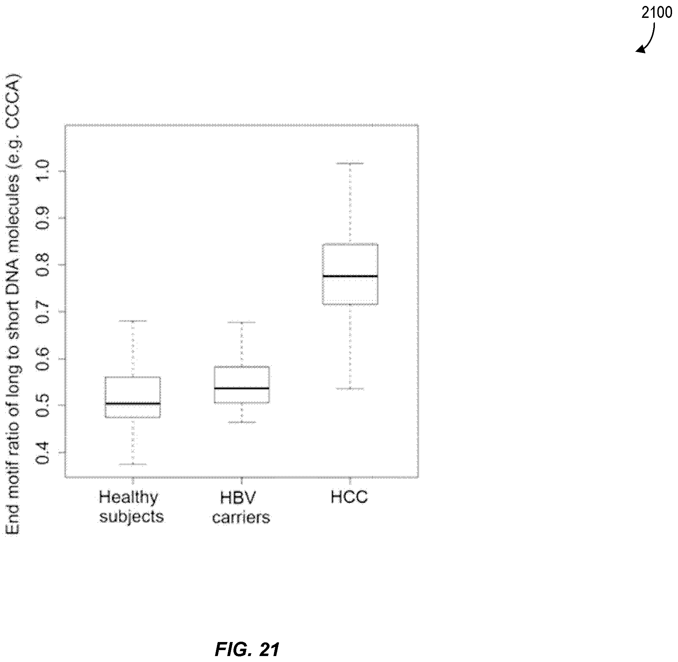

In some embodiments, information from both long and short DNA molecules are integrated into one analytical model for enhancing the power of disease classification. FIG. 21 shows a boxplot 2100 that identifies CCCA ratios in HCC patients, HBV carriers, and healthy subjects. The CCCA ratio was calculated by dividing the CCCA motif frequency of long DNA molecules (>1 kb) by that of short DNA molecules (<200 bp) in HCC patients, HBV carriers and healthy subjects. HCC patients displayed a significantly higher CCCA ratio than non-HCC subjects (P value: 3.919 x 10⁻¹⁰, Mann-Whitney U-test). Compared with either CCCA% of short DNA molecules (FIG. 18) or of long DNA molecules (FIG. 19), the discriminative power between HCC and non-HCC subjects had been greatly enhanced when using the CCCA ratio.

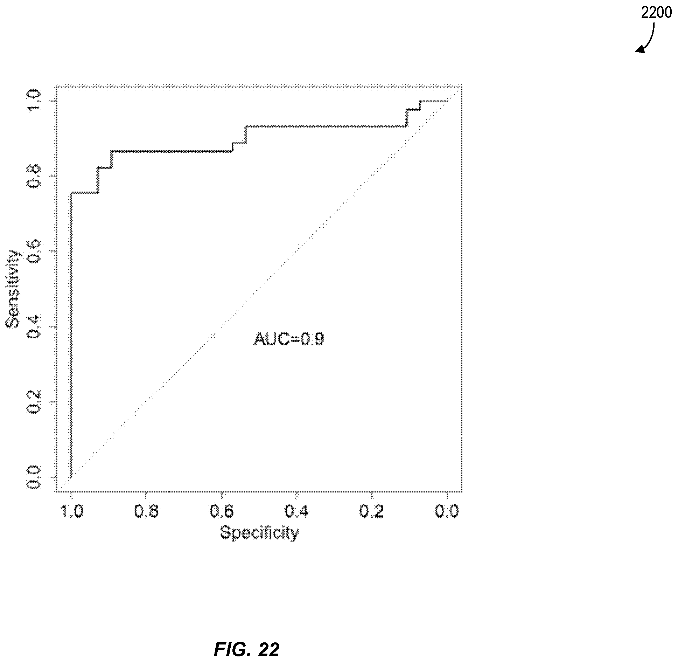

As an example, FIG. 22 shows an ROC curve 2200 that identifies performance of CCCA ratio in distinguishing subjects with and without HCC. FIG. 22 shows an AUC of 0.9 for the long-to-short CCCA ratio analysis. In some embodiments, another end motif ratio (e.g. CCCT, CCCC, CCCG, TTTA) is used for cancer detection or multiple end motif ratios could be used together for cancer detection. These results are surprising, as short-read sequencing technology lacks the capacity of analyzing long DNA molecules (e.g. > 600 bp), such end motif ratio analyses were thought to be impractical in conventional studies.

### 2. Colorectal cancer

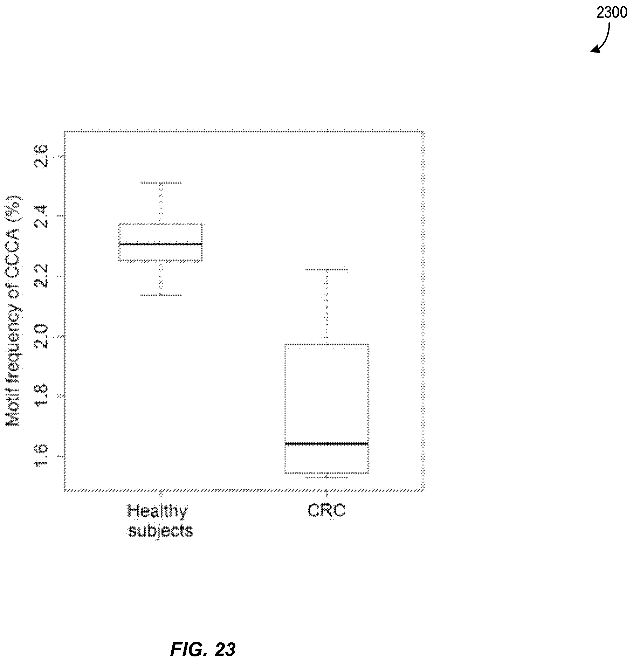

We performed SMRT-sequencing on plasma DNA molecules from 4 colorectal cancer (CRC) patients and 15 healthy subjects. FIG. 23 shows a boxplot 2300 that identifies end-motif frequency of CCCA in plasma DNA molecules <200 bp in CRC patients and healthy subjects. The plasma DNA molecules were sequenced using SMRT-sequencing. FIG. 23 shows that CCCA frequency is significantly decreased in CRC patients when compared to healthy subjects (P value: <0.01, Mann-Whitney U-test).

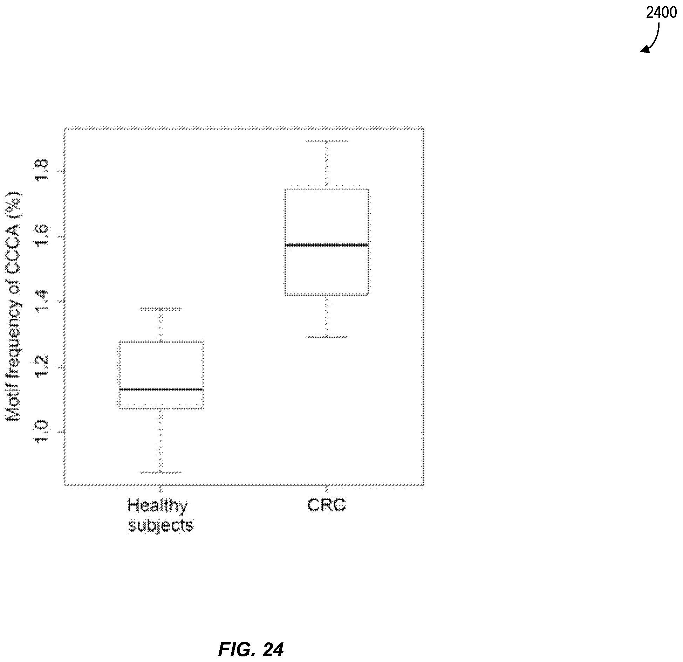

FIG. 24 shows a boxplot 2400 that identifies motif frequencies of CCCA in plasma DNA molecules longer than 1 kb in CRC patients and healthy subjects. The plasma DNA molecules were sequenced using SMRT-sequencing. FIG. 24 shows that CCCA frequency is significantly increased in CRC patients when compared to healthy subjects (P value: 0.01, Mann-Whitney U-test). Such increase in CCCA frequency demonstrates that long cfDNA end motif features can be applied to the detection of multiple cancer types, including but not limited to colorectal cancer and hepatocellular carcinoma presented in this disclosure. This is also surprising as conventional sequencing methods (e.g., Illumina sequencing) cannot identify long DNA molecules (e.g., plasma DNA molecules having sizes greater than 600 bp).

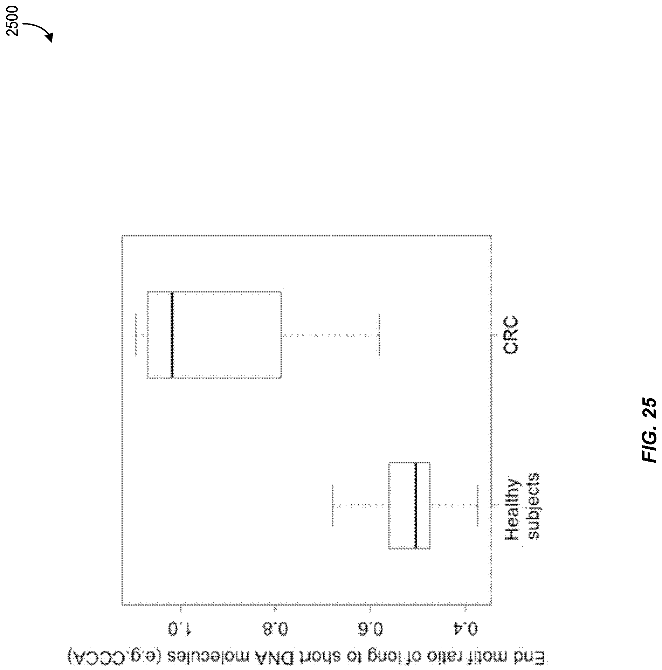

In some embodiments, information from both long and short DNA molecules are integrated into one analytical model for enhancing the power of disease classification in colorectal cancer patients. FIG. 25 shows a boxplot 2500 that identifies CCCA ratios in CRC patients and healthy subjects in SMRT-sequencing. As shown in the boxplot 2500, the CCCA ratio was calculated by dividing the CCCA motif frequency of long DNA molecules (>1 kb) by that of short DNA molecules (<200 bp) in CRC patients and healthy subjects. CRC patients displayed a significantly higher CCCA ratio than healthy subjects (P value: 0.004, Mann-Whitney U-test).

### F. End motif analysis by nanopore sequencing

In some embodiments, nanopore sequencing from Oxford Nanopore Technologies (ONT) is utilized in the end motif analysis of nucleic acids. To demonstrate the effectiveness of nanopore sequencing, plasma DNA molecules from 8 HCC patients and 6 HBV carriers were sequenced by nanopore sequencing.

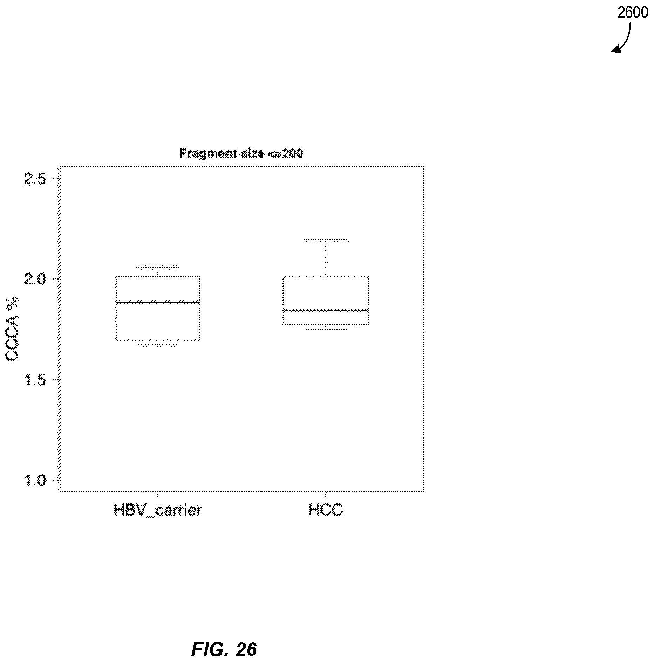

FIG. 26 shows a boxplot 2600 that identifies end-motif frequency of CCCA in plasma DNA molecules <200 bp in HCC patients and HBV carriers. As shown in FIG. 26, the decrease of CCCA end motif in HCC group for short cfDNA molecules was consistent with the previous finding revealed by Illumina platform (Jiang et al. Cancer Discov. 2020; 10:664-673). However, long DNA molecules were barely detectable in Illumina sequencing platform (0% of molecules were >600 bp), thus it was not known as to the utility of long cfDNA molecules in end motif analysis.

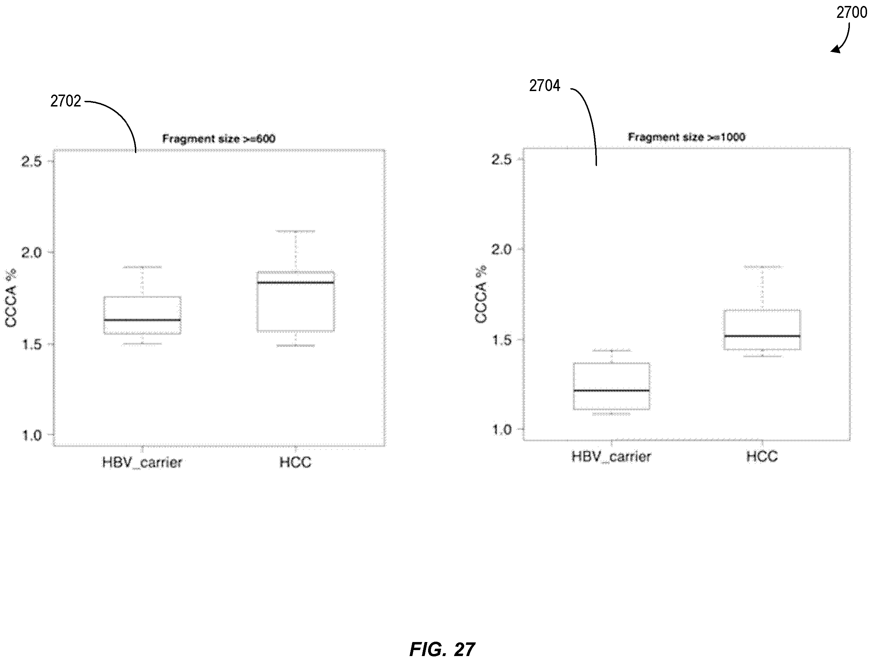

FIG. 27 shows a set of box plots 2700 that identify motif frequencies of CCCA in plasma DNA molecules. As shown in FIG. 27, boxplot 2702 shows CCCA frequencies for plasma DNA molecules longer than 600 bp in HCC and HBV carriers, and boxplot 2704 shows CCCA frequencies for plasma DNA molecules longer than 1 kb in HCC and HBV carriers. In contrast to FIG. 26, when long DNA molecules were identified and analyzed with the use of nanopore sequencing in our cohort, it was found that a higher motif frequency of CCCA in long cfDNA molecules was observed in HCC patients compared to non-HCC subjects, and this is consistent with our data generated from SMRT sequencing platform described in this present disclosure.

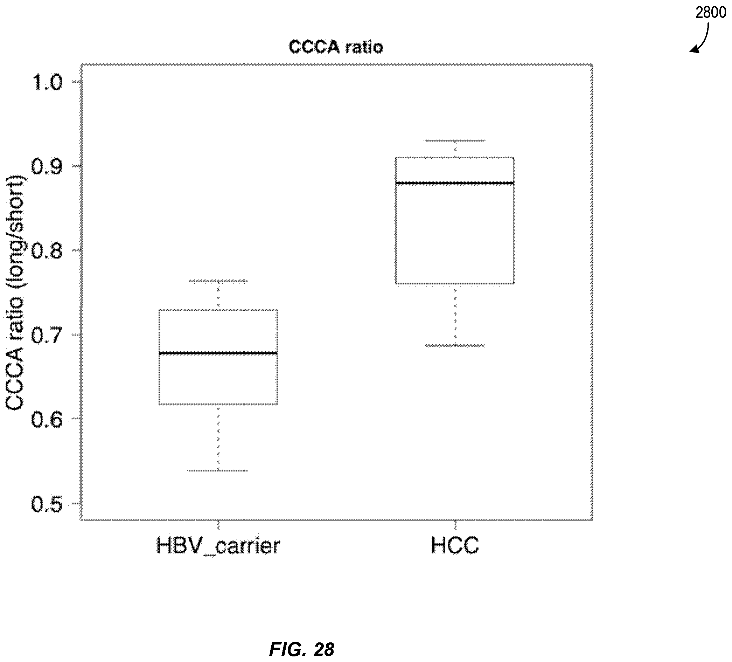

In some embodiments, information from both long and short DNA molecules are integrated into one analytical model for enhancing the power of disease classification in nanopore sequencing. FIG. 28 shows a boxplot 2800 that identifies CCCA ratios in HCC patients and HBV carriers in nanopore sequencing. The CCCA ratio was calculated by dividing the CCCA motif frequency of long DNA molecules (>1 kb) by that of short DNA molecules (<200 bp) in HCC patients and HBV carriers. As shown in FIG. 28, HCC patients displayed a significantly higher CCCA ratio than HBV carriers (P value: 0.013, Mann-Whitney U-test). These findings are consistent with our data generated by SMRT sequencing presented in embodiments of this disclosure, highlighting the diagnostic potential of long cfDNA fragmentomic features across multiple platforms.

### G. Machine-learning techniques for disease classification based on end-motif frequencies of long cell-free DNA molecules

In some embodiments, the end motif analysis is implemented with the use of machine learning models that can extract useful information from end motif signatures for the classification of patients with and without cancers. The machine learning models can include, but not limited to, convolutional neural network (CNN), linear regression, logistic regression, deep recurrent neural network (e.g., fully-connected recurrent neural network (RNN), Gated Recurrent Unit (GRU), long short-term memory, (LSTM)), transformer-based methods (e.g. XLNet, BERT, XLM, RoBERTa), Bayes's classifier, hidden Markov model (HMM), linear discriminant analysis (LDA), k-means clustering, density-based spatial clustering of applications with noise (DBSCAN), random forest algorithm, adaptive boosting (AdaBoost), eXtreme Gradient Boosting (XGBoost), support vector machine (SVM), or a composite model comprising one or more models proposed above.

### 1. Logistic regression

In some embodiments, logistic regression analysis is used to assess the discriminative power for classifying HCC from non-HCC subjects using 4-mer end motifs. A logistic regression (LR) model allows one to establish a relationship between a binary outcome variable. For example, binary outcomes 0 and 1 denote cancer states of non-cancer and cancer, respectively. LR models the logit-transformed probability as a linear relationship with the predictor variables, herein, including end motifs derived from different size ranges of cfDNA molecules. For example, let ***Y*** be the binary outcome variable indicating non-cancer and cancer with {0, 1} and ***p*** be the probability of ***y*** to be 1, ***p** =* ***P*(*Y =*** 1). The higher the ***p*** would indicate the higher likelihood of having a cancer. Let ***x₁**, **x₂**,* ..., ***xₖ*** be a set of predictor variables. In one example, a set of predictor variables could be the frequencies of 256 5' 4-mer end motifs of cfDNA molecules > 1 kb. The logistic regression of ***Y*** on ***x₁*, *x₂,*** ..., ***xₖ*** could allow for deducing parameter values for ***β_{0,} β₁,*** ..., ***βₖ*** via the maximum likelihood method of the following equation: $logit \left(p\right) = log \left(\frac{p}{1 - p}\right) = {β}_{0} + {β}_{1} {x}_{1} + ⋯ + {β}_{k} {x}_{k} ,$ which can be further translated into: $p = \frac{{e}^{{β}_{0} + {β}_{1} {x}_{1} + ⋯ + {β}_{k} {x}_{k}}}{1 + {e}^{{β}_{0} + {β}_{1} {x}_{1} + ⋯ + {β}_{k} {x}_{k}}} .$

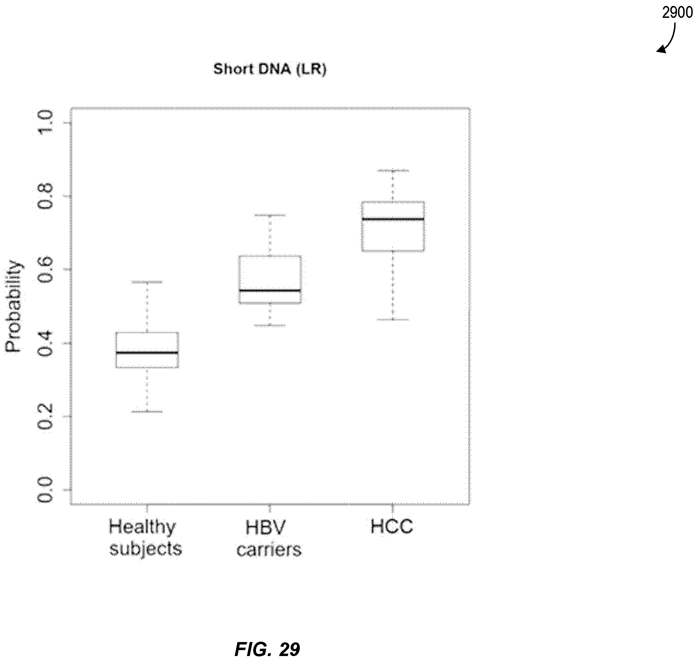

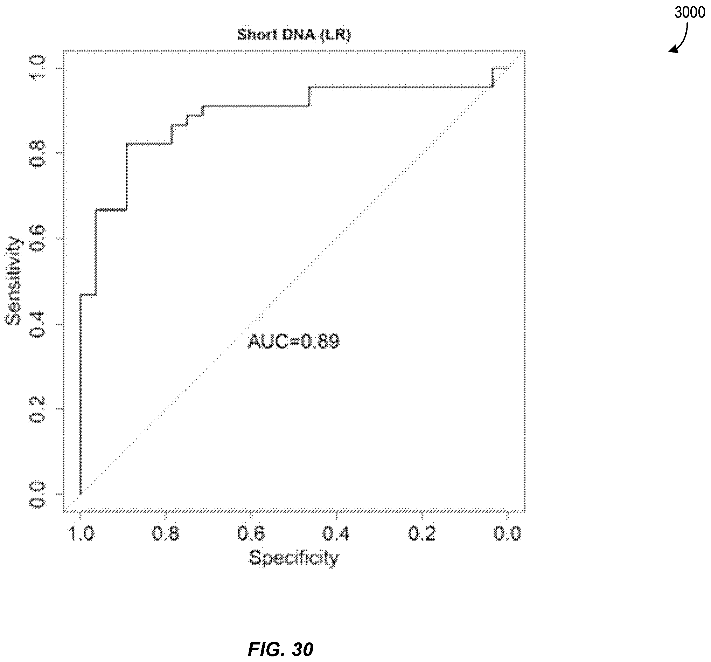

FIG. 29 shows a boxplot 2900 that identifies results generated by logistic regression analysis of end motif features in short DNA molecules having sizes less than 200 bp. FIG. 29 shows the logistic regression analysis with the use of short cfDNA molecules <200 bp. As shown in FIG. 29, HCC patients had a higher probability of being classified as having cancer than control subjects. FIG. 30 shows an ROC curve 3000 that identifies performance of logistic regression with the use of end motif features in short DNA molecules (<200 bp) in distinguishing subjects with and without HCC. FIG. 30 shows an AUC of 0.89 for logistic regression analysis of end-motifs of short DNA molecules.

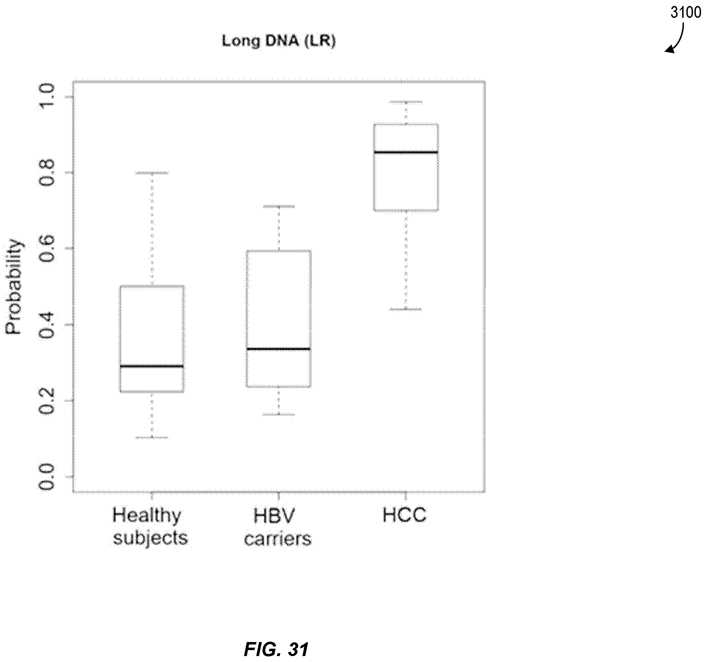

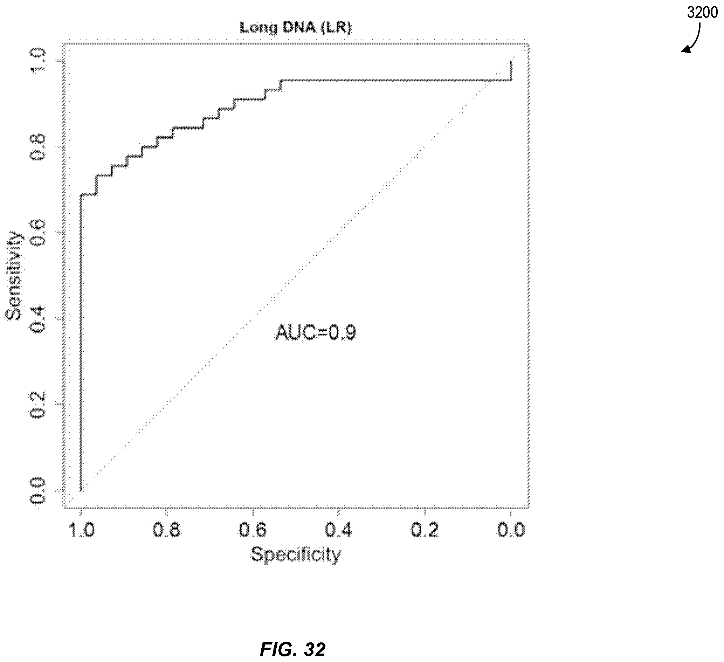

In addition to short DNA molecules, the logistic regression analysis can be extended to long DNA molecules as well. FIG. 31 shows a boxplot 3100 that identifies results generated from logistic regression analysis of end motif features in long DNA molecules with sizes greater than 1 kb. FIG. 32 shows an ROC curve 3200 that identifies performance of logistic regression with the use of end motif features in long DNA molecules (>1 kb) in distinguishing subjects with and without HCC. When long cfDNA molecules >1000 bp were used for the logistic regression analysis, the HCC patients show higher probability than the healthy and HBV-carrier subjects, relative to the results in FIG. 29. Further, the accuracy of HCC classification achieved an AUC of 0.9 as shown in FIG. 32. If one used a cut-off of the probability score to enable the specificity of 99%, the use of short DNA molecules for end motif analysis only gave a sensitivity of 42%, whereas the use of long cfDNA molecules for end motif analysis would improve the sensitivity to 70%.

The above data suggested that the use of long cfDNA molecules implemented in this present disclosure could enhance diagnostic performance. As studies revealed that the tumoral DNA fraction in the plasma of a cancer patient was enriched in short cfDNA molecules using Illumina short-read sequencing technologies (Jiang et al. Proc Natl Acad Sci USA. 2015;112:E1317-25), many studies attempted to focus on the analysis of short cfDNA molecules to improve the performance of cancer detection (Underhill et al. PloS Genet. 2016;12:e1006162; Mouliere et al. Sci Transl Med. 2018;10:eaat4921;Liu et al. Transl Lung Cancer Res. 2021;10:1501-1511). For example, one study tried to remove the long cfDNA molecules using *in vitro* size selection with a bench-top microfluidic device prior to sequencing (Mouliere et al. Sci Transl Med. 2018; 10:eaat4921). Hence, the inclusion of long cfDNA molecules to enhance diagnostic performance has not been explored before, and the improved diagnostic performance using long cfDNA molecules is surprising.

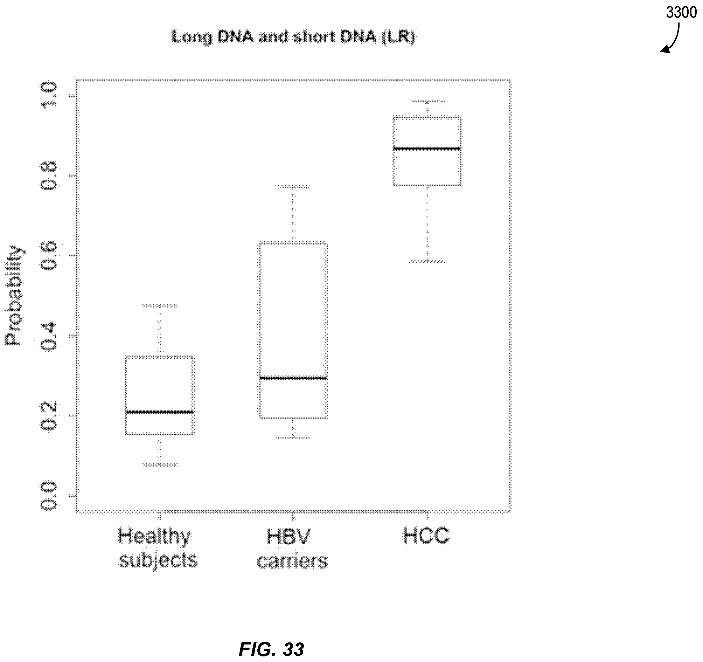

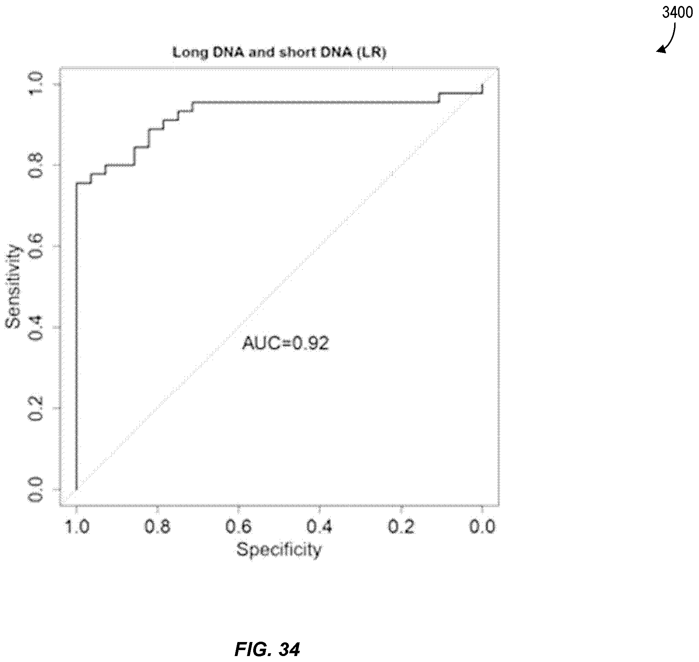

The end-motif features of short and long DNA molecules can be used together to further enhance performance of cancer classification using logistic regression. FIG. 33 shows a boxplot 3300 that identifies logistic regression analysis with the use of end motif features in both long DNA molecules > 1 kb and short DNA molecules <200 bp. As shown in FIG. 33, when the end motif information from both long DNA molecules (>1 kb) and short DNA molecules (<200 bp) were integrated together into the logistic regression analysis, the HCC subjects can be clearly distinguished from healthy subjects and subjects with HBV. FIG. 34 shows an ROC curve 3400 that identifies performance of logistic regression with the combined use of end motif features derived from both long DNA molecules (>1 kb) and short DNA molecules (<200 bp) in distinguishing subjects with and without HCC. As shown in FIG. 34, the diagnostic power between HCC and non-HCC subjects had been further enhanced to an AUC of 0.92. In some embodiments, one could use patterns of end motifs from three or more size ranges. As an example, the frequencies for 256 motifs from molecules with size range < 200 bp, 256 motifs from molecules within size range of from 200 to 600 bp, and 256 motifs from molecules with size range > 600 bp could be integrated together into the logistic regression analysis (no. of 4-mer features: 256 x 3), with an AUC of 0.93 in differentiating patents with HCC from those without cancer.

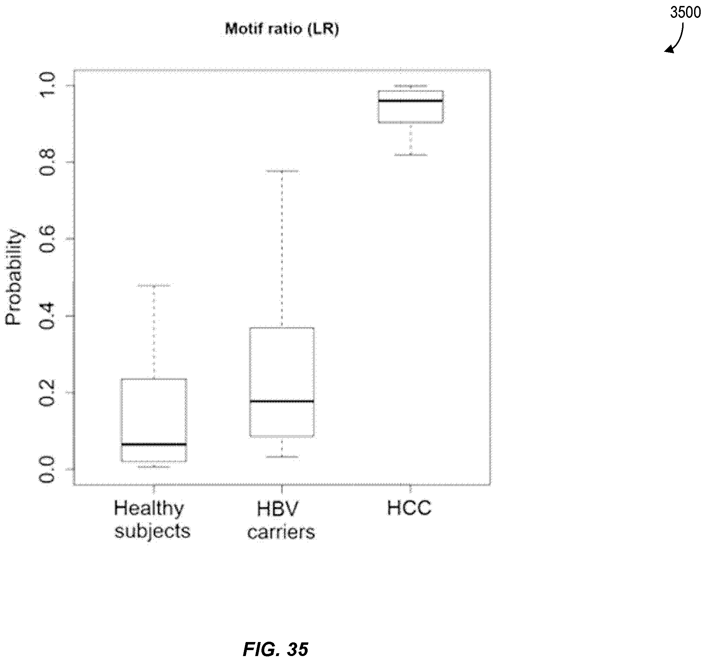

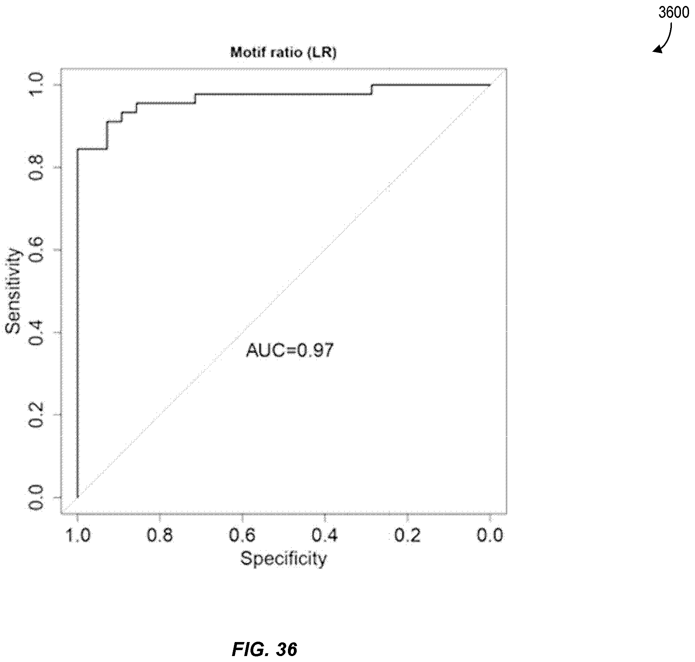

In some instances, a motif ratio calculated by dividing the motif frequency in long DNA molecules (>1 kb) by that in short DNA molecules (<200 bp) is used for logistic regression. FIG. 35 shows a boxplot 3500 that identifies results generated by logistic regression analysis with the use of motif ratio. When such motif ratios were used for the logistic regression analysis, the probabilities generated for the HCC subjects were substantially higher than healthy and HBV-carrier subjects. FIG. 36 shows an ROC curve 3600 that identifies performance of logistic regression with the use of motif ratios in distinguishing subjects with and without HCC. As shown in FIG. 36, the AUC had been further improved to 0.97, reflecting that the enhanced diagnostic potential for cancer could be enabled by synergistically taking advantage of end motif information derived from both short and long cfDNA molecules.

### 2. Support Vector Machine

In some embodiments, a support vector machine (SVM) analysis is used for classifying cancer from non-cancer subjects based on 4-mer end motifs. Given a training dataset for building a SVM classifier comprising n samples: $\left({M}_{1}, {Y}_{1}\right) , … , \left({M}_{n}, {Y}_{n}\right)$ (1) where Yᵢ are either 1 (indicating a cancer subject) or -1 (indicating a non-cancer subject) for a sample *i;* Mᵢ is a *p*-dimensional vector comprising the end motif patterns for a sample *i.* For example, Mᵢ can be a vector containing 256 4-mer end motifs. Alternatively, Mᵢ can be a vector containing values derived from 256 4-mer end motifs, such as ratios between long and short cfDNA molecules. The SVM can be trained using the training dataset to determine a "hyperplane" that separates the non-cancer and cancer groups as accurate as possible. There are various ways to find such a hyperplane. One way is to find a set of coefficients (W with p- dimensional vector) satisfying: $W ⋅ {M}_{i} - b \geq 1 \left(for any subject in cancer group\right)$ (2). and $W ⋅ {M}_{i} - b \leq - 1 \left(for any subject in non-cancer group\right)$ (3). where Wis a *p*-dimensional vector of coefficients determining the hyperplane; *M* is a matrix (*pxn* dimensions) with *p* end motifs and *n* samples; *b* is an intercept.

The formulas (2) and (3) can be rewritten as: ${Y}_{i} \left({W * M}_{i}-b\right) \geq 1$ (4) where Yᵢ is either -1 (non-cancer) or 1 (cancer).

The margin distance (D) between (2) and (3) would be: $\frac{2}{\left‖W\right‖}$
where ∥*W*∥ is computed using the distance from a point to a plane equation.

Thus, we need to maximize *D* by minimizing ∥*W*∥ subject to (4). Based on this principle, the parameters (*W* and *b*) of a classifier can be determined. The cancer risk score for a new sample could be calculated by using the trained parameters (*W* and *b*) in this example.

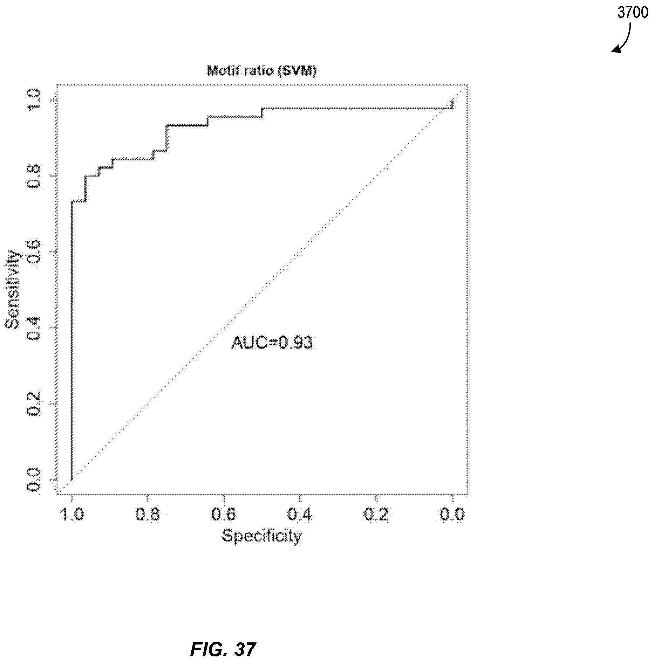

FIG. 37 shows an ROC curve 3700 that identifies performance of SVM with the use of end-motif ratio in distinguishing subjects with and without HCC. The SVM was used to classify a biological sample of a given subject using 256 end-motif ratios, in which each end-motif ratio corresponded to a ratio of frequencies between long and short DNA molecules for a respective end motif (e.g., CCCA). As shown in FIG. 37, when the end motif information from both long DNA molecules (>1 kb) and short DNA molecules (<200 bp) were integrated together into the SVM analysis, the diagnostic power between HCC and non-HCC subjects achieved an AUC of 0.93. In some embodiments, one could use patterns of end motifs from three or more size ranges. As an example, the frequencies for 256 motifs from molecules with size range < 200 bp, 256 motifs from molecules within size range of from 200 to 600 bp, and 256 motifs from molecules with size range > 600 bp could be integrated together into the logistic regression analysis.

### 3. Random forest

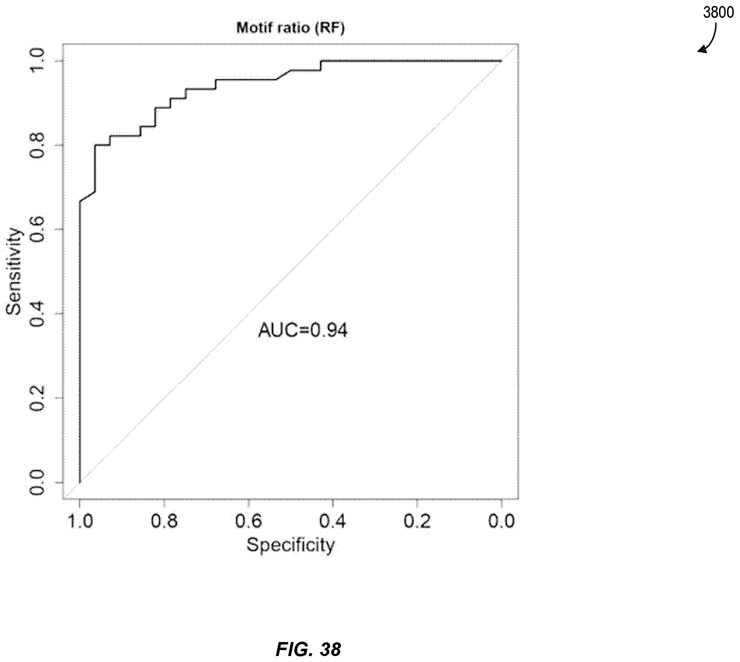

In some embodiments, one can perform a random forest tree analysis for classifying HCC from non-HCC subjects using 4-mer end motifs. FIG. 38 shows an ROC curve 3800 that identifies performance of random forest analysis with the use of motif ratio in distinguishing subjects with and without HCC. The random forest trees were used to classify a biological sample of a given subject using 256 end-motif ratios, in which each end-motif ratio corresponded to a ratio of frequencies between long and short DNA molecules for a respective end motif (e.g., CCCA). As shown in FIG. 38, when the end motif information from both long DNA molecules (>1 kb) and short DNA molecules (<200 bp) were integrated together into the random forest tree analysis, the diagnostic power between HCC and non-HCC subjects achieved an AUC of 0.94.

### 4. Linear discriminant analysis

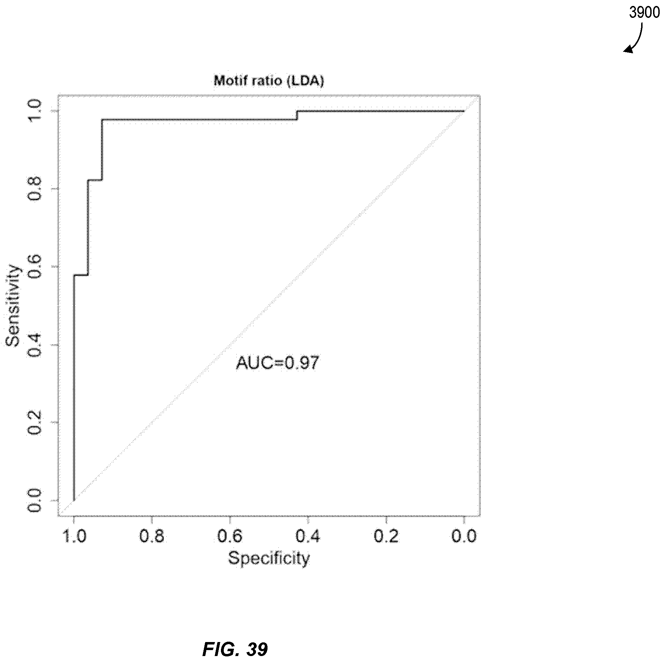

In some embodiments, one can perform a linear discriminant analysis (LDA) for classifying HCC from non-HCC subjects using 4-mer end motifs. FIG. 39 shows an ROC curve 3900 that identifies performance of LDA analysis with the use of motif ratio in distinguishing subjects with and without HCC. The linear discriminant analysis was used to classify a biological sample of a given subject using 256 end-motif ratios, in which each end-motif ratio corresponded to a ratio of frequencies between long and short DNA molecules for a respective end motif (e.g., CCCA). As shown in FIG. 39, when the end motif information from both long DNA molecules (>1 kb) and short DNA molecules (<200 bp) were integrated together into the LDA analysis, the diagnostic power between HCC and non-HCC subjects achieved an AUC of 0.97.

### H. Methods for analysis of short and long DNA molecules

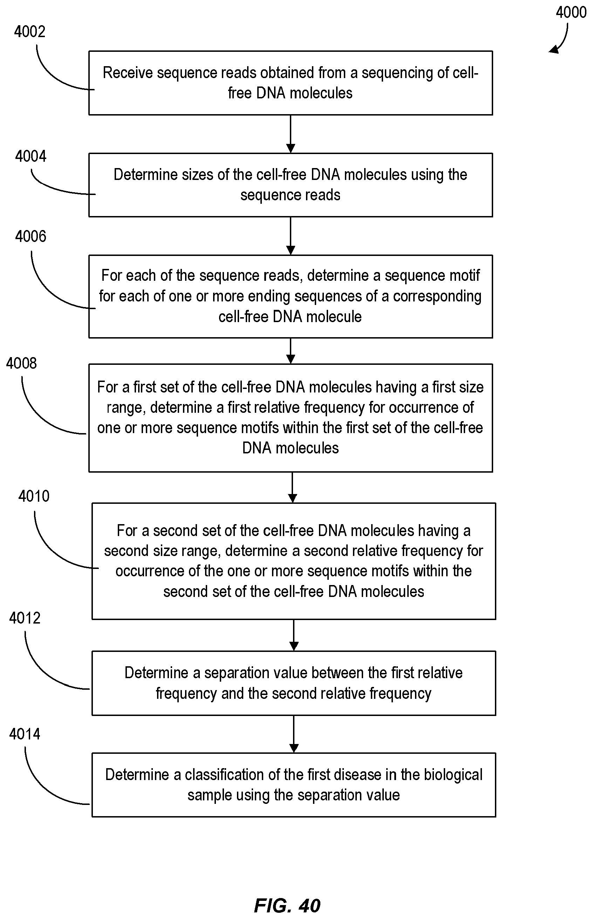

FIG. 40 shows a flowchart 4000 illustrating an example process for analyzing a biological sample of a subject based on relative frequencies of sequences having one or more end motifs, according to some embodiments. The biological sample can include DNA originating from normal cells and potentially from cells associated with a disease (e.g., a cancer). In addition, at least some of the DNA is cell-free in the biological sample.

At step 4002, sequence reads obtained from a sequencing of cell-free DNA molecules can be received. For example, single molecule real-time sequencing (i.e. SMRT-seq) (e.g. from Pacific Biosciences, PacBio SMRT-seq) and nanopore sequencing (e.g. from Oxford Nanopore Technologies) can be used to obtain the sequence reads from the biological sample. Other sequence techniques can be used, e.g., as described herein.

At step 4004, sizes of the cell-free DNA molecules using the sequence reads can be determined. For example, a number of nucleotides can be counted to determine the size of a cell-free DNA molecule. Other techniques can also be used, e.g., using paired-end sequencing and aligning a pair of sequence reads to a reference genome.

At step 4006, for each of the sequence reads, a sequence motif for each of one or more ending sequences of a corresponding cell-free DNA molecule can be determined. For example, a 4-mer end motif of the sequence read could be determined by analyzing the 4 nucleotides at its ends. Continuing with the example, a first sequence read may include CCCA as the sequence motif, and a second sequence read may include CCAG as the sequence motif. Similarly, in some embodiments, other types of end motifs could be used, including but not limited to, 1-mer, 2-mer, 3-mer, 5-mer, 6-mer, 7-mer, 8-mer, 9-mer, 10-mer, 15-mer, 20-mer, or other combinations.

At step 4008, for a first set of the cell-free DNA molecules having a first size range, a first relative frequency for occurrence of one or more sequence motifs within the first set of the cell-free DNA molecules can be determined. The relative frequency can be a ranking of the sequence motif. As another example, the relative frequency can be a percentage of the DNA molecules that have a particular sequence motif.

In some instances, the first relative frequency is a proportion of first set of the cell-free DNA molecules relative to the cell-free DNA molecules from the biological sample. Additionally or alternatively, the first relative frequency is a proportion of first set of the cell-free DNA molecules relative to a number of cell-free DNA molecules having other sequence motifs. In some instances, the first size range includes an upper bound selected from one of at least 80 bases, at least 100 bases, at least 150 bases, at least 200 bases, or at least 300 bases. For example, the first size range can be 1-200 bp.

At step 4010, for a second set of the cell-free DNA molecules having a second size range, a second relative frequency for occurrence of the one or more sequence motifs within the second set of the cell-free DNA molecules can be determined. In some instances, the second relative frequency can be a proportion of second set of the cell-free DNA molecules relative to the cell-free DNA molecules from the biological sample. Additionally or alternatively, the second relative frequency is a proportion of second set of the cell-free DNA molecules relative to a number of cell-free DNA molecules having other sequence motifs.

In some instances, the second size range has an upper bound that is larger than the upper bound for the first size range. For example, the first size range can be less than 600 bp, and the second size range can be greater than 1000 bases. In some examples, the two size ranges can overlap, e.g., the first size range can be less than 800 bp and the second size range can be between 700 bp and 2000 bp. Additionally or alternative, the second size range includes a lower bound selected from one of at least 300 bases, at least 400 bases, at least 500 bases, at least 600 bases, or at least 800 bases. In some instances, the lower bound of the second size range is greater than the upper bound of the first size range.

At step 4012, a separation value between the first relative frequency and the second relative frequency can be determined. In some instances, the separation value is a ratio between the first relative frequency and the second relative frequency or a ratio of respective functions of the frequencies. For example, the separation value can be a ratio of the second set of cell-free DNA molecules (e.g., long DNA molecules) relative to the first set of cell-free DNA molecules (e.g., short DNA molecules), in which the first and second sets have ending sequences corresponding to CCCA. In other instances, the separation value can include subtraction of the two frequencies, as well as combinations of functions providing a measure of separation between the frequencies. Determining the separation values is additionally described in Sections III.D and III.E of the present disclosure.

At step 4014, a classification of the disease in the biological sample can be determined using the separation value. In some instances, the classification is determined by comparing the separation value to one or more cutoff values. The disease can be cancer (e.g., HCC, CRC), and the classification can include a plurality of stages of cancer. As examples, the cancer can be hepatocellular carcinoma, lung cancer, breast cancer, gastric cancer, glioblastoma multiforme, pancreatic cancer, colorectal cancer, nasopharyngeal carcinoma, and head and neck squamous cell carcinoma. In some instances, the classification of the disease identifies a classification of a severity of the disease. Determining the disease classification can include a histological status of the cancer, e.g., whether vascular invasion exists. The one or more cutoff values can be determined from reference samples with know classifications of the disease (e.g., a healthy sample, a sample obtained from a subject classified as having the disease). In some instances, a cutoff value of the one or more cutoff values can be selected from one of 0.6, 0.65, 0.7, or 0.75.

As described above, the one or more cutoff values can be determined using machine learning with training samples with know classifications of the disease (e.g., those shown in FIG. 17). In another example, the comparison to the one or more cutoff values can be performed using a machine learning model. The machine-learning model can be applied to the separation value to generate the classification of the disease. The machine learning models can include, but not limited to, convolutional neural network (CNN), linear regression, logistic regression, deep recurrent neural network (e.g., fully-connected recurrent neural network (RNN), Gated Recurrent Unit (GRU), long short-term memory, (LSTM)), transformer-based methods (e.g. XLNet, BERT, XLM, RoBERTa), Bayes's classifier, hidden Markov model (HMM), linear discriminant analysis (LDA), k-means clustering, density-based spatial clustering of applications with noise (DBSCAN), random forest algorithm, adaptive boosting (AdaBoost), eXtreme Gradient Boosting (XGBoost), support vector machine (SVM), or a composite model comprising one or more models proposed above.

### IV. METHYLATION-PATTERN ANALYSIS OF LONG CELL-FREE DNA MOLECULES FOR DISEASE CLASSIFICATION

As any tissue in a human body could suffer from tumorigenesis, the determination of tissue of origin for a single molecule would be useful for cancer tests and guiding cancer treatments. One approach could be based on the hypothesis that targeted tissue of origin for a plasma DNA corresponded to the least mismatches in terms of methylation status (i.e. methylation mismatches) across CpG sites between plasma DNA methylation haplotype and the methylation haplotype of that tissue, herein named the least methylation mismatches approach. The number of methylation mismatches could be determined by pair-wise comparison of methylation status across CpG sites between two methylation haplotypes originating from the same genomic positions. If two methylation status between two methylation haplotypes at the same CpG position are different, it will be counted as one mismatch.

In some embodiments, the methylation haplotypes of long DNA molecules obtained from tissues and plasma DNA molecules are used for enhancing the accuracy, as the long methylation haplotype would have a higher chance of containing the informative methylation patterns unique to a particular tissue, in comparison to the short methylation haplotype.

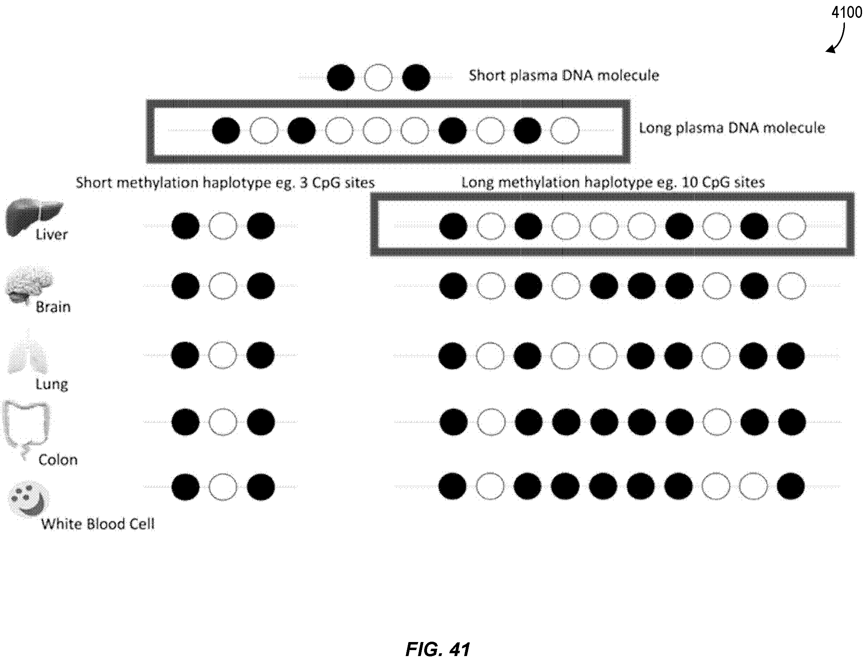

FIG. 41 shows an example illustration 4100 of comparing methylation pattern of a long cell-free DNA molecule with methylation patterns of reference tissues, according to some embodiments. In particular, FIG. 41 shows that the short methylation haplotype of plasma DNA with 3 CpG sites could not allow for determining which tissue contributed such a plasma DNA molecule (e.g., liver, brain and lung tissues shared the same short methylation haplotype). In contrast, the long methylation haplotype of plasma DNA with 10 CpG sites could allow for the unambiguous determination of the liver as the tissue of origin of such a plasma DNA molecule, as the methylation haplotype from the liver exhibited the least methylation mismatches of 0 relative to that from the plasma DNA while the methylation haplotypes from the brain, lung, colon and white blood cells exhibited 2, 3, 4 and 5 methylation mismatches, respectively.

In some embodiments, the pattern recognition analysis for methylation haplotypes would improve the performance of determining the tissue of origin for each long plasma DNA molecule. The determination of the tissue of origin can then be used to determine a disease classification.

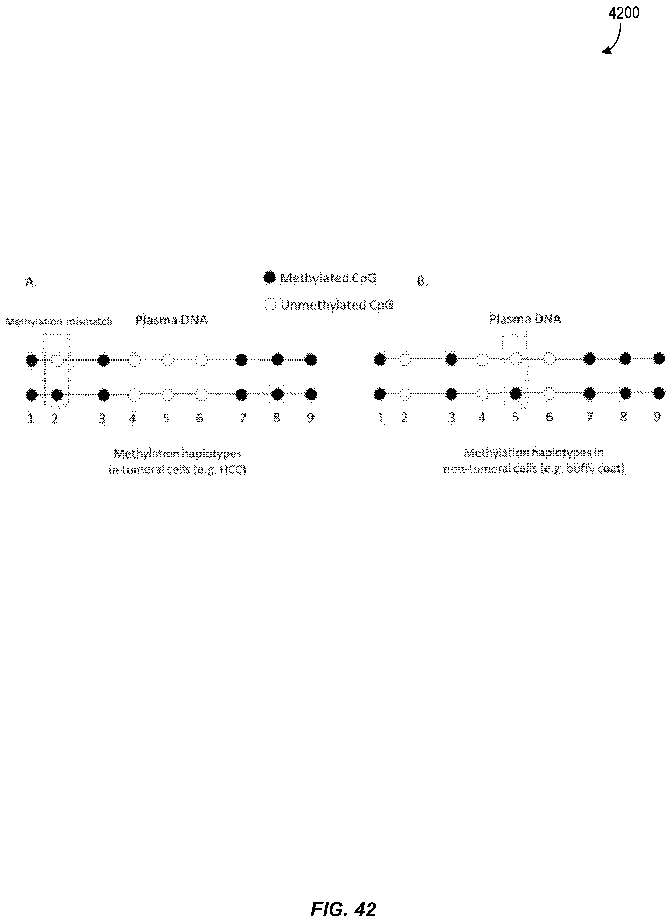

FIG. 42 illustrates a technique 4200 for analyzing methylation patterns in long cell-free DNA molecules that include at least one methylation mismatch, according to some embodiments. As shown in FIG. 42, determining the tissue of origin for a plasma DNA molecule can be challenging for the least methylation mismatches approach. In particular, a given plasma DNA molecules has a methylation mismatch at site "2" when compared against methylation haplotype in tumor cells, but also has a methylation mismatch at site "5" when compared against the methylation haplotype in non-tumoral cells (e.g., buffy coat). The pattern recognition analysis can address this challenge. For example, it can be determined that presence of the three consecutive unmethylated CpG sites at positions 4, 5, and 6 would indicate a higher likelihood of HCC. Based on this information, CpG sites at positions 4, 5, and 6 would be given higher weights indicating tumoral patterns relative to CpG sites at other positions. Based on the weights, the given plasma DNA molecule can be predicted as being associated with tumoral cells based on its unmethylated CpG sites at positions 4, 5, and 6. These types of pattern analyses can be more effective when the given plasma DNA molecule is greater than a certain length.

In some embodiments, the lengths of plasma DNA include, but are not limited to, ≥ 500 bp, ≥ 600 bp, ≥ 1 kb, ≥ 2 kb, ≥ 3 kb, ≥ 4 kb, ≥ 5 kb, ≥ 10 kb or other combinations. The number of CpG sites can include, but are not limited to, ≥ 3, ≥ 4, ≥ 5, ≥ 6, ≥ 7, ≥ 8, ≥ 9, ≥ 10, ≥ 15, ≥ 20, ≥ 25, ≥ 30, ≥ 35, ≥ 40, ≥ 45, ≥ 50, ≥ 60, ≥ 70, ≥ 80, ≥ 90, ≥ 100, ≥ 200, ≥ 300, ≥ 400, ≥ 500, ≥ 1000, or other combinations. In some embodiments, the methylation haplotypes of long DNA molecules from various tissues and tumor tissues are determined by methylation-aware enzymatic conversion. One example of such conversion method is methyl-seq (EM-seq) which involved non-destructive enzymatic reactions, utilizing TET2 and APOBEC3A to convert unmethylated (but not methylated) cytosines to uracils (e.g. NEBNext^{®} Enzymatic Methyl-seq Kit), which was sequenced as thymines. Conventional bisulfite sequencing would have a disadvantage for obtaining long DNA molecules, as it would degrade long DNA molecules, thus shortening the methylation haplotype information and adversely affecting the accuracy in determining the tissue of origin of plasma DNA.

### A. CpG sites in long cell-free DNA molecules

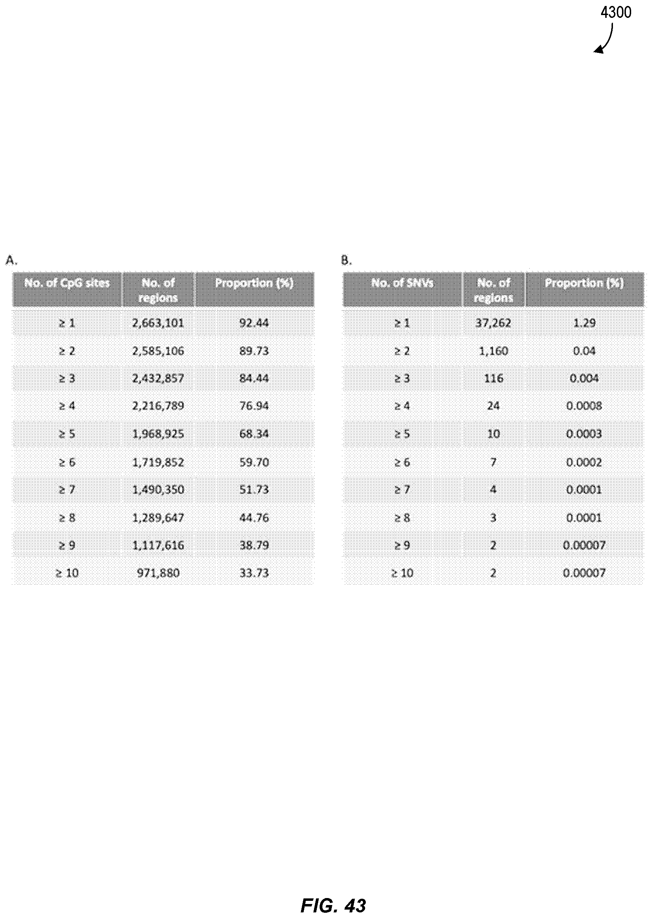

FIG. 43 shows a comparison 4300 of the pervasiveness of CpG sites and cancer-derived single nucleotide variants (SNVs) across the genome at 1-kb resolution. In particular, table A shows a number of 1-kb genomic regions of a given genome (e.g., a reference genome) having at least a corresponding number of CpG sites (e.g., > 1). Table B shows a number of 1-kb genomic regions of the genome having at least a corresponding number of SNVs (e.g., > 2). As shown in FIG. 43, there were 971,880 1-kb regions containing at least 10 CpG sites, accounting for 33.7% of a human genome, whereas there are only 2 1-kb regions containing at least 10 SNVs when analyzing 38,465 somatic mutations from a tumor tissue. Thus, plasma DNA molecules having a large amount of CpG sites can be sufficiently obtained, such that their methylation patterns can be used to predict a disease.

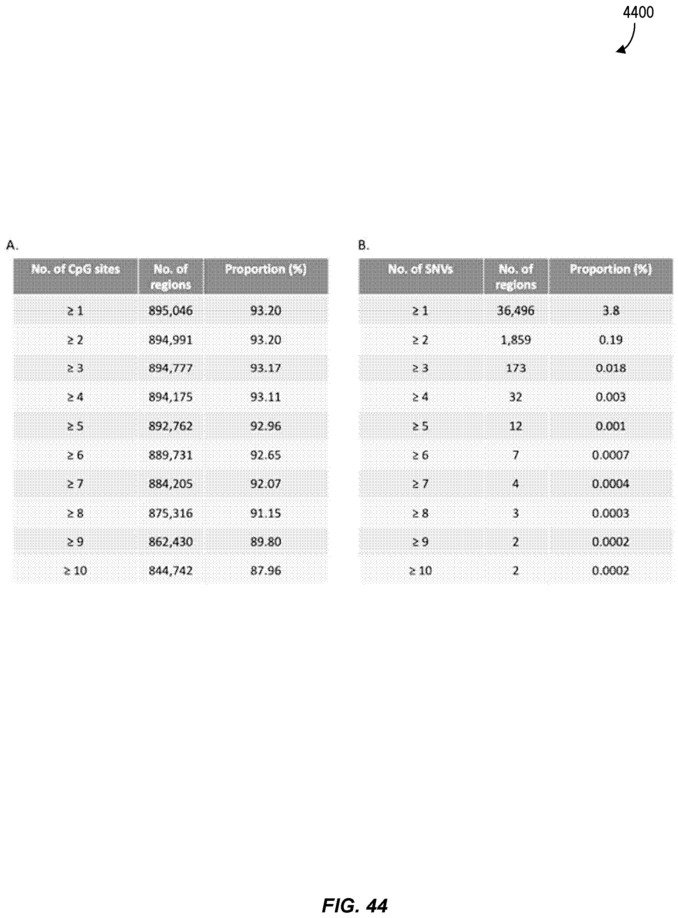

FIG. 44 shows a comparison 4400 of the pervasiveness of CpG sites and cancer-derived SNVs across the genome at 3-kb resolution. Table A shows a number of 3-kb genomic regions of a given genome (e.g., a reference genome) having at least a corresponding number of CpG sites (e.g., > 1). Table B shows a number of 3-kb genomic regions of the genome having at least a corresponding number of SNVs (e.g., > 2). As shown in FIG. 44, there were 844,742 3-kb regions containing at least 10 CpG sites, accounting for 88.0% of a human genome, whereas there were only 2 3-kb regions containing at least 10 SNVs. These results suggested that the analysis based on methylation haplotypes of long plasma DNA molecules can provide a significant improvement in the information of plasma DNA molecules that could be used for informing the presence of cancer.

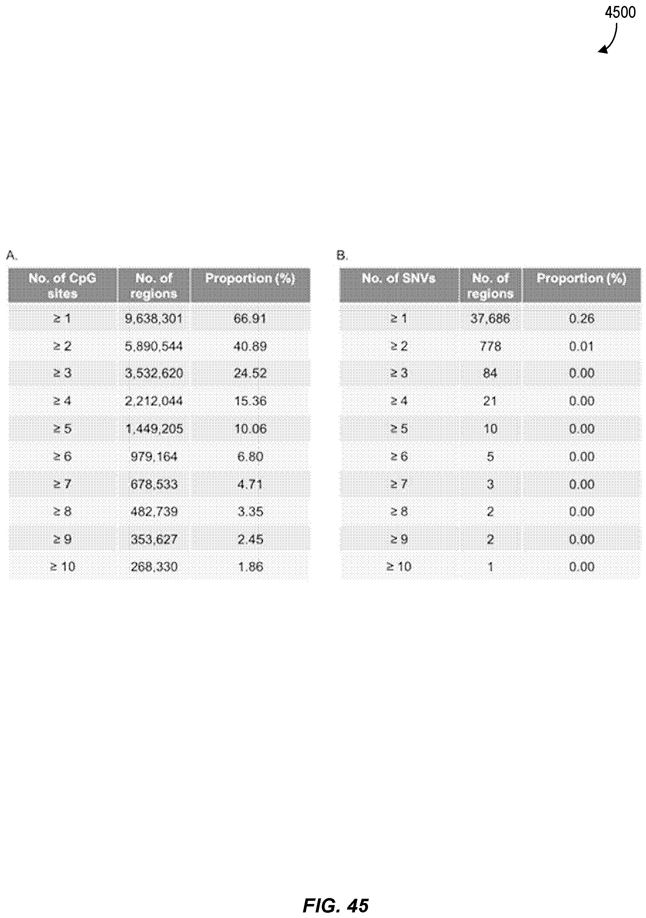

In contrast, the quantity of CpG sites of short cell-free DNA molecules may not be sufficient enough for disease classification. FIG. 45 shows a comparison 4500 of the pervasiveness of CpG sites and cancer-derived SNVs across the genome at 200 bp resolution. Table A shows a number of 200 bp genomic regions a given genome (e.g., a reference genome) having at least a corresponding number of CpG sites (e.g., > 1). Table B shows a number of 200 bp genomic regions of the genome having at least a corresponding number of SNVs (e.g., > 2). As shown in FIG. 45, the percentage of 200-bp regions containing at 10 CpG sites rapidly decreased to as low as 1.9%. This result suggests that the number of CpG sites present on short cell-free DNA molecules (e.g. < 200 bp) would be limited, thereby adversely affecting the accuracy of tissue of origin analysis or disease classification based on plasma DNA.

Thus, methylation patterns of several CpG sites in long cell-free DNA molecules can be used to identify one or more biomarkers that can be predictive of a presence of disease (e.g., a cancer). For example, sequence reads corresponding to long cell-free DNA molecules of a plasma sample can be obtained using methylation-aware sequencing (e.g., Enzymatic Methyl-seq). Each sequence read can include a methylation pattern that identifies methylation status at a set of CpG sites on the sequence read. The methylation pattern of each sequence read can be compared with a reference methylation pattern of a tissue type, so as to determine a tissue classification for the sequence read. The tissue classifications of the sequence reads can then be used to determine a disease classification.

The per-molecule methylation analysis can be performed even if there is low resolution in the reference tissue methylome, e.g., because the pattern specificity of long cell-free DNA molecules is much higher. For instance, even if the reference tissue methylome does not cover every base of the genome or includes fragmented sections of information, there are likely to be fewer matches for a long cell-free DNA molecule in the whole genome. In effect, the long cell-free DNA molecule can still be aligned to its true match despite any fuzziness of the reference. As long as a reference methylation pattern exists, the comparison of methylation patterns can be used to predict whether a proportion of long cell-free DNA molecules correspond to a particular tissue type. A high proportion of long cell-free DNA molecules associated with the particular tissue type in the plasma sample can be predictive of cancer

### B. Tissue of origin

The presence of long plasma DNA molecules in patients with HCC, accompanied by a series of CpG sites carrying distinct methylation patterns (i.e. methylation haplotype information), would facilitate the trace of their tissues/tumors of origin at the single molecule level.

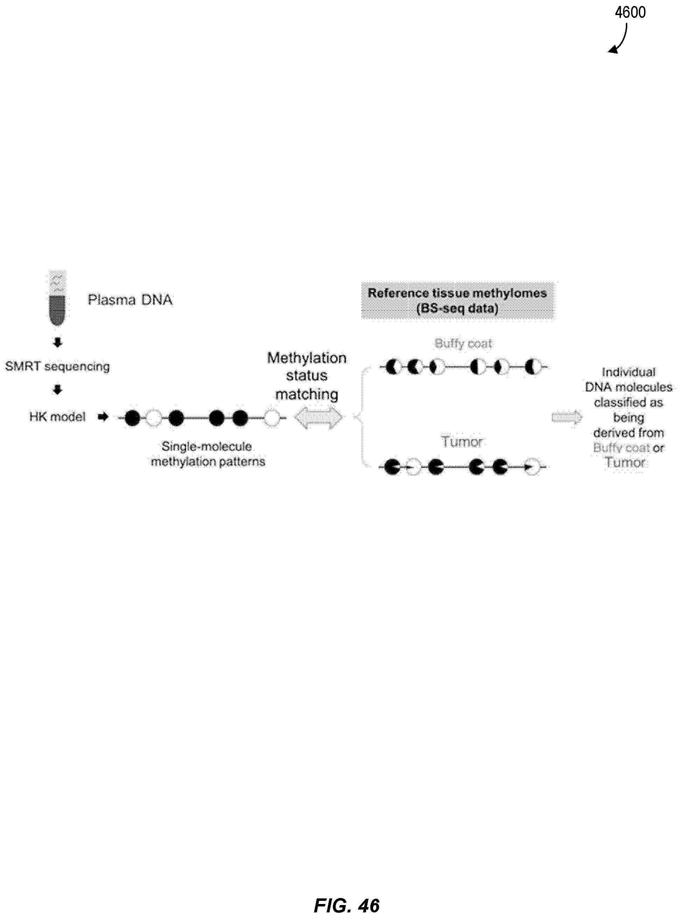

FIG. 46 shows a schematic diagram 4600 that illustrates an example process for predicting whether a cell-free DNA molecule corresponds to tumor DNA, according to its methylation haplotype information. In FIG. 46, plasma DNA molecules were subjected to SMRT-sequencing. The methylation status across CpG sites of each sequenced read was deduced using HK model (Tse et al. Proc Natl Acad Sci USA. 2021;118: e2019768118). To demonstrate the feasibility of tracing the tissue of origin of plasma DNA by its methylation haplotype, one could calculate the distance in terms of methylation status between a plasma DNA molecule (methylation status (0/1) across CpG sites) and the aggregate methylation indices corresponding to CpG sites (continuous values each ranging from 0 to 1) in each reference tissue, for example, buffy coat and HCC tumor. In some instances, a dark color indicates methylation of a corresponding CpG site ("1"), whereas the white color indicates unmethylation of the CpG site ("0"). Each pie chart in a reference tumor tissue can represent a proportion (percentage) of reference DNA molecules that were methylated in a corresponding CpG site. Thus, a predominantly dark color in a pie chart would mean that a high proportion of reference DNA molecules had methylation at the corresponding CpG site.

Methylation status of each CpG site of a given long cell-free DNA molecule can thus be compared with corresponding pie charts of each of the reference tissues, and the tissue matching can be determined based on a reference tissue pattern that deviates the least from the methylation status of all the CpG sites present on the long cell-free DNA molecule considered collectively. For example, for a CpG site, a first distance between the methylation status of a long cell-free DNA molecule and the proportion of the reference DNA molecules in the reference tumor tissue can be calculated. As an example, the distance between a methylated site (1) on a DNA molecule and a reference having a 60% methylation index (density) can be 0.4. If the DNA molecule were unmethylated, the distance could be 0.6. For the same CpG site, a second distance between the methylation status of the long cell-free DNA molecule and the proportion of the reference DNA molecules in the reference buffy coat can be calculated. The first and second distances can be compared. In this example, the first distance is less than the second distance, which can indicate that the CpG site has similar methylation status as the reference tumor tissue.

In some embodiments, the methylation index for each of the CpG sites in a reference tissue is obtained from bisulfite sequencing (BS-seq) data, which was defined as the percentage or proportion of sequenced CpGs identified to be methylated. Additionally or alternatively, the aggregate methylation index in a reference tissue could be obtained from Enzymatic Methyl-seq data (i.e. EM-seq).

The distance between the methylation haplotype of a plasma DNA and a reference tissue methylome could include, but not limited to, Euclidean distance, cosine similarity, Hamming distance, edit distance, etc. In some embodiments, the distance calculation could be adjusted by a weighting vector depending on different genomic positions. For example, a higher weight would be assigned to a position showing a high degree of differential methylation between a tumor and non-tumoral tissue. In contrast, a lower weight would be assigned to a position showing a low degree of differential methylation between a tumor and non-tumoral tissue.

### C. Tissue-of-origin analysis using methylation scores

### 1. Hepatocellular carcinoma (HCC)

In some embodiments, methylation-pattern analysis described herein can include using additional calculations to further exemplify the analysis for the tissue-of-origin of plasma DNA molecules. As an illustrative example, the methylation pattern of each plasma DNA molecule was determined according to the polymerase kinetic signals surrounding CpG sites using the holistic kinetic (HK) model (Tse et al. Proc Natl Acad Sci USA. 2021;118: e2019768118). Such methylation pattern of each plasma DNA molecule was compared with the reference methylation profiles such as but not limited to liver tissues, buffy coat, colon tissues, lung tissues etc. In some embodiments, the reference methylation profiles is obtained based on high-depth bisulfite sequencing results. For each CpG site in the genome of each reference tissue, a methylation index (***MI***) was calculated by the following formula: $MI = \frac{C}{C + T} \times 100 % ,$ where "C" represents the number of sequenced cytosines (i.e. methylated CpGs) and "T" represents the number of sequenced thymines (i.e. unmethylated CpGs).

The CpG sites with *MI* difference between the liver tissue and buffy coat greater than 30% were considered informative for downstream analysis. In some embodiments, the *MI* difference includes, but are not limited to, 5%, 10%, 15%, 20%, 25%, 40%, 45%, 50%, 55%, 60%, 65%, 70%, 75%, 80%, 85% 90%, etc. In some embodiments, a scoring system is used to determine the likelihood of a DNA molecule originating from a particular tissue based on the comparison between observed methylation pattern in that molecule and reference methylation profiles. For each DNA molecule carrying n informative CpG sites, a methylation score, *S(liver),* was calculated by the formula as follows: $S \left(liver\right) = {\sum }_{i = 1}^{i = n} \left[1-\left({P}_{i}-M{I}_{i , liver}\right)\right] ,$ where ***Pᵢ*** denotes the methylation status for a CpG site ***i***; ***Pᵢ*** of 0 and 1 represent unmethylated and methylated CpG site, respectively; ***MI_{i,liver}*** denotes the methylation index for a CpG site ***i*** in the liver. A higher ***S(liver)*** indicates a higher likelihood that the DNA molecule would have originated from the liver tissue.

Similarly, another methylation score, ***S(buffy coat),*** was calculated to determine the similarity of methylation pattern between a plasma DNA molecule and the buffy coat as follows: $S \left(buffy coat\right) = {\sum }_{i = 1}^{i = n} \left[1-\left({P}_{i}-M{I}_{i , buffy coat}\right)\right] .$

Similarly, another methylation score, *S(colon),* was calculated to determine the similarity of methylation pattern between a plasma DNA molecule and the colon as follows: $S \left(colon\right) = {\sum }_{i = 1}^{i = n} \left[1-\left({P}_{i}-M{I}_{i , colon}\right)\right] .$

Similarly, another methylation score, *S(lung),* was calculated to determine the similarity of methylation pattern between a plasma DNA molecule and the lung as follows: $S \left(lung\right) = {\sum }_{i = 1}^{i = n} \left[1-\left({P}_{i}-M{I}_{i , lung}\right)\right] .$

If ***S(liver)*** is the highest among ***S(liver), S(buffy coat), S(colon)*** and ***S(lung),*** the corresponding DNA molecule would be classified as liver origin. Otherwise, it would be classified as hematopoietic, colon, or lung origin, depending on which methylation score is the highest.

### 2. Predicting HCC stages using cancer methylation scores

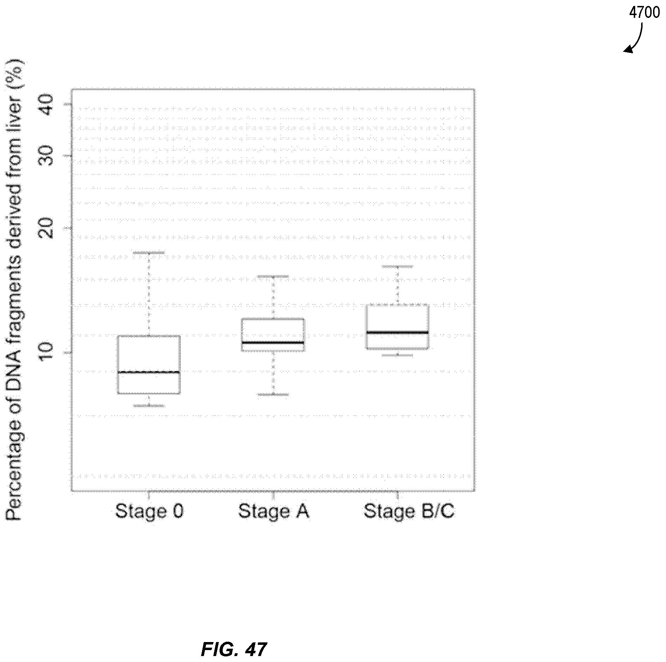

FIG. 47 shows a boxplot 4700 that identifies percentage of DNA molecules determined to be of liver origin in HCC patients of different stages, on the basis of the methylation haplotype analysis according to embodiments of the present disclosure. In particular, FIG. 47 shows the percentage of plasma DNA molecules in patients with different stages of HCC according to the BCLC staging system. As the stage advanced, there was an increasing trend in liver-derived fragments. From the tissue-of-origin analysis of plasma DNA molecules, one could determine the severity of disease, such as the stage of cancer which the patient is suffering from. Thus, the methylation haplotype-based analysis can be effectively used to guide treatment modality selection and prognosis prediction.

Based on the methylation haplotype analysis and methylation scores according to embodiments described in the present disclosure, a metric named the cancer methylation score can be used for reflecting the presence and/or severity of a cancer. We compared the methylation patterns of plasma DNA molecules with the methylation profiles of reference tissues including cancers. A first score, ***S(cancer),*** which reflected the similarity between a DNA molecule and a tumor to be analyzed in terms of methylation patterns, was calculated by the following formula: $S \left(cancer\right) = \frac{{\sum }_{j = 1}^{n} \left(1-\left|{P}_{j}-{r}_{j , cancer}\right|\right)}{n} ,$ where ***Pⱼ*** is the methylation status for a CpG site ***j*** in a plasma DNA molecule, ***r_{j,cancer}*** is the methylation index for the corresponding CpG site in the reference methylome of tumor tissue (e.g., lung tissue, liver tissue, colon tissue, buffy coat), and ***n*** is the total number of CpG sites in a plasma DNA molecule.

Similarly, a second score, ***S(non-cancer),*** was calculated to determine the highest similarity among the comparisons between the methylation pattern of a DNA molecule and the tissue reference methylation profiles including not limited to the buffy coat, the liver tissues, the colon tissues, the lung tissues, etc. by: $S \left(non-cancer\right) = \frac{{\sum }_{j = 1}^{n} \left(1-\left|{P}_{j}-{r}_{j , non - cance}\right|\right)}{n} .$

Finally, both ***S(cancer)*** and ***S(non-cancer)*** were integrated to generate the cancer methylation score using the following formula: $cancer methylation score = \frac{{\sum }_{k = 1}^{T} \left[1+\left({S}_{cancer}-{S}_{non - cance}\right)\right]}{T} ,$ where T is the total number of plasma DNA molecules being analyzed in one individual. The higher the cancer methylation score, the more likely a testing sample would have a cancer. The cancer types in this analysis could include but not limited to HCC, bladder cancer, breast cancer, colon and rectal cancer, endometrial cancer, kidney cancer, leukemia, lung cancer, melanoma, non-Hodgkin lymphoma, pancreatic cancer, thyroid cancer, etc.

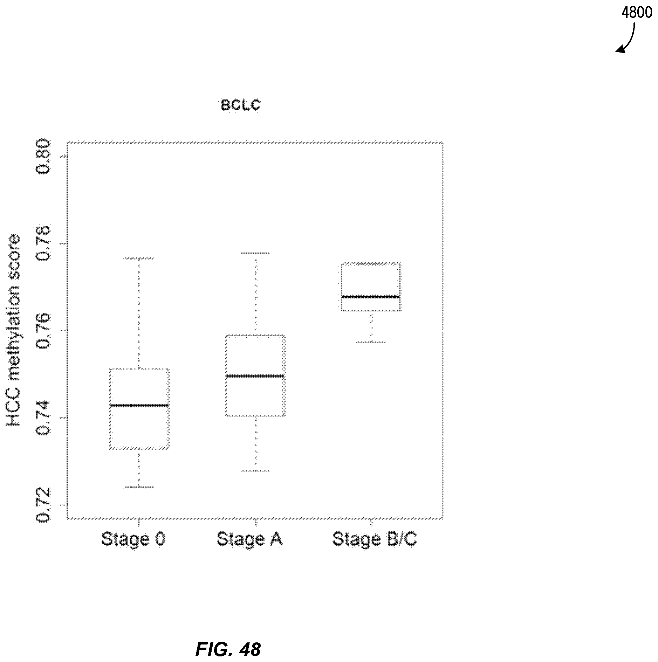

FIG. 48 shows a boxplot 4800 that identifies cancer methylation scores in HCC patients across different stages, according to some embodiments. FIG. 48 shows the cancer methylation score analysis in which cancer methylation scores for patients with HCC were determined (also referred to as "HCC methylation scores"). The HCC patients had different stages of HCC according to the BCLC staging system. As the stage advanced, the HCC methylation score increased progressively. Thus, on the basis of the cancer methylation score analysis, one could determine the severity of a disease, such as the stage of cancer which the patient is suffering from, Thus, the cancer methylation scores can be effectively used to guide treatment modality selection and prognosis prediction.

In some embodiments, a survival analysis is applied to a cohort of HCC patients on the basis of the HCC methylation scores. For example, cases with HCC methylation scores less than or equal to the median HCC methylation scores were classified as "Group A", while cases with HCC methylation scores greater than the median HCC methylation score were classified as "Group B". Kaplan-Meier survival curves can be used for reflecting the survival probability distributions between different groups. As described herein, survival curve corresponds to a graph showing a number or proportion of individuals surviving to each age for a given group. A fast decline of survival curve indicates that the given group die earlier, relative to a slow decline of survival curve.

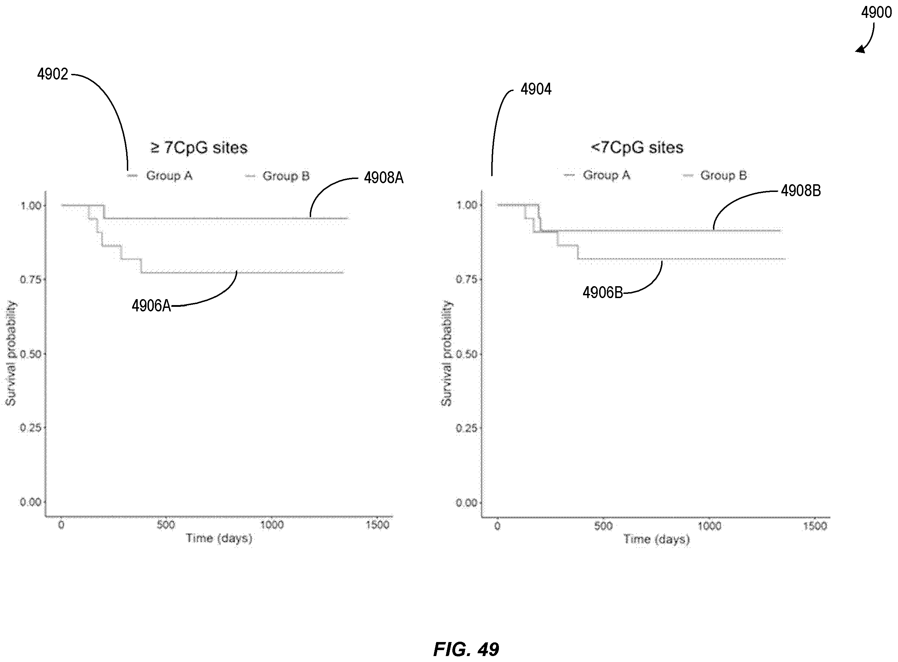

FIG. 49 shows a set of survival curves 4900 that identify survival analysis in HCC patients, according to some embodiments. Curve 4902 shows DNA molecules with at least 7 CpG sites used for HCC methylation score analysis. Curve 4904 shows DNA molecules with less than 7 CpG sites used for HCC methylation score analysis. As shown in FIG. 49, HCC patients in Group B (4906A and 4906B) tended to have worse survival than those in Group A (4908A and 4908B). The use of longer cfDNA molecules with at least 7 CpG sites (median length: 758 bp, curve 4902) could lead to the bigger difference in Kaplan-Meier survival curves between the two groups than the use of shorter DNA molecules with less than 7 CpG sites (median length: 311 bp, curve 4904), suggesting that the use of long cfDNA molecules would be more effective than the use of short cfDNA molecules in prognosis prediction. For example, after two years, the curve 4902 shows that 91% of the Group A patients could survive and 81% of Group B patients could survive, in which the corresponding cancer methylation scores were derived from short cfDNA molecules. In contrast, the curve 4904 shows that 96% of the Group A patients could survive and 77% of Group B patients could survive, in which the corresponding cancer methylation score were derived from long cfDNA molecules. Thus, the cancer methylation score analysis can be used to determine the survival probability of a disease.

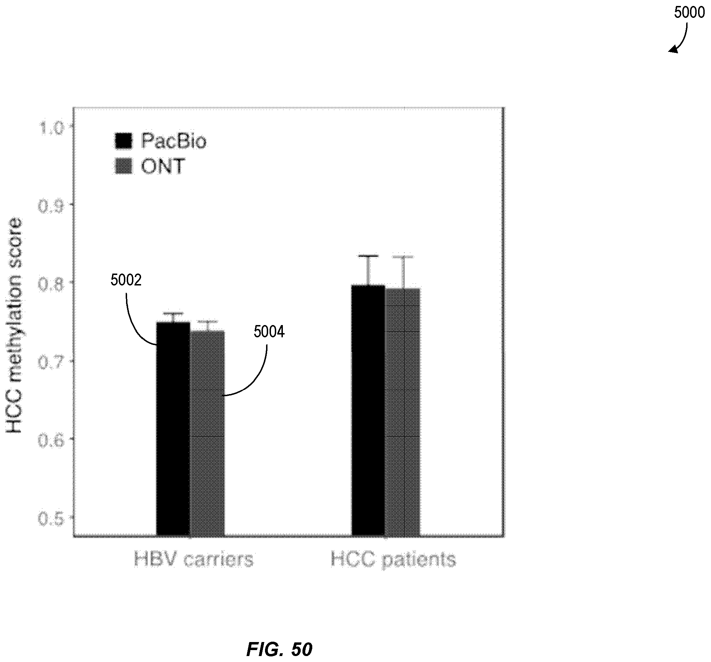

In addition, the cancer methylation scores can be effectively used based on long sequence reads obtained using various sequencing platforms. FIG. 50 shows a boxplot 5000 that identify HCC methylation scores for HBV carriers and HCC patients calculated using data from SMRT-seq (5002) and nanopore sequencing (5004). HCC methylation score was calculated according to embodiments in this present disclosure. As shown in FIG. 50, HCC patients showed significantly higher HCC methylation scores than HBV carriers both in SMRT-seq (P < 0.001, Mann-Whitney U-test) and nanopore sequencing (P = 0.0026, Mann-Whitney U-test).

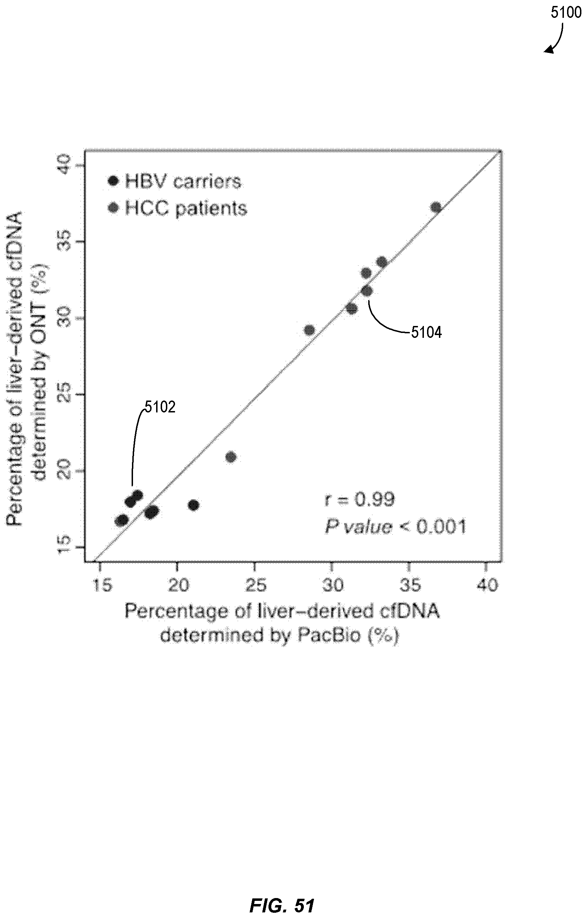

Thus, nanopore sequencing from Oxford Nanopore Technologies (ONT) can also be utilized in the analysis of nucleic acids. To demonstrate the effectiveness of nanopore sequencing, plasma DNA molecules from 8 HCC patients and 6 HBV carriers were sequenced by both nanopore sequencing and SMRT sequencing. FIG. 51 shows a graph 5100 that identifies the percentages of liver-derived cfDNA determined by the single-molecule tissue-of-origin analysis in plasma samples from HBV carriers (5102) and HCC patients (5104) using data from SMRT-seq and nanopore sequencing. As shown in FIG. 51, the percentages of cfDNA molecules being classified as liver origin in HBV carriers and HCC patients using nanopore sequencing showed in line with that determined from SMRT sequencing (Pearson's correlation, r = 0.99, P < 0.001).

### 3. Colorectal cancer (CRC)

In addition to HCC, we analyzed plasma DNA molecules from CRC patients, HCC patients and healthy subjects using SMRT-sequencing and analyzed the tissue-of-origin with methylation scores.

The CpG sites with *MI* difference between the colon tissue and buffy coat greater than 30% were considered informative for downstream analysis. In some embodiments, the *MI* difference includes, but are not limited to, 5%, 10%, 15%, 20%, 25%, 40%, 45%, 50%, 55%, 60%, 65%, 70%, 75%, 80%, 85% 90%, etc. In some embodiments, a scoring system is used to determine the likelihood of a DNA molecule originating from a particular tissue based on the comparison between observed methylation pattern in that molecule and reference methylation profiles. For a DNA molecule carrying n informative CpG sites, a methylation score, *S(colon),* was calculated by the formula as follows: $S \left(colon\right) = {\sum }_{i = 1}^{i = n} \left[1-\left({P}_{i}-M{I}_{i , colon}\right)\right] ,$ where ***Pᵢ*** denotes the methylation status for a CpG site ***i***; ***Pᵢ*** of 0 and 1 represent unmethylated and methylated CpG site, respectively; ***MI_{i,colon}*** denotes the methylation index for a CpG site ***i*** in the colon. A higher ***S(colon)*** indicates a higher likelihood that the DNA molecule would have originated from the colon tissue.

Similarly, another methylation score, ***S(buffy coat),*** was calculated to determine the similarity of methylation pattern between a plasma DNA molecule and the buffy coat as follows: $S \left(buffy coat\right) = {\sum }_{i = 1}^{i = n} \left[1-\left({P}_{i}-M{I}_{i , buffy coat}\right)\right] .$

Similarly, another methylation score, *S(liver),* was calculated to determine the similarity of methylation pattern between a plasma DNA molecule and the liver as follows: $S \left(liver\right) = {\sum }_{i = 1}^{i = n} \left[1-\left({P}_{i}-M{I}_{i , liver}\right)\right] .$

Similarly, another methylation score, *S(lung),* was calculated to determine the similarity of methylation pattern between a plasma DNA molecule and the lung as follows: $S \left(lung\right) = {\sum }_{i = 1}^{i = n} \left[1-\left({P}_{i}-M{I}_{i , lung}\right)\right] .$

If ***S(colon)*** is the highest among ***S(colon), S(buffy coat), S(liver)*** and ***S(lung),*** the corresponding DNA molecule would be classified as colon origin. Otherwise, it would be classified as hematopoietic, liver, or lung origin, depending on which methylation score is the highest.

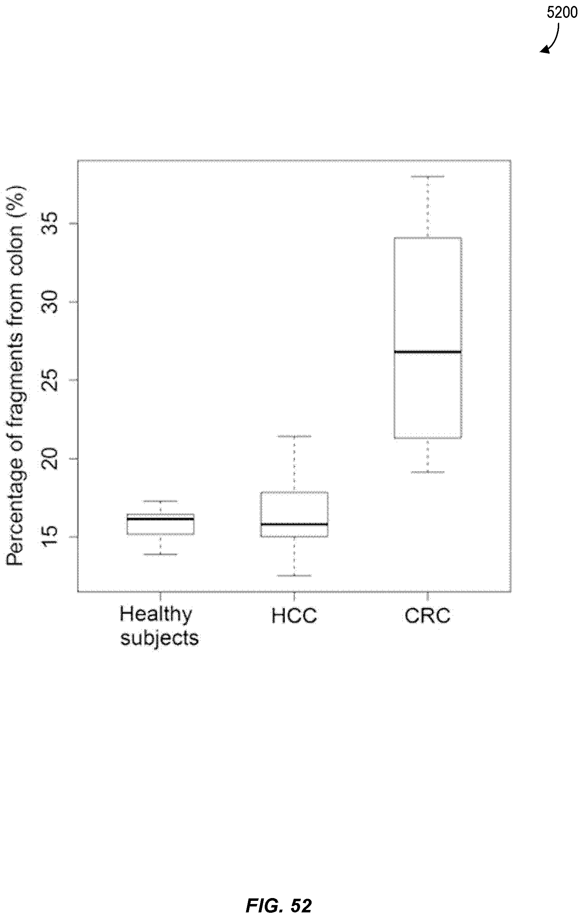

FIG. 52 shows a boxplot 5200 that identifies the percentage of plasma DNA molecules being classified as colon origin based on embodiments presented in this disclosure in 15 healthy subjects, 45 HCC patients and 4 CRC patients. In this example analysis, DNA molecules with at least 7 CpG sites (median length: 896 bp) were included. As shown in FIG. 52, CRC patients show a significantly higher percentage of DNA molecules being classified as colon origin than healthy subjects (P value: 0.0005, Mann-Whitney U-test), and it shows clear separation between CRC and HCC patients (P value: 0.0018, Mann-Whitney U-test). This not only demonstrated the diagnostic power of methylation score analysis presented in the embodiments of this disclosure in distinguishing between subjects with and without colorectal cancer, but also highlighted its specificity in pinpointing the tissue-of-origin of the cancer.

### D. Histological status of disease

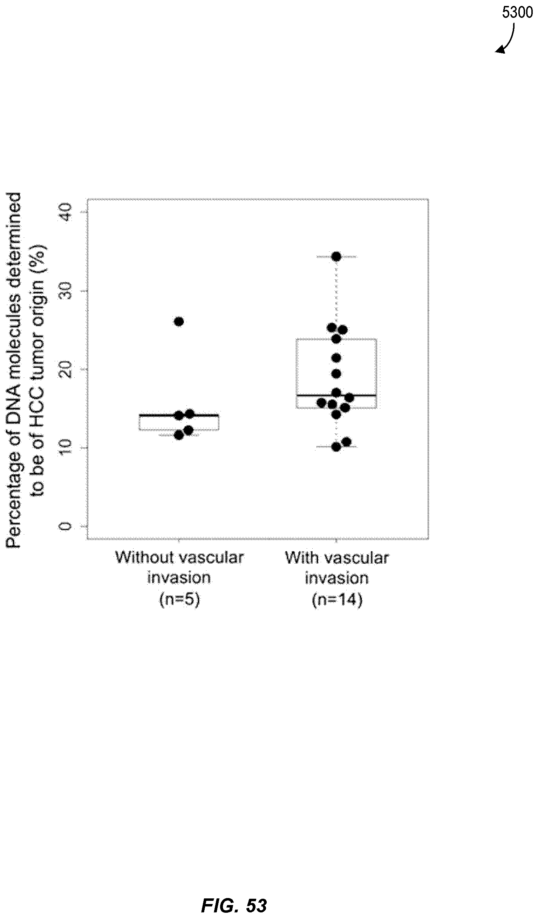

Additional analysis can be performed on the long cell-free DNA molecules to obtain histological status of a disease. FIG. 53 shows a set of bar plots 5300 that identify percentages of DNA molecules determined to be of HCC tumor origin between HCC patients with and without vascular invasion, on the basis of the methylation haplotype analysis according to some embodiments. FIG. 53 shows that the median percentage of DNA molecules determined to be of HCC tumor origin were higher in HCC patients with a vascular invasion (16.68%) than those without (14.08%). The data implied that the tumor-derived DNA molecules identified by the methylation haplotype-based analysis would be used for informing the histological status of a tumor.

### E. Comparison between methylation-based analyses using long cell-free DNA molecules and methylation-based analyses using short cell-free DNA molecules

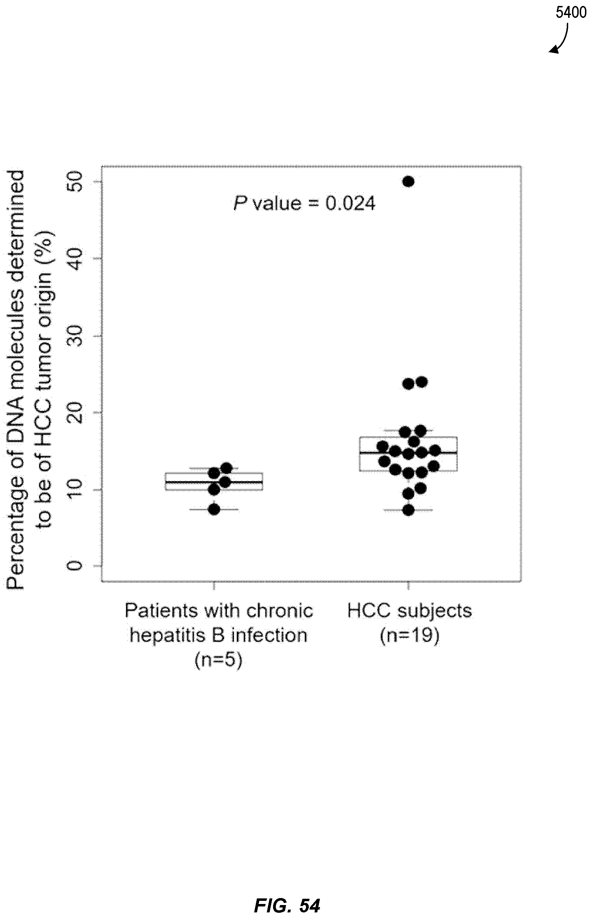

With the use of plasma DNA greater than 1 kb in size, accurate disease classification can be performed for a biological sample. FIG. 54 shows a set of bar plots 5400 that identify a percentage of DNA molecules determined to be of HCC tumor origin, according to some embodiments. FIG. 54 shows that the percentage of DNA molecules determined to be of HCC tumor origin was significantly higher in patients with HCC than the patients without HCC (median: 14.78% versus 10.98%; P value: 0.024, Mann-Whitney U test). The result suggested that the analysis of tissue/tumor origin for each long plasma DNA molecule would serve as a tool for cancer detection.

Further, to assess whether the methylation haplotype analysis of long plasma DNA would have an advantage over the use of short DNA molecules (< 600 bp), the plasma DNA sequence data obtained using PacBio direct methylation HK model analysis (Tse et al. Proc Natl Acad Sci USA. 2021;118: e2019768118) of samples from patients with and without HCC were divided into two groups. The first group of molecules corresponded to a size of > 1 kb, while the second group of molecules corresponded to a size of < 600 bp. For the first group, we attempted to detect the tumor-derived molecules based on the methylation haplotypes according to the embodiments present in this disclosure. For the second group, we calculated the global methylation level (the percentage of methylated CpG sites in a whole human genome using plasma DNA molecule) and determined the liver DNA contribution based on aggregated methylation levels instead of using methylation haplotype information.

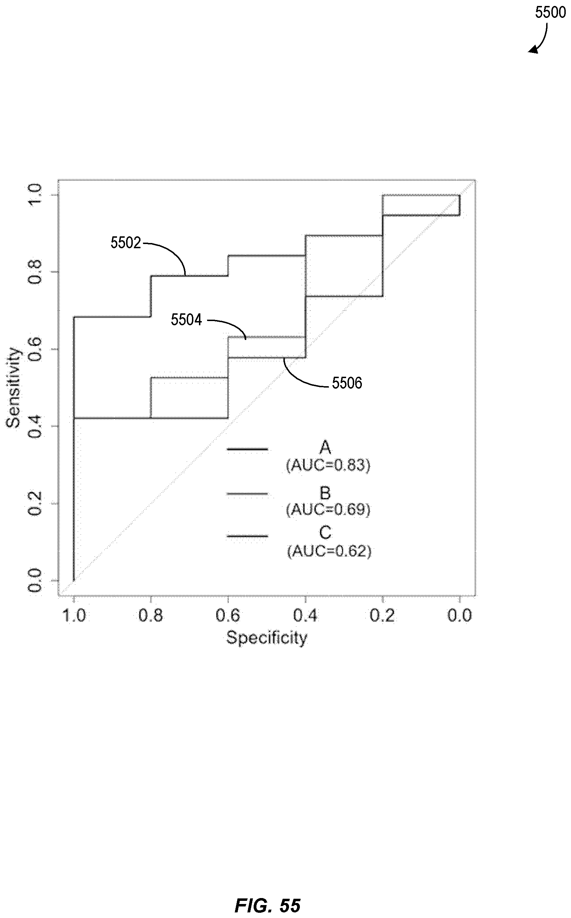

FIG. 55 shows a set of ROC curves 5500 that identify cancer-detection accuracy of an analysis of single molecule methylation sequence data of long cell-free DNA and cancer-detection accuracy of other analyses that use methylation sequence data of short cell-free DNA. Line A (4802) indicates methylation haplotype analysis for those plasma DNA molecules > 1 kb in size according to embodiments present in this disclosure. Line B (4804) indicates the percentage of methylated CpG sites in a whole human genome using plasma DNA molecules < 600 bp. Line C (4806) indicates liver contribution deduced by aggregated methylation level for those plasma DNA molecules < 600 bp instead of methylation haplotype information, using a quadratic programming approach.

FIG. 55 shows that the methylation haplotype analysis using the first group of molecules (e.g., long cell-free DNA molecules) (AUC: 0.83) outperformed the other two methods being tested in the second group of molecules (AUC: < 0.7). These results demonstrated that the methylation haplotype-based analysis for long plasma DNA molecules would be superior to methylation analysis of shorter plasma DNA molecules for cancer detection.

Another comparative analysis was performed to compare performance between methylation haplotype-based analysis using long cell-free DNA molecules and plasma DNA tissue mapping analysis of short cell-free DNA molecules. To obtain the short cell-free DNA molecules, we used short-read bisulfite sequencing technology (Illumina) to sequence 34 controls and 38 HCC subjects, with a median of 211 million 75 bp x 2 paired-end reads (range: 112 - 1,681 million).

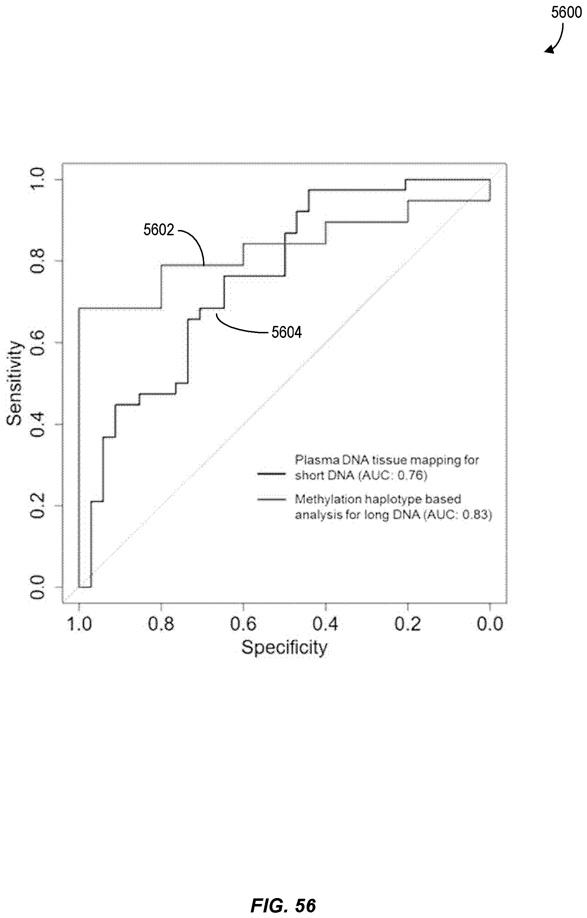

FIG. 56 shows a set of ROC curves 5600 that identify HCC-detection accuracy of a methylation haplotype-based analysis using long DNA 5602 (> 1 kb) and HCC-detection accuracy of a plasma DNA tissue mapping analysis using short-read bisulfite sequencing of short plasma DNA molecules 5604 (< 600 bp). As shown in FIG. 56, for this cohort of samples, the plasma DNA tissue mapping analysis (Sun et al. Proc Natl Acad Sci USA. 2015;112:E5503-5512) gave a AUC of 0.76 in differentiating between patients with and without HCC. Such AUC value shows that performance of analysis based on short plasma DNA molecules inferior to performance of analysis based on the methylation haplotypes of long plasma DNA molecules (AUC: 0.83). Plasma DNA tissue mapping (Sun et al. Proc Natl Acad Sci USA. 2015;112:E5503-5512), making use of the aggregated methylation probability in a genomic region from a population of short DNA molecules, had not taken into account the information and utilities regarding the methylation haplotype of individual long plasma DNA molecule.

### F. Methods for methylation-pattern analysis of long cell-free DNA molecules

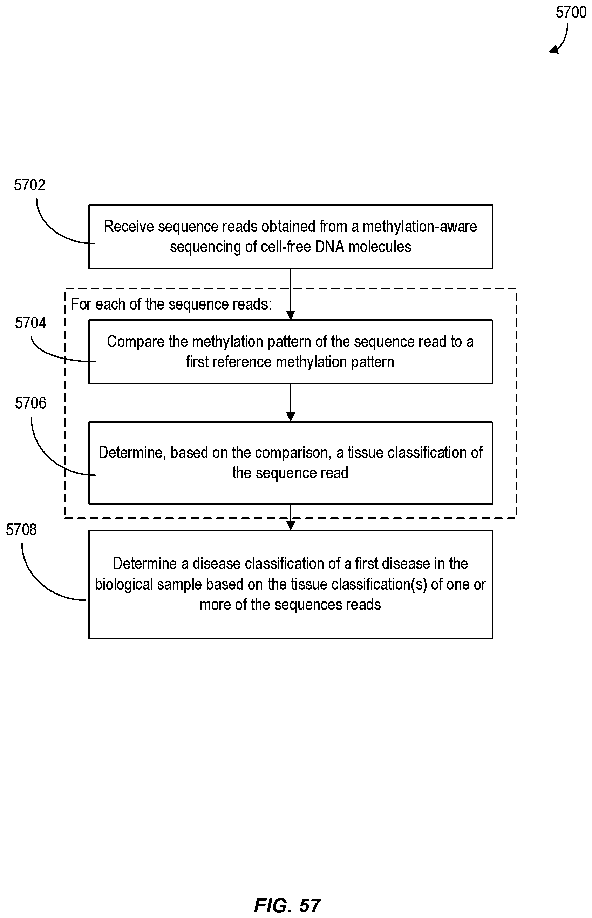

FIG. 57 shows a flowchart of a process 5700 illustrating an example process for analyzing a biological sample of a subject based on methylation patterns of the long cell-free DNA molecules, according to some embodiments. The biological sample can include DNA originating from normal cells and potentially from cells associated from one or more of a plurality of tissue types. In addition, at least some of the DNA is cell-free in the biological sample.

At step 5702, sequence reads obtained from a methylation-aware sequencing of cell-free DNA molecules can be received. The methylation-aware sequencing may include enzymatic treatment. In some instances, the methylation-aware sequencing does not include bisulfite treatment. In other instances, bisulfite treatment is used. Each of the sequence reads can include a methylation pattern of methylation statuses at a set of sites (e.g., CpG sites) on the sequence read. For example, a sequence read can include six CpG sites displaying the methylation pattern as '-M-M-M-U-U-U-' where 'M' represents a methylated state and 'U'' represents an unmethylated state. In another example, a given sequence read can include at least 3 CpG sites. The methylation pattern can include a number of bases (e.g., a specified number of bases) between pairs of sites of the set of sites, as well as the identity of the bases.

The methylation statuses at sites of the cfDNA molecules can be interrogated using bisulfate conversion, as described herein. Apart from bisulfite conversion, other processes known to those skilled in the art can be used to interrogate the methylation status of DNA molecules, including, but not limited to enzymes sensitive to the methylation status (e.g. methylation-sensitive restriction enzymes), methylation binding proteins, single molecule sequencing using a platform sensitive to the methylation status (e.g. nanopore sequencing (Schreiber et al. Proc Natl Acad Sci 2013; 110: 18910-18915) and by the Pacific Biosciences single molecule real time analysis (Tse et al. Proc Natl Acad Sci U S A 2021; 118: e2019768118).

In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least 300 bases, at least 400 bases, at least 500 bases, at least 600 bases, or at least 800 bases.

The set of sites can be various numbers. In some instances, the set of sites for each of the sequence reads can include at least an N number of sites. For example, a given sequence read can include at least 3 CpG sites. Other numbers can be contemplated, including but are not limited to at least 2, 3, 4, 5, 6, 7, 8, 9, 10, 15, 20, 25, 30, 35, 40, 45, 50, or greater than 50 sites. Additionally or alternatively, the sequence reads can correspond to long cell-free DNA molecules having sizes within a first size range (e.g., greater than 500 bps) and include at least an N number of sites (e.g., 3 CpG sites).

Steps 5704 and 5706 can be performed for each sequence read of the sequence reads received from step 5702. At step 5704, the methylation pattern of the sequence read can be compared to a first reference methylation pattern. In some instances, the first reference methylation pattern corresponds to a first tissue type. The first tissue type can be a diseased tissue type. In some instances, the first tissue type is associated with a disease. Additionally or alternatively, the methylation pattern of the sequence read can be additionally compared to each reference methylation pattern of one or more other reference methylation patterns. Each reference methylation pattern can correspond to a tissue type of a plurality of tissue types.

The values for the reference methylation pattern can be binary (e.g., 0 and 1 as in FIGS. 41 or 42) or have fractions (e.g., 0.2 signifying 20% methylation index). The reference pattern can be general to a tissue type or be specific to a particular location. In such a case, a location of the sequence read can be determined. Accordingly, in some embodiments, comparing the methylation pattern to the reference pattern can include determining a location of the sequence read (e.g., relative to a reference genome), in which the reference methylation pattern corresponding to a reference sequence at the location.

In some instances, the comparison between the methylation pattern of the sequence read and the first reference methylation pattern can include calculating a similarity metric based on a difference between a methylation status of a site and a methylation index of the first reference methylation pattern at the same site. The similarity metric can be a distance (e.g., Euclidean distance), cosine similarity, or a methylation score.

In some instances, the methylation status indicates whether a corresponding site is methylated or unmethylated. In this instance, the methylation status includes a binary value indicative of the methylation of the site. The similarity metrics can be determined for the set of sites to determine an aggregate value (e.g., a sum, an average, a median) for the sequence read. The aggregate value can be compared to one or more cutoffs to determine the tissue classification of the sequence read, in which the one or more cutoffs can be identified using reference samples known to be associated with the first tissue type. Additionally or alternatively, the comparison between the methylation pattern and the first reference methylation pattern can include determining a methylation level of the sequence read based on the methylation statuses on the corresponding set of sites, determining a difference based on the methylation level with another methylation level determined from the first reference methylation pattern, and comparing the difference to one or more cutoff values. The methylation level can be a methylation index, a methylation density, count of molecules methylated at one or more sites of the set of sites, or proportion of molecules methylated (e.g., cytosines) at one or more sites of the set of sites.

In some instances, the similarity metric is a methylation score. To calculate the methylation score, a reference methylation profile representing the first reference methylation pattern can be determined by calculating a methylation index for each CpG in the genome from which the first reference methylation pattern was obtained. Then, for each CpG site of the sequence read, a difference between a methylation status (e.g., binary value between 0 and 1) of the CpG site and the corresponding methylation index at the same CpG site can be determined. The determined differences across the CpG sites can be aggregated to determine the methylation score of the sequence read. In some instances, the aggregated value is normalized (e.g., by the total number of CpG sites of the sequence read) to determine the methylation score of the sequence read. The steps for determining the methylation score are additionally described in Sections IV.C and IV.D of the present disclosure.

At step 5706, based on the comparison, a tissue classification of the sequence read can be determined. The tissue classification can be performed as described above for FIGS. 41, 42, and 46. For example, the comparison can be determined by, for each site of the set of sites of the methylation pattern, determining a similarity metric between the methylation status of the site and a methylation index of a corresponding site of the first reference methylation pattern. The similarity metrics across the set of sites can be aggregated to determine an aggregate value (e.g., a sum of similarity metrics). If the aggregated value exceeds a cutoff value, the sequence read can be classified as being associated with the first tissue type. If the aggregated value does not exceed a cutoff value, then the sequence read can be classified as being associated with one of other tissue types. The cutoff value can be determine using one or more reference samples known to be associated with the first tissue type.

In another example, the reference methylation pattern that is the closest can be identified among a set of reference methylation patterns (e.g., the first reference methylation pattern and the one or more other reference methylation patterns) based on the respective aggregate values, and the tissue classification can be determined to be the corresponding tissue type of the reference methylation pattern with the highest aggregate value. In particular, an aggregated value as described in the above paragraph can be determined for each reference methylation pattern. Then, the sequence read can be classified as being derived from a tissue type associated with a reference methylation pattern having the highest aggregate value. The tissue classification can thus indicate that the sequence read is derived (or a level of derivation) from one of the plurality of tissue types. The tissue classification may include a probability that the sequence read is derived from one of the plurality of tissue types. The probability for more than one tissue types can be determined.

Additionally or alternatively, the reference methylation pattern that is the closest can be identified among a set of reference methylation patterns (e.g., the first reference methylation pattern and the one or more other reference methylation patterns) based on a direct comparison of methylation statuses, and the tissue classification can be determined to be the corresponding tissue type of the closest methylation reference pattern. For example, a sequence read with six CpG site displaying the methylation pattern as '-M-M-M-U-U-U-' where 'M' represents a methylated state and 'U'' represents an unmethylated state. Other molecules containing the corresponding CpG sites from other tissues displaying the reference methylations patterns as '-M-U-M-U-M-U-', '-M-U-M-M-U-U-', '-M-U-U-U-M-M-', '-M-M-U-U-M-U-', '-M-M-U-U-U-M-', '-U-M-M-M-U-U-', '-U-U-M-U-M-M-', '-U-U-M-M-M-U-', '-U-U-U-M-M-M-'. But, the reference methylation pattern corresponding to a liver tissue (for example) can be the '-M-M-M-U-U-M-'. In this example, the sequence read can be determined as being associated with liver tissue. Thus, the combination of methylation pattern across a set of CpG sites in a molecule could serve as a 'molecular barcode' indicating the cell identity or a disease status.

If the comparison involves the use of methylation scores, the tissue classification can include determining other methylation scores based on the methylation statuses of the sequence read and methylation indices from other reference methylation patterns. For example, the first reference methylation pattern can correspond to the first tissue type (e.g., liver), a second methylation pattern can correspond to a second tissue type (e.g., buffy coat), a third methylation pattern can correspond to a third tissue type (e.g., colon), and so on. Then, the tissue type associated with the highest methylation score can be determined as the tissue classification of the sequence read. In some instances, the comparisons include comparing methylation scores for two reference methylation patterns (e.g., first and second reference methylation patterns), in which the first reference methylation pattern corresponds to the first tissue type and second reference methylation pattern corresponds to one or more other tissue types. The steps for performing tissue classification using the methylation score is additionally described in Section IV.C of the present disclosure.

If the first tissue type is a diseased type, a first methylation score (e.g., S(cancer) score) corresponding to reference methylation profile of the diseased tissue (e.g., HCC) can be determined and a second methylation score (e.g., S(non-cancer) score) corresponding to reference methylation profile of the healthy tissue (e.g., non-HCC) can be determined. Then, to perform disease classification, the first and second methylation scores determined for the sequence reads can be used together to determine a cancer methylation score. The steps for performing disease classification using the cancer methylation score is additionally described in Section IV.D of the present disclosure.

At step 5708, a disease classification of a disease in the biological sample can be determined based on the tissue classifications of the sequences reads. If the tissue type is a diseased tissue type, the tissue classifications and the disease classification can be equivalent. For the disease classification, the cancer methylation score determined in step 5706 can be used to determine the disease classification. The disease can be cancer. Determining the disease classification can include determining whether vascular invasion exists from cancer. In some instances, determining the disease classification includes: (i) determining a first amount of sequence reads classified as being derived from the first tissue type; and (ii) determining a classification of the disease in the biological sample based on comparing the first amount to one or more reference values.

The one or more reference values can be determined from reference samples with known classification of the disease. If the first amount of sequence reads exceed the one or more cutoff values, the subject can be classified as having the disease. In contrast, if the first amount of sequence reads does not exceed the one or more cutoff values, the subject can be classified as having the disease.

The amount can be sum of probabilities for the first tissue type. For example, if a tissue classification corresponds to a probability value or a methylation score, the first amount of sequence reads can include the sum of the probability values or the methylation scores of sequence reads classified as being derived from the first tissue type. In some instances, the sum is determined based on probability value or the methylation scores of sequence reads that are above a probability threshold.

In some instances, the disease classification is determined by comparing the first amount of sequence reads corresponding to the first reference methylation pattern to amounts corresponding to one or more other reference methylation patterns, in which each of the one or more other reference methylation patterns is associated with one or more other tissue types. The one or more other amounts of sequence reads classified as being derived from one or more other tissue types can be determined. Based on the comparison between the first amount of sequence reads and the one or more other amounts, the classification of the disease in the biological sample can be determined. For example, if the first amount of sequence reads is the highest amount, the subject can be determined as having the disease of the first tissue type.

The classification of the disease can include a classification of a severity of the disease (e.g., no disease, early stage, intermediate stage, advanced stage). For example, the classification of the disease can include a stage of cancer in accordance with BCLC stages. The classification can then select one of the stages. Accordingly, the classification can be determined from a plurality of stages of disease (e.g., one of BCLC stages for HCC). In some instances, the disease is cancer. As examples, the cancer can be hepatocellular carcinoma, lung cancer, breast cancer, gastric cancer, glioblastoma multiforme, pancreatic cancer, colorectal cancer, nasopharyngeal carcinoma, and head and neck squamous cell carcinoma.

### V. SINGLE-MOLECULE METHYLATION LEVEL ANALYSIS OF LONG CELL-FREE DNA MOLECULES FOR PREDICTION OF DISEASE SEVERITY

Assessment of disease severity is crucial in guiding treatment modality decisions, prognosis prediction and monitoring. Taking hepatocellular carcinoma (HCC) as an example, patients with early stage HCC (e.g. Barcelona Clinic Liver Cancer (BCLC) 0/A) have the expected median survival of >5 years, and are often offered curative treatment like ablation, resection and transplantation. In contrast, patients with advanced stage HCC (e.g. BCLC C) have the expected median survival of more than 2 years and are often offered systemic treatment (Reig. et al. J. Hepatol. 2022;76:681-693).

According to embodiments of this present disclosure, the analyses based on methylation of cfDNA molecules, including but not limited to single molecule methylation pattern analysis and cfDNA tissue-of-origin analysis, could be used for prognosticating the severity of a disease, including but not limited to prediction of cancer stages.

### A. Disease classification using single-molecule methylation level

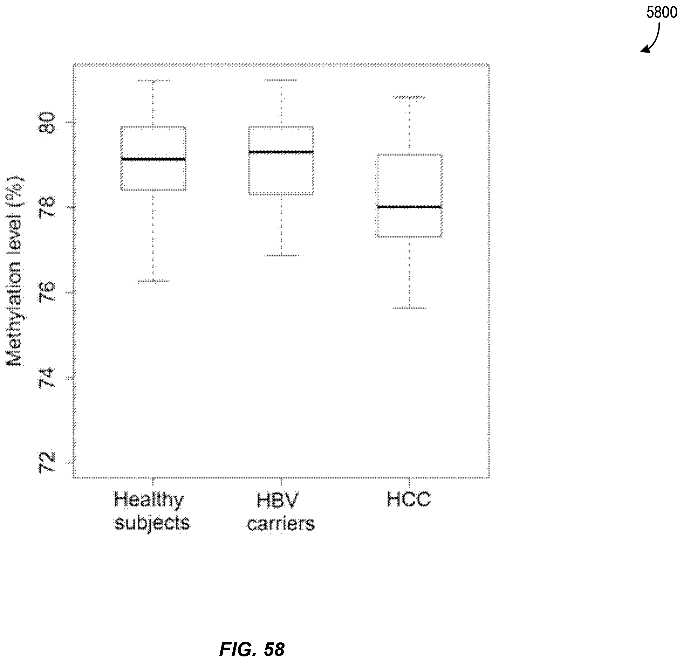

We used single-molecule real-time sequencing (SMRT-Seq) to sequence plasma DNA molecules from 45 HCC patients, 13 hepatitis B virus (HBV) carriers and 15 healthy individuals. FIG. 58 shows a boxplot 5800 that identifies single-molecule methylation levels in different groups of individuals in single-molecule real-time sequencing (SMRT-Seq), according to some embodiments. Plasma DNA molecules from HCC patients had significantly lower mean single-molecule methylation levels than the control individuals (P value: 0.005, Mann-Whitney U-test) (FIG. 58). The single-molecule level herein was defined by the percentage of CpG sites determined to be methylated in a single molecule. For example, if a DNA molecule contained 10 CpG sites and 5 of them were determined to be methylated, the single-molecule methylation level would be 50% (i.e. 5/10*100%). The single-molecule methylation level can be determined for each of the sequence reads of a given biological sample, at which a statistical value (e.g., mean, median) can be determined from the single-molecule methylation levels. These data in FIG. 58 suggested that the use of single molecule methylation levels allowed for the detection of HCC patients.

In some embodiments, as SMRT-seq enables obtaining more long cfDNA molecules based on criteria including but not limited to sizes of molecules, the number of CpG sites and methylation levels. The criteria can be used to further enhance diagnostic performance, which were not suitable for Illumina sequencing platforms that were not capable of sequencing long cfDNA molecules (e.g. >600 bp).

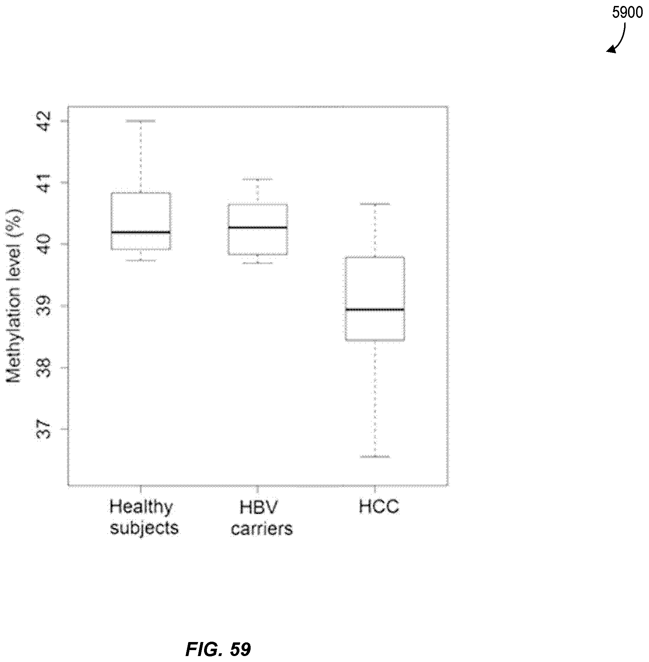

FIG. 59 shows a boxplot 5900 that identifies single-molecule methylation levels in DNA molecules with sizes >500 bp, containing at least 3 CpG sites and with methylation level ≤60% in SMRT-Seq. As shown in FIG. 59, we analyzed the mean single-molecule methylation levels in healthy subjects, HBV carriers and HCC patients, respectively, for those DNA molecules with sizes >500 bp, containing at least 3 CpG sites and methylation level ≤60%. We found that HCC patients exhibited significantly lower methylation levels compared with patients without HCC (P-value: 2.132 x 10⁻⁸, Mann-Whitney U-test).

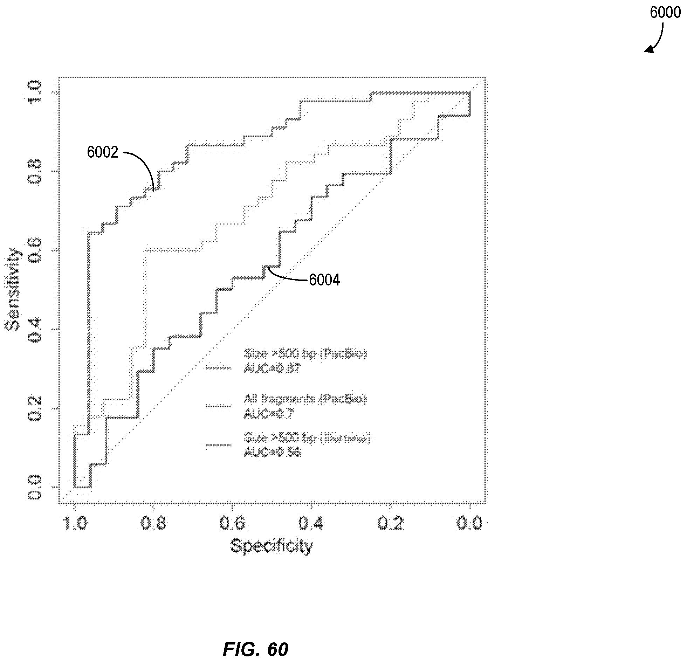

FIG. 60 shows ROC curves 6000 that identify performance of single-molecule methylation levels in distinguishing between HCC and non-HCC subjects in SMRT-Seq and short-read sequencing (e.g., Illumina sequencing), according to some embodiments. As shown in FIG. 60, compared with using all DNA molecules without size selection, such a selective analysis based on molecules with a size of >500 bp (5302) enhanced the diagnostic performance, with an area under the receiver operating characteristic (ROC) curve (AUC) improved from 0.7 to 0.87. In addition, molecules >500 bp used for such methylation analysis based on short-read sequencing (5304) only gave a AUC of 0.56 (Jiang et al. Cancer Discov. 2020; 10:664-673), which was much worse than the embodiments disclosed herein.

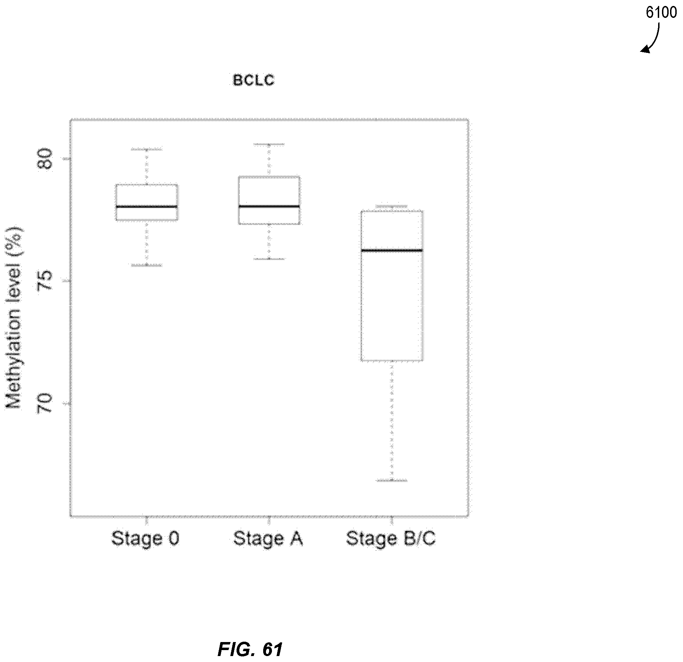

FIG. 61 shows a boxplot 6100 that identify single-molecule methylation levels in HCC patients of different Barcelona Clinic Liver Cancer (BCLC) stages. FIG. 61 shows that the mean single-molecule methylation levels in patients varied with different stages of HCC according to the BCLC staging system. As the cancer stage advanced, the single-molecule methylation levels decreased progressively. The single-molecule methylation level of plasma DNA molecules can thus be used to inform the severity of a disease, such as the stage of cancer which the patient is suffering from. In effect, single-molecule methylation levels of plasma DNA molecules can guide treatment modality selection and prognosis prediction. In some instances, single-molecule methylation levels for long plasma DNA molecules (e.g., sizes greater then 600 bp) are used to improve accuracy of determining the severity of the disease.

### B. Methods for determining a disease classification using single-molecule methylation levels in DNA molecules

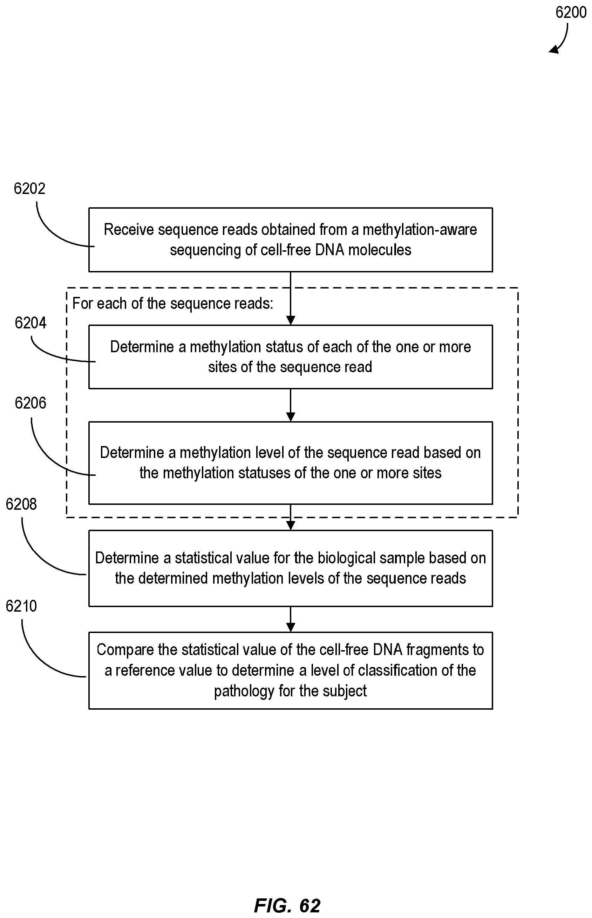

FIG. 62 shows a flowchart 6200 illustrating an example process for determining a disease classification using single-molecule methylation levels in DNA molecules, according to some embodiments. The biological sample can include DNA originating from normal cells and potentially from cells associated from one or more of a plurality of tissue types. In addition, at least some of the DNA is cell-free in the biological sample.

At step 6202, sequence reads obtained from a methylation-ware sequencing of cell-free DNA molecules can be received. Each of the sequence reads can include methylation statuses corresponding to a set of sites (e.g., CpG sites) on the sequence read. The methylation-ware sequencing may include single-molecule sequencing or nanopore sequencing that can be used to identify a methylation status for each CpG site of each cell-free DNA molecule. For example, single-molecule real-time sequencing (SMRT-Seq) or nanopore sequencing can be used to sequence the cell-free DNA molecules to obtain the sequence reads. Additionally or alternatively, other processes can be used to identify the methylation statuses of the CpG sites, including bur are not limited to bisulfite conversion, enzymes sensitive to the methylation status (e.g. methylation-sensitive restriction enzymes), and methylation binding proteins.

In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least 300 bases, at least 400 bases, at least 500 bases, at least 600 bases, or at least 800 bases.

In some instances, each of the sequence reads includes one or more sites having one or more methylation statuses, from which a methylation level of the corresponding cell-free DNA molecule can be determined. Each site of one or more sites can be associated with a methylation status. For example, one or more sites can be CpG sites, and each site can be a CpG site at which a particular methylation status is determined. In some instances, the one or more sites for each of the sequence reads include at least an N number of sites. For example, a given sequence read can include at least 3 CpG sites. Other numbers can be contemplated, including but are not limited to 2, 3, 4, 5, 6, 7, 8, 9, 10, 15, 20, 25, 30, 35, 40, 45, 50, or greater than 50 sites. Additionally or alternatively, the sequence reads can correspond to long cell-free DNA molecules having sizes within a first size range (e.g., greater than 500 bps) and include at least an N number of sites (e.g., 3 CpG sites). The steps for determining methylation statuses of the set of sites are additionally described in step 5702 of FIG. 57.

Steps 6204 and 6206 can be performed for each sequence read of the sequence reads received from step 6202. At step 6204, a methylation status for each of the one or more sites of the sequence read can be determined. In some instances, the methylation status of a given site includes a binary value (e.g., 0 and 1 as in FIG. 58) that identifies whether the site is methylated or unmethylated.

At step 6206, a methylation level of the sequence read can be determined based on the methylation statuses of the one or more sites. The methylation level can be a methylation index, a methylation density, count of molecules methylated at one or more sites of the set of sites, or proportion of molecules methylated (e.g., cytosines) at one or more sites of the set of sites. For example, the methylation level identifies a percent methylation of CpG sites, which is determined based on a count of methylated CpG sites and a total count of CpG sites of the sequence read. For example, if a DNA molecule contained 10 CpG sites and 5 of them were determined to be methylated, the single-molecule methylation level would be 50 % (i.e. 5/10*100%).

At step 6208, a statistical value for the biological sample can be determined based on the determined methylation levels of the sequence reads. For example, the statistical value can be a mean, median, or average of the methylation levels corresponding to the sequence reads. Additionally or alternatively, the statistical value can be an aggregate value (e.g., sum) determined from the methylation levels of the sequence reads.

At step 6210, the statistical value of the cell-free DNA fragments is compared to a reference value to determine a level of classification of the pathology for the subject. The reference value may comprise or be used to determine a cutoff or a threshold value. The cutoff or threshold may be derived from a reference value that is representative of a particular classification or discriminates between two or more classifications. In some instances, the reference value is determined using a reference sample with known classification of the pathology. A subject with statistical values above or below the cutoff (threshold) value may be classified as carrying a genetic disorder. The cutoff value may be defined by a statistical metric (e.g., significance, P-value, Z-score) relative to a reference value.

As examples, the pathology can be a cancer. As examples, the levels can be no cancer, early stage, intermediate stage, or advanced stage. The classification can then select one of the stages. Accordingly, the classification can be determined from a plurality of stages of cancer (e.g., one of BCLC stages). As examples, the cancer can be hepatocellular carcinoma, lung cancer, breast cancer, gastric cancer, glioblastoma multiforme, pancreatic cancer, colorectal cancer, nasopharyngeal carcinoma, and head and neck squamous cell carcinoma.

### VI. MACHINE-LEARNING TECHNIQUES FOR DISEASE CLASSIFICATION BASED ON METHYLATION PATTERNS IN LONG CELL-FREE DNA MOLECULES

In some embodiments, the pattern recognition analysis for methylation haplotypes could be implemented with the use of machine learning models which could extract the useful information from methylation haplotypes for the classification of patients with and without cancers. Sequence reads can be obtained from a methylation-aware sequencing of cell-free DNA molecules that does not include bisulfite treatment. This is because chemical reactions of the bisulfite treatment may prevent one from obtaining sequence reads that correspond to long cell-free DNA molecules (e.g., > 600 bp). In some instances, the methylation pattern for each long cell-free DNA molecule is transformed into a matrix of values, in which the long cell-free DNA molecule can be associated with a particular tissue type. The matrices can be used for training a machine-learning model for determining a tissue classification.

Compared to previously known techniques, the machine-learning model can identify that certain sites of the long cell-free DNA molecules are more predictive in disease classification than other sites. Further, an increased number of CpG sites in long cell-free DNA molecules allow the machine-learning model to be trained with more diverse methylation patterns. In effect, the machine-learning model can determine a more accurate classification of a disease compared to another model trained on short DNA molecules that would generally have fewer CpG sites.

The machine learning models could include, but not limited to, convolutional neural network (CNN), linear regression, logistic regression, deep recurrent neural network (e.g., fully-connected recurrent neural network (RNN), Gated Recurrent Unit (GRU), long short-term memory, (LSTM)), transformed-based methods (e.g. XLNet, BERT, XLM, RoBERTa), Bayes's classifier, hidden Markov model (HMM), linear discriminant analysis (LDA), k-means clustering, density-based spatial clustering of applications with noise (DBSCAN), random forest algorithm, adaptive boosting (AdaBoost), eXtreme Gradient Boosting (XGBoost), support vector machine (SVM), or a composite model comprising one or more models proposed above.

### A. Machine-learning model

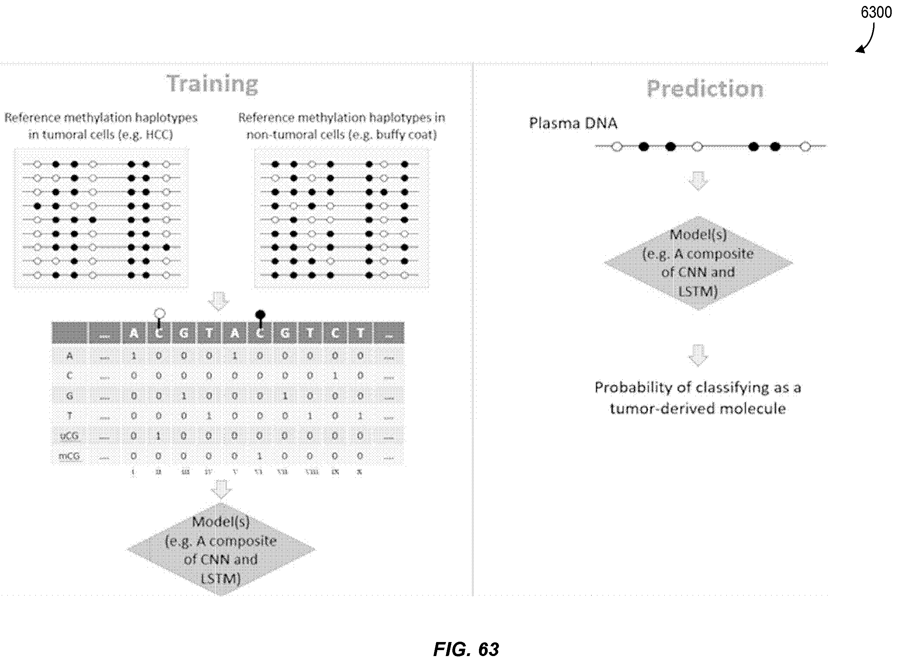

FIG. 63 shows an illustrative diagram 6300 for pattern recognition of methylation haplotypes using machine-learning models, according to some embodiments. In some embodiments, the machine-learning model is a composite model that includes CNN followed by LSTM. In particular, FIG. 63 shows an example of the use of pattern recognition of methylation haplotypes for classifying tumoral and non-tumoral DNA in plasma of patients with cancers.

### 1. Training Data

The long methylation haplotypes (e.g. > 5 kb) from tumoral cells (green) and non-tumoral cells were obtained with the use of EM-seq (blue). A horizontal line (DNA) with a series of filled and unfilled dots (i.e. methylated and unmethylated CpG sites) represents a methylation haplotype. In some instances, to obtain the DNA fragments from tumor cells, sonication is performed on the tissue DNA to obtain molecules with a certain size (e.g., 5 kb, 10 kb). Methylation-aware sequencing with bisulfite sequencing can be used for obtaining sequence reads used for the training data. In some instances, the non-tumor DNA fragments is obtained from T-cells, B-cells, neutrophils, lung tissue, liver, etc.

Each long methylation haplotype would be programmed into a data matrix for which contained both the sequence context and methylation patterns. The data matrix can include one-hot encoding of bases and identify methylation status of each CpG site of a corresponding cell-free DNA molecule. The term "one-hot encoding" refers to a technique for quantifying categorical data such that the categorical data is transformed into a numerical representation. In particular, the technique can include producing a vector (e.g., a base) with length equal to the number of categories in the data set. For example, if a base belongs to the T category, then components of this vector are assigned the value 0 except for the T component, which is assigned a value of 1. One-hot encoding can allow one to keep track of the categories in a numerically meaningful way.

The first row of the matrix indicated the sequence information, '...ACGTACGTCT...', wherein '...' indicated those bases were left out for the sake of simplicity. For illustration purposes, the first CpG site was unmethylated and the second CpG site was methylated. At the column of 'i', corresponding to the 'A' nucleotide, '1' was filled in the intersection place (called cell herein) between the column of 'i' and a row of 'A'. The other cells in the same column were filled in by '0'. At the column of 'ii', corresponding to the 'C' nucleotide immediately followed by a 'G' nucleotide which was unmethylated, '1' was filled in the cells corresponding to the row of 'uCG'. Other cells at the column of 'ii' were filled by '0'. At the column of 'vi', corresponding to the 'C' nucleotide immediately followed by a 'G' nucleotide which was methylated, '1' was filled in the cells corresponding to the row of 'mCG'. Other cells at the column of 'vi' were filled by '0'. Based on these rules, the data matrix comprising sequence context and methylation patterns associated with a methylation haplotype was constructed.

### 2. Training

In some embodiments, a number of data matrices, obtained from tumoral cells and non-tumoral cells, respectively, could be used to train a machine-learning model for differentiating tumor-associated methylation haplotype and non-tumor-associated methylation haplotype. The trained machine-learning model could be used for determining the likelihood of a methylation haplotype present in a plasma DNA being derived from tumoral cells or non-tumoral cells. Additionally or alternatively, a number of data matrices, obtained from plasma DNA associated with patients with and without cancers, respectively, could be used to train a machine-learning model for differentiating tumor-associated methylation haplotype and non-tumor-associated methylation haplotype.

In some embodiments, a 2-dimensional (2-D) matrix with the shape of [length of molecules x 6] was input to a convolutional neural network (CNN). The matrix was passed to a 1D convolutional layer of the CNN which was composed of 128 filters with a kernel size of 10. The activation function of rectified linear unit (ReLU) was adopted. After that, a maximum pooling layer with a pool size of 2 and stride of 2 is applied. Subsequently thereafter, a bidirectional long short-term memory (LSTM) layer with 32 units was used. The LSTM can be interpreted in a manner that each time point corresponds to a location of the CpG site. Thus, methylation statuses of a sequence of CpG sites can be analyzed by the LSTM such that it is trained to associate a methylation pattern with a presence of disease. In some instances, a bidirectional LSTM is used. Hyperbolic tangent (tanh) activation function is adopted in this layer. The output was then flattened and passed to 2 dense layers with 128 and 64 neurons, respectively. ReLU was adopted for the activation function for both dense layers. The final layer employed a single neuron with a sigmoid activation function and outputs the probability value indicating the likelihood of being as a tumoral and non-tumoral DNA molecule. The higher the probability value corresponding to a plasma DNA molecule, suggested that plasma DNA molecule would have a higher likelihood of being derived from a tumor.

In some embodiments, the cut-off of the probability could be greater than a certain value to detect a tumor-derived plasma DNA molecule, including but not limited to 0.5, 0.6, 0.7, 0.8, and 0.9, etc. The cut-off of the probability could be less than a certain value to detect a non-tumor-derived plasma DNA molecule, including but not limited to 0.5, 0.4, 0.3, 0.2, and 0.1, etc. In some embodiments, the activation functions could include but not limited to, rectified linear unit (ReLU), exponential linear unit (ELU), leaky rectified linear unit (Leaky ReLU), parametric rectified linear unit (Parametric ReLU), scaled exponential linear unit (SELU), Gaussian Error Linear Unit (GELU), hyperbolic tangent(tanh) function, sigmoid function, softmax function, swish function, etc.

In some embodiments, when the model was trained by the data matrices derived from the methylation haplotypes from different tissues, including but not limited to neutrophils, T cells, B cells, megakaryocytes, erythrocytes, monocytes, NK cells, liver, lungs, esophagus, heart, pancreas, colon, small intestines, adipose tissues, adrenal glands, brain, breast, kidney, bladder, thyroid, prostate, uterus, etc., such a model could be used for determining the tissue/tumor of origin for each plasma DNA molecule based on its methylation haplotype.

### B. Example

To assess the feasibility and potential performance of using the above-proposed pattern recognition of methylation haplotypes, we simulated various numbers of methylation haplotypes of plasma DNA molecules derived from HCC tumors and buffy coat (i.e. white blood cells) samples, respectively. A probabilistic model was used to simulate methylation haplotypes of a plasma DNA molecule with a certain size (e.g. 2-kb). The methylation status of ***k*** CpG sites **(*k*** ≥ 1) on a plasma DNA molecule was denoted as ***M*** = **(*m₁, m₂, ..., mₖ*),** where **mᵢ** was 0 (for unmethylated status) or 1 (for methylated status) at the CpG site ***i*** on a plasma DNA molecule. The probability of M related to a plasma DNA molecule derived from the HCC tumors could depend on the prior methylation distributions in the HCC tissues. The probability of M related to a plasma DNA molecule derived from the buffy coat could depend on the prior methylation distributions in the buffy coat.

The prior methylation distributions in the HCC tissues and buffy coat samples for those corresponding CpG sites at ***1, 2,** ..., **k*** would follow beta distributions. The beta distribution is parameterized by two positive parameters ***α*** and ***β,*** denoted by ***Beta(α, β).*** The values derived from beta distribution would range from 0 to 1. Based on high-depth bisulfite sequencing data for a tissue of interest, the parameters ***α*** and ***β*** were determined by the numbers of sequenced cytosines (methylated) and thymines (unmethylated) at each CpG site for that particular tissue, respectively. For the HCC tumor tissues, such a beta distribution was denoted as ***Beta(α^{T}, β^{T}).*** For the buffy coat samples, such a beta distribution was denoted as ***Beta(α^{N}, β^{N}).*** We sampled the methylation status of ***k*** CpG sites (k ≥ 1) for tumor-derived and non-tumor-derived plasma DNA molecules from ***Beta(α^{T}, β^{T})*** and ***Beta(α^{N}, β^{N}),*** respectively. The prior probability distributions regarding co-methylation and co-unmethylation within a certain nucleotide distance could be integrated into the simulation. For example, 79.6%, 75.6%, 71.6%, 68.6%, 66.4%, 65.1%, 62.5%, 61.1%, and 60.7% of two consecutive CpG sites were found to be co-methylated or co-unmethylated within a nucleotide distance of 5 bp, 10 bp, 20 bp, 30 bp, 40 bp, 50 bp, 100 bp, 200 bp and 500 bp, respectively.

We simulated tumor-derived DNA molecules and non-tumor-derived DNA molecules contributing to the plasma DNA pool, with a number of different depths including 1x, 5x, 10x, 20x, 25x, 30x, 35x, 40x, 50x, 60x, 70x, 80x, 90x, and 100x across 5,000 randomly selected genomic regions. At a certain depth, the data matrices were constructed according to the embodiments in this disclosure for tumor-derived DNA molecules and non-tumor-derived DNA molecules, respectively. During the training process, the output values corresponding to the data matrices of tumor-derived DNA molecules were labeled as '1'. The output values corresponding to the data matrices of non-tumor-derived DNA molecules were labeled as '0'. The data matrices, related to tumor-derived and non-tumor-derived DNA molecules, were used to train a deep learning model comprising CNN and LSTM. Model parameters for deep learning were determined by minimizing the prediction error between the predicted and expected output values. The trained model was applied to classify a newly-simulated plasma DNA which was not used during the training process. The area under the receiver operating characteristic curve (AUC) was used for assessing the model performance with different depths.

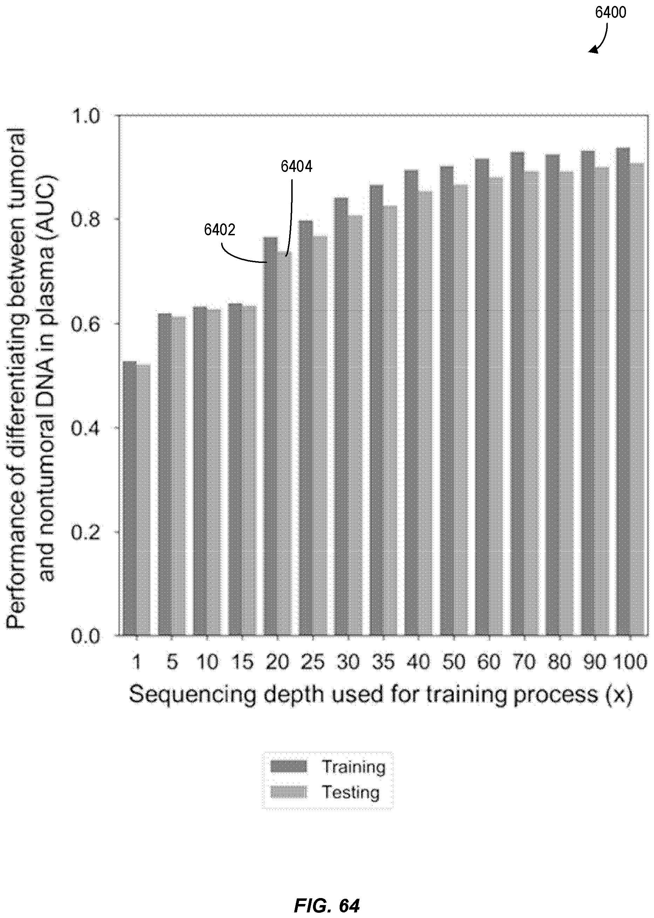

FIG. 64 shows a set of bar graphs 6400 that identify performance of the machine-learning model for differentiating between tumoral and non-tumoral DNA in plasma across different sequencing depths used in the training process. For each sequencing depth (X-axis), a blue bar 6402 identifies AUC of the machine-learning model based on classifying the training data, and an orange bar 6404 identifies AUC of the machine-learning model based on classifying the testing data. FIG. 64 shows that the performance of differentiating between tumoral and non-tumoral DNA in plasma was improved as the sequencing depth used in the training increased. The plateaued performance arrived at a sequencing depth of 70x used in the training, with an AUC of 0.90. The performance using the deep learning model on the basis of methylation haplotypes (AUC: 0.90) was significantly better than the least methylation mismatches approach (AUC: 0.8). These data suggested that the proposed pattern recognition of methylation haplotypes would be a generic and informative approach for detecting cancer-derived cell-free DNA from any genomic regions.

In some embodiments, plasma DNA molecules from the differentially methylated regions (DMRs) between tumoral and non-tumoral genomes were selectively analyzed, which would further enhance the model performance. We obtained 5,000 DMRs that were hypermethylated in tumoral genomes, compared with buffy coat genomes (e.g. at least 20% difference in methylation levels). We simulated tumor-derived DNA molecules and non-tumor-derived DNA molecules with depths of 1x, 5x, 10x, 20x, 25x, 30x, 35x, 40x, 50x, 60x, 70x, 80x, 90x, and 100x for those 5,000 DMRs.

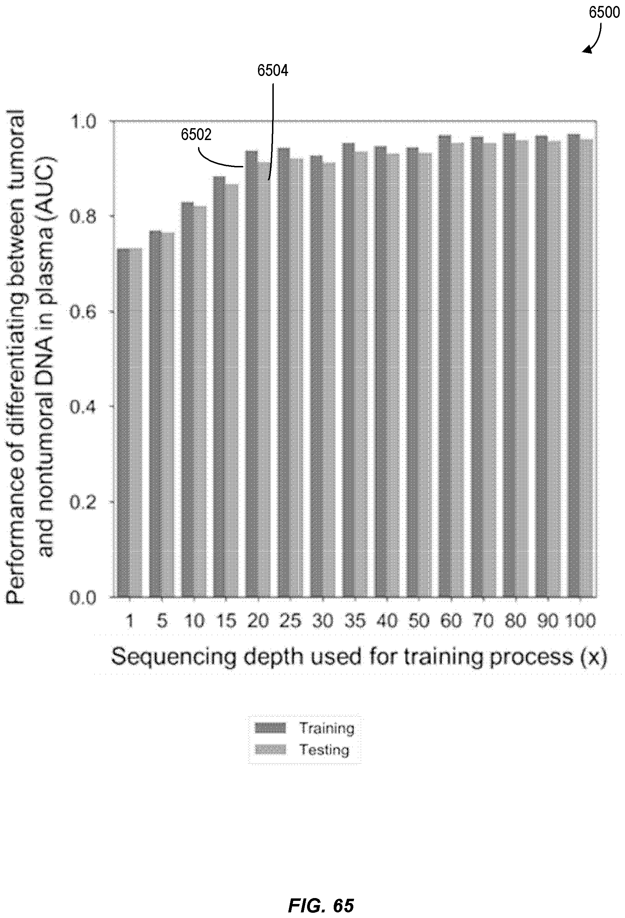

FIG. 65 shows a set of bar graphs 6500 that identify performance of the machine-learning model for differentiating between tumoral and non-tumoral DNA in plasma, in which the machine-learning was trained using differentially methylated regions across different sequencing depths. For each sequencing depth (X-axis), a blue bar 6502 identifies AUC of the machine-learning model based on classifying the training data, and an orange bar 6504 identifies AUC of the machine-learning model based on classifying the testing data. FIG. 65 shows that the performance of differentiating between tumoral and non-tumoral DNA in plasma was improved as the sequencing depth used in the training increased. The plateaued performance arrived at a sequencing depth of 30x used in the training, with an AUC of 0.91 (FIG. 65), which outperformed the non-DMRs genomic regions at the same sequencing depth (FIG. 64). In some embodiments, if one used 0.5 as a cut-off of the probability of classifying as a tumor-derived molecule, 86% specificity and 81% sensitivity could be achieved, with a sequencing depth of 30x. By contrast, 91% specificity and 87% sensitivity could be achieved, with a sequencing depth of 100x. The performance using the deep learning model on the basis of methylation haplotypes (AUC: 0.91) was significantly better than the least methylation mismatches approach (AUC: 0.87). These data suggested that, in some embodiments, the selective analysis of a subset of the genome would enhance cancer detection.

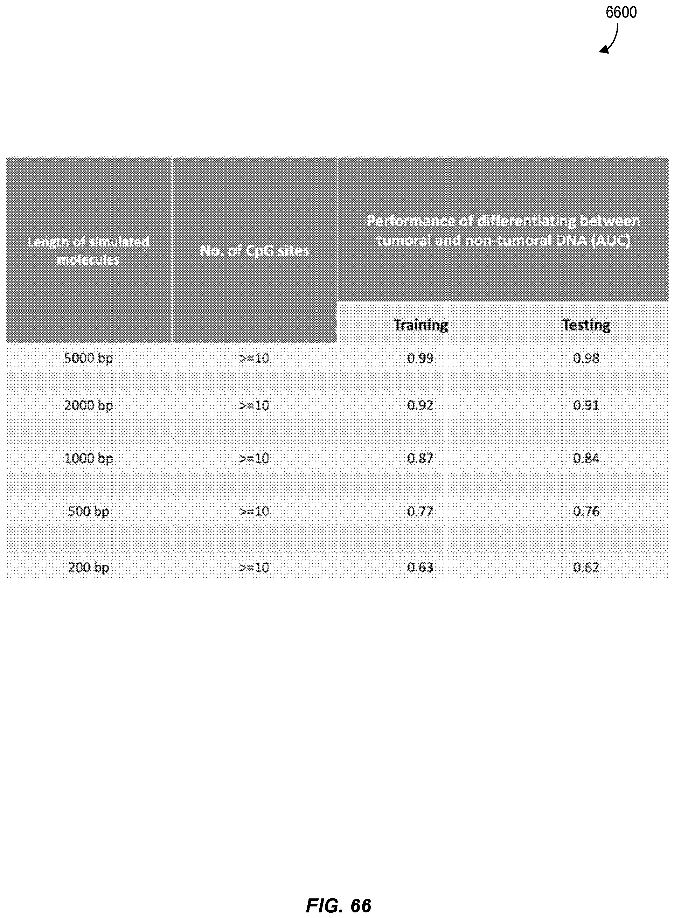

Longer cell-free DNA molecules include more CpG sites. The more CpG sites in a plasma DNA molecule can generally improve the accuracy of the tissue of origin determination for a plasma DNA molecule. FIG. 66 shows a table 6600 that identifies performance of a machine-learning model differentiating between tumoral and non-tumoral DNA in plasma of cancer patients, with different lengths of plasma DNA molecules. FIG. 66 shows that the analysis of methylation haplotypes for 200 bp DNA molecules gave an AUC of only 0.62, whereas the analysis of methylation haplotypes for 1-kb plasma DNA molecules improved the AUC to 0.84. The analysis of methylation haplotypes for 5-kb plasma DNA molecules further improved the AUC to 0.98. These results suggested that the analysis of long cell-free DNA in patients with cancer would provide a more accurate approach for determining the tissue/tumor of origin of a plasma DNA molecule, leading to a much higher performance in the detection and monitoring of cancers or other diseases such as, but not limited to, autoimmune diseases, organ transplantation, trauma, etc.

### C. Methods for using machine-learning models to determine a disease classification based on methylation patterns of long cell-free DNA molecules

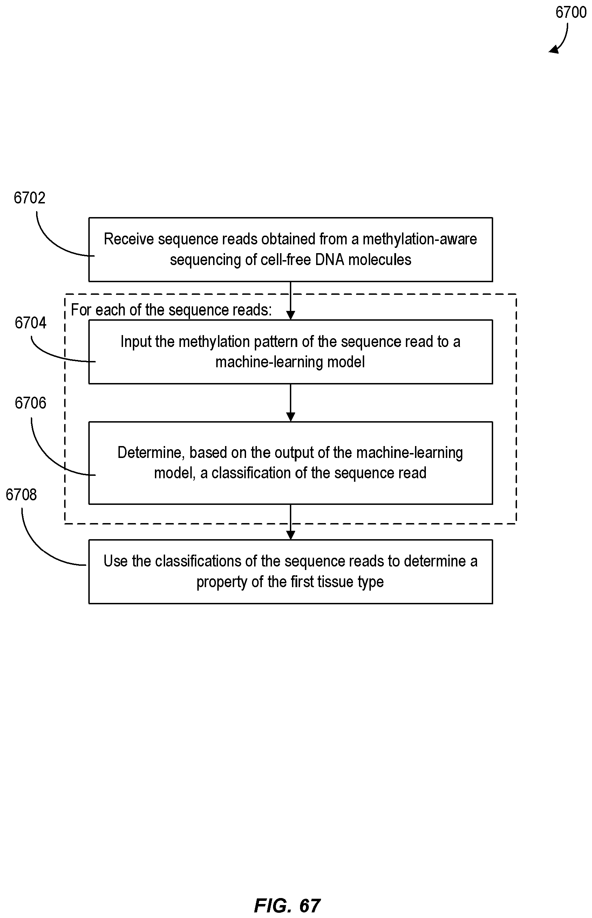

FIG. 67 shows a flowchart 6700 illustrating an example process for analyzing a biological sample of a subject using machine-learning models to determine a tissue-type property based on methylation patterns of long cell-free DNA molecules, according to some embodiments. The biological sample can include DNA originating from normal cells and potentially from cells associated from one or more of a plurality of tissue types. In addition, at least some of the DNA is cell-free in the biological sample.

At step 6702, sequence reads obtained from a methylation-aware sequencing of cell-free DNA molecules can be received. The methylation-aware sequencing can include enzymatic treatment. In some instances, the methylation-aware sequencing does not include bisulfite treatment for generating sequence reads for disease classification. In other instances, bisulfite treatment is used. For generating the training data to train the machine-learning model, bisulfite treatment can be used for methylation-aware sequencing. Each of the sequence reads can include a methylation pattern of methylation statuses at a set of sites (e.g., CpG sites) on the sequence read. For example, a given sequence read can include at least 3 CpG sites. The methylation pattern can include a number of bases between pairs of sites of the set of sites, as well as the identity of the bases.

In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least 300 bases, at least 400 bases, at least 500 bases, at least 600 bases, or at least 800 bases.

The set of sites can be various numbers. In some instances, the set of sites for each of the sequence reads can include at least an N number of sites. For example, a given sequence read can include at least 3 CpG sites. Other numbers can be contemplated, including but are not limited to 2, 3, 4, 5, 6, 7, 8, 9, 10, 15, 20, 25, 30, 35, 40, 45, 50, or greater than 50 sites. Additionally or alternatively, the sequence reads can correspond to long cell-free DNA molecules having sizes within a first size range (e.g., greater than 500 bps) and include at least an N number of sites (e.g., 3 CpG sites). The steps for obtaining the sequence reads and determining methylation statuses of the sequence reads are additionally described in step 5702 of FIG. 57.

Steps 6704 and 6706 can be performed for each sequence read of the sequence reads received from step 6702. At step 6704, the methylation pattern of the sequence read can be inputted to a machine-learning model. In some instances, inputting the methylation pattern to the machine-learning model includes inputting a sequence of the sequence read into the machine-learning model. Inputting the methylation pattern of the sequence read to the machine-learning model can include forming a matrix of the sequence read, e.g., in which the matrix includes one-hot encoding of the bases and methylation status of the set of sites of the sequence read. In some instances, a location of the sequence read can be determined (e.g., by aligning the sequence read to a corresponding location of a reference sequence), at which the location is also inputted to the machine-learning model.

The machine-learning model can be trained using a first training set of sequence reads labeled as being from the first tissue type and a second training set of sequence reads labeled as being from one or more other tissue types. In some instances, the machine-learning model includes a convolutional neural network (CNN) and a recurrent neural network (RNN), e.g., as described for FIG. 63. In some instances, the first or second training set of sequence reads are obtained from one or more differentially methylated regions (DMR). The one or more other tissue types can include 1, 2, 3, 4, 5, 10, 15, 20, or more than 20 tissue types. The one or more other tissue types can include, but are not limited to, T-cells, B-cells, neutrophils, lung tissue, or liver. The one or more other tissue types can include buffy coat.

At step 6706, based on the output of the machine-learning model, a classification of the sequence read can be determined. The classification can indicate that the sequence read is derived (or a level of derivation) from the first tissue type. The tissue classification may include a probability that the sequence read is derived from the first tissue type. The first tissue type can be a diseased tissue type. In some instances, the first tissue type is associated with a disease. The probability of more than one tissue type can be determined.

At step 6708, the classifications of the sequence reads can be used to determine a property of the first tissue type. The property of the first tissue type can identify an amount of sequence reads classified as being derived from the first tissue type. In some instances, the property of the first tissue type can identify a disease state of a disease associated with the first tissue type. The disease can be cancer. The property of the first tissue type can further identify a predicted prognosis of the disease associated with the first tissue type. For example, the predicted prognosis can be a presence of vascular invasion associated with cancer.

In some instances, determining the property includes: (i) determining a first amount of sequence reads classified as being derived from the first tissue type; and (ii) determining a classification of a disease in the biological sample for the first tissue type based on the first amount. The steps for determining the classification of the disease using the first amount is additionally described in step 5708 of FIG. 57.

### VII. COMBINED ANALYSIS OF VARIANTS AND METHYLATION PATTERNS

To enhance accuracy of disease classification using long cell-free DNA molecules, the methylation-pattern analysis of the long cell-free DNA molecules can be combined with SNV-based analysis. For example, in a plasma sample, we can identify the mutation (e.g., an SNV) of a sequence read based on comparing the sequence read with a reference sequence, such as a reference sequence determined from the white blood cell represented in the constitutional genome. Then, we can analyze the methylation pattern for those sequence reads that are linked to such gene mutation.

A method of using the combined analysis of SNVs and methylation patterns of long cell-free DNA molecules includes obtaining sequence reads from methylation-aware sequencing of cell-free DNA molecules of a biological sample. Each sequence read includes a methylation pattern, in which the methylation pattern identifies methylation statuses at a set of CpG sites on the sequence read. A sequence read of the sequence reads can be aligned to a corresponding portion of a reference genome. Then, the sequence read is compared with sequence at the corresponding portion to determine a presence of one or more variants (e.g., a single-nucleotide variant, single-nucleotide polymorphisms, amplification). The variant can be a microsatellite expansion, insertion, deletion, structural variation, sequence duplication, amplification, rearrangement, translocation, inversion, and/or microdeletion. In some instances, an SNV for sequence read is identified if the SNV is detected above a threshold number of other reads (e.g., 5 times). If the one or more variants are identified, the methylation pattern of the sequence read can be further analyzed to determine disease classification.

### A. SNV and methylation patterns

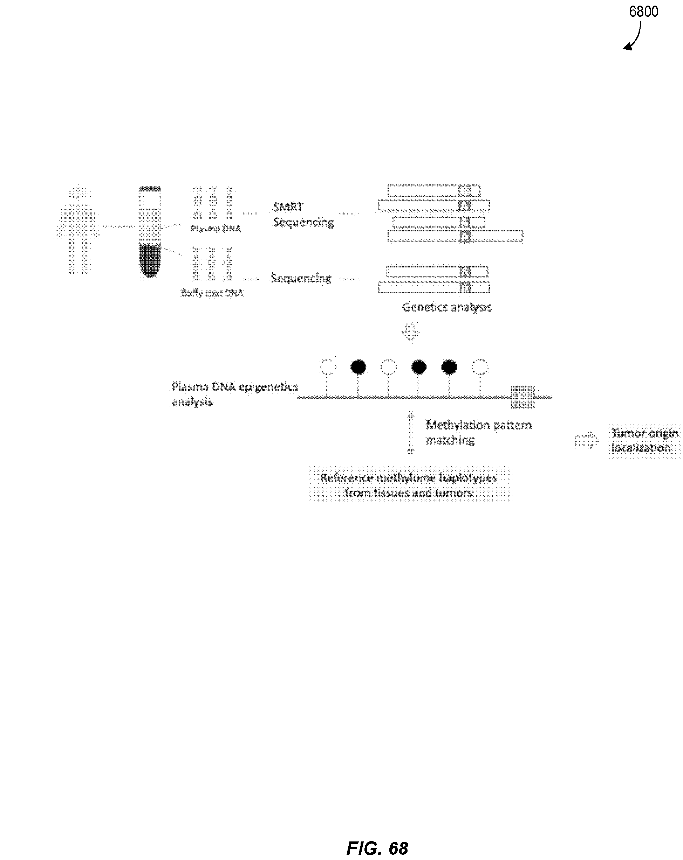

In some embodiments, buffy coat DNA and plasma DNA for a patient are sequenced. The buffy coat DNA could be sequenced using, but not limited to, Illumina sequencing. The plasma DNA could be sequenced using, but not limited to, SMRT-seq, such that sequence reads corresponding to long cell-free DNA molecules can be obtained. FIG. 68 shows a schematic diagram 6800 that illustrates an example of combined analysis using SNV and CpG methylation haplotype information, according to some embodiments. As shown in FIG. 68, the plasma DNA carrying an allele (e.g. G nucleotide) that was absent in the sequencing results of buffy coat DNA was called a somatic mutation. The analysis of the methylation haplotypes of plasma DNA molecules carrying such a somatic mutation would allow for determining the anatomical location of potential cancer.

In some embodiments, the methylation haplotype associated with cancer signals can be linked to a called somatic mutation for disease classification. The combined analysis can be used for reducing the false positives of only using somatic mutations for disease classification. For example, a somatic mutation supported by sequenced plasma DNA molecules determined to be of tumor of origin (e.g., based on methylation patterns) would be more likely to be a true mutation, compared with a somatic mutation supported by sequenced plasma DNA molecules not determined to be of tumor origin. Thus, the selection of those somatic mutations which are supported by sequenced plasma DNA molecules determined to be of tumor origin could improve the positive predictive value in detecting the tumor-derived mutations.

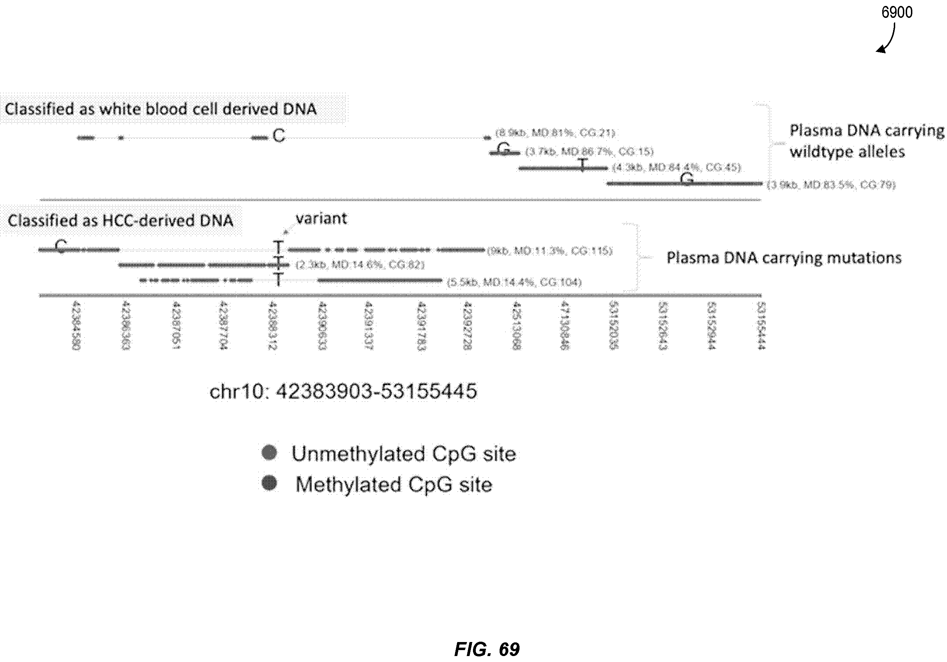

FIG. 69 shows characteristics 6900 of a first group of plasma DNA molecules carrying wildtype alleles and a second group of plasma DNA molecules carry mutations. For the first group, we identified 4 long plasma DNA molecules carrying wildtype alleles, with sizes of 8.9 kb, 3.7 kb, 4.3 kb and 3.9 kb, respectively. These 4 plasma DNA molecules were determined to be of white blood cell origin, based on their respective abundance of methylation statuses (red bars) across CpG sites. For the second group, we identified 3 long plasma DNA molecules carrying mutations not present in white blood cells, with sizes of 9 kb, 2.3 kb, and 5.5 kb, respectively. These 3 plasma DNA molecules were determined to be of HCC tumor origin, based on their respective abundance of unmethylation statuses (green bars) across the CpG sites. Thus, a patient having the second group of plasma DNA molecules can be diagnosed with HCC based on the synergistic analysis of combining SNVs and CpG methylation haplotypes, which was consistent with the clinical diagnosis.

In some embodiments, as the longer DNA molecules would contain more CpG sites for facilitating the tissue of origin analysis, the longer DNA molecules could enable a more accurate classification between tumoral DNA and non-tumoral DNA molecules. Thus, the longer the DNA molecule carrying a SNV, the more accurate tumor localization analysis would be achieved. For example, we analyzed the number of CpG sites in a region surrounding a somatic mutation identified from a tumor tissue, with a certain size such as, 200 bp and 1 kb for illustration purposes. In total, we analyzed 38,465 somatic mutations.

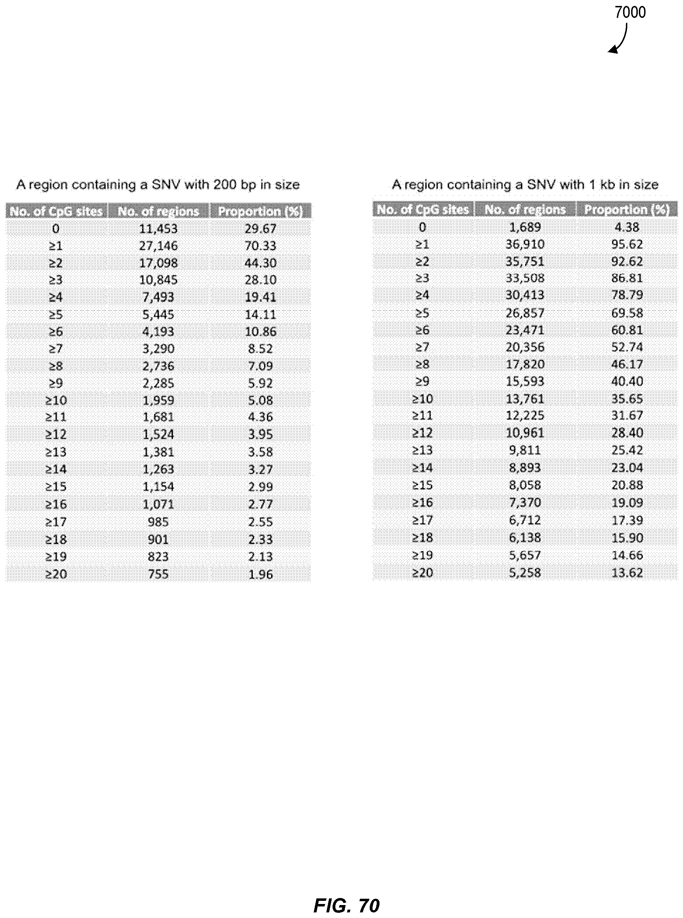

FIG. 70 shows a table 7000 identifying distributions of the number of CpG sites in a 200 bp or 1kb region surrounding a somatic mutation. A reference genome is divided into respective equal-size regions (e.g., 200 bp, 1 kb). A number of these regions having a corresponding number of CpG sites (e.g., 0, ≥1, ≥10) and at least one SNV were determined. As shown in FIG. 70, there was 29.7% of regions with 200 bp in size having no CpG site, whereas there was only 4.4% of regions with 1 kb in size having no CpG site. Further, there was 5.1% of regions with 200 bp in size having at least 10 CpG sites, whereas such a percentage was increased to 35.7% for regions with 1 kb in size. These results suggested that the use of long plasma DNA carrying a mutation would be beneficial for the determination of tissue of origin of plasma DNA, based on its methylation haplotype with more abundant CpG sites. A similar conclusion can be reached for other lengths. 90% of regions with 3kb in size had at least ten CpG sites, but for the SNV, actually there are only two regions across the whole genome.

### B. Allelic imbalance between haplotypes and methylation patterns for disease classification

Cancer cells frequently exhibit copy number aberrations (Chan et al. Proc Natl Acad Sci USA. 2013;110:18761-8; Chan et al. Clin Chem. 2013; 59:1,211-224; Zeira and Raphael. Bioinformatics. 2020;36: i344-i352). Such copy number aberrations are generally not present in non-tumor cells. Copy number aberrations include copy number gains and copy number losses.

For a patient with cancer, the plasma DNA is a mixture comprising tumor-derived and non-tumor-derived DNA molecules. Variants can cause a difference in copy numbers between tumor and non-tumor cells. Such difference can result in the apparent different concentrations of tumor-derived DNA across a human genome. For example, the copy number gain regions would lead to a relatively higher tumoral DNA concentration, whereas the copy number loss regions would lead to a relatively lower tumoral DNA concentration. The copy number gains and losses often occur in a monoallelic manner in cancer cells and cause allelic imbalance (e.g. loss of heterozygosity (LOH)) (Vattathil et al. Genome Res. 2013:23:152-158).

In other words, variants such as the copy number gains and losses generally involve one haplotype block. In some situations, both haplotypes are subjected to copy number gains but the number of amplified copies between two haplotype blocks might be different. Thus, the observed amount of plasma DNA molecules between two constitutional haplotype blocks that are affected by copy number gains or losses would be different. As genome-wide hypomethylation is frequently observed in cancers (Chan et al. Proc Natl Acad Sci USA. 2013;110:18761-8; Ehrlich. Oncogene. 2002;21: 5400-13), the haplotype with increased contributions from the tumor DNA would be expected to be of a lower methylation level than the other haplotype with decreased contributions from the tumor DNA. Thus, the relative haplotype methylation imbalance would be a new metric for informing the presence of cancer. In some embodiments, the imbalanced haplotype methylation levels between haplotypes in cancerous cells would contribute to such a relative haplotype methylation imbalance in the plasma of cancer patients, when their cancer-derived DNA molecules are shed into the blood circulation.

Other types of variants can be considered for this analysis, including but are not limited to microsatellite expansion, insertion, deletion, structural variation, sequence duplication, amplification, rearrangement, translocation, inversion, and/or microdeletion.

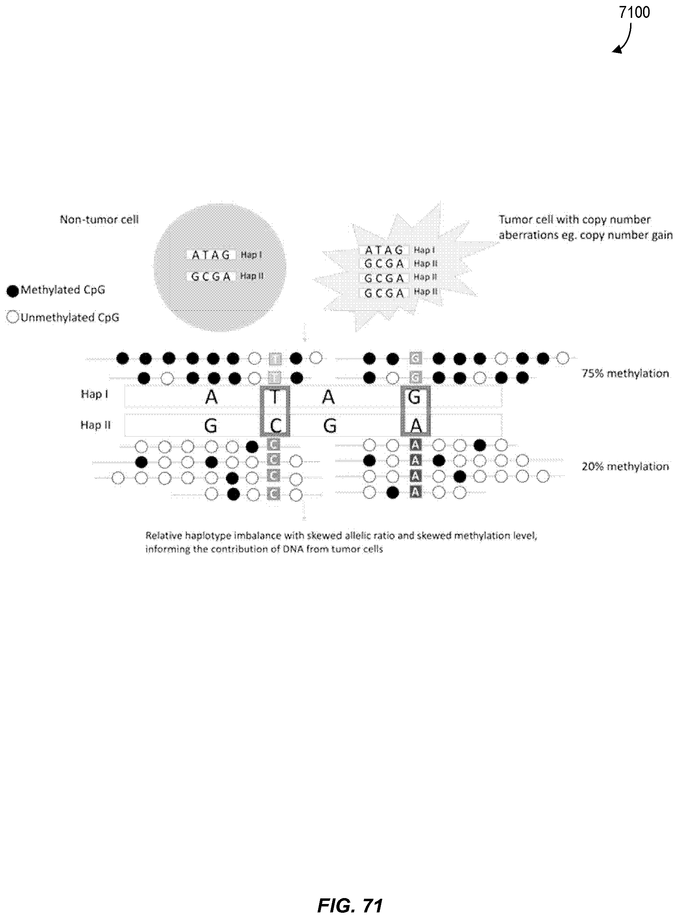

FIG. 71 shows a schematic diagram 7100 of DNA molecules having relative haplotype imbalance with skewed allelic ratio and skewed methylation level informs the presence or absence of cancer. As shown in FIG. 71, one could make use of the resultant skewed allelic ratio and methylation level between two haplotypes to determine the presence of cancer in a patient. In FIG. 71, a non-tumor cell contains haplotype I and II (denoted by Hap I and Hap II respectively). A tumor cell with copy number aberrations, for example copy number gains, contains one haplotype I and three haplotype II. Plasma DNA molecules are sequenced and assigned to haplotype I and haplotype II, respectively.

For simplicity, two allelic sites are chosen for illustrative purpose. A higher number of molecules are assigned to Hap II compared to Hap I, resulting in a higher allelic ratio of C and A alleles on Hap II compared to T and G alleles on Hap I. The CpG sites upstream and downstream of the alleles are analyzed. The CpG sites associated with the C and A alleles are hypomethylated with a methylation level of 20% in this case. Such methylation levels differ from those of the CpG sites associated with the T and G alleles, which has a methylation level of 75% in this case. The increased allelic ratio, coupled with a decreased methylation level in the CpG sites associated with the alleles in Hap II, reflect copy number gains and hypomethylation, thereby informing the contribution of the plasma DNA from tumor cells.

In some embodiments, the number of plasma DNA molecules assigned to alleles in the same haplotype block could be aggregated together to improve the classification power, as the increase of number of plasma DNA molecules would reduce the sampling variation. In some instances, the methylation pattern of each of the plasma DNA molecules in the same haplotype block is used to determine disease classification. The statistical approaches used for determining whether the haplotype methylation imbalance is present in plasma could include but not limited to sequential probability ratio test, binomial proportional test, Pearson's chi-squared test, a two proportion z-test, etc. The number of CpG sites analyzed could include, but not limited to, ≥ 3, ≥ 4, ≥ 5, ≥ 6, ≥ 7, ≥ 8, ≥ 9, ≥ 10, ≥ 15, ≥ 20, ≥ 25, ≥ 30, ≥ 35, ≥ 40, ≥ 45, ≥ 50, ≥ 60, ≥ 70, ≥ 80, ≥ 90, ≥ 100, ≥ 200, ≥ 300, ≥ 400, ≥ 500, ≥ 1000, or other combinations.

### C. Methods for combined analysis of variants and methylation patterns for determining tissue of origin

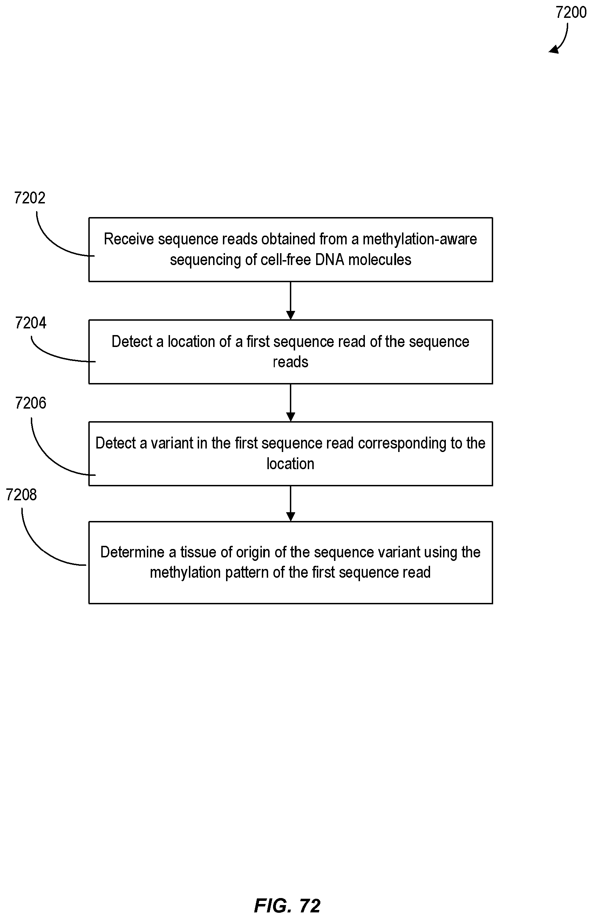

FIG. 72 shows a flowchart 7200 illustrating an example process for analyzing a biological sample of using variants and methylation patterns to determine a tissue of origin based on methylation patterns of long cell-free DNA molecules, according to some embodiments. The biological sample can include DNA originating from normal cells and potentially from cells associated from one or more of a plurality of tissue types. In addition, at least some of the DNA is cell-free in the biological sample.

At step 7202, sequence reads obtained from a methylation-aware sequencing of cell-free DNA molecules can be received. In some instances, the methylation-aware sequencing does not include bisulfite treatment for generating sequence reads for disease classification. In other instances, bisulfite treatment is used. Each of the sequence reads can include a methylation pattern of methylation statuses at a set of sites (e.g., CpG sites) on the sequence read. For example, a given sequence read can include at least 15 CpG sites. The methylation pattern can include a number of bases between pairs of sites of the set of sites, as well as the identity of the bases.

In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least at least 500 bp, 600 bp, 1 kbp, 2 kbp, 3 kbp, 4 kbp, 5 kbp, 6 kbp, 7 kbp, 8 kbp, 9 kbp, 10 kbp.

The set of sites can be various numbers. In some instances, the set of sites for each of the sequence reads can include at least an N number of sites. For example, a given sequence read can include at least 3 CpG sites. Other numbers can be contemplated, including but are not limited to at least 3, 5, 10, 20, 30, 40, 50, 60, 70, 80, 90, 100 sites. Additionally or alternatively, the sequence reads can correspond to long cell-free DNA molecules having sizes within a first size range (e.g., greater than 1 kbp) and include at least an N number of sites (e.g., 10 CpG sites). The steps for obtaining the sequence reads and determining methylation statuses of the sequence reads are additionally described in step 5702 of FIG. 57.

At step 7204, a location of a first sequence read of the sequence reads can be determined. The location of the first sequence read can be determined by aligning the first sequence read to a reference genome. In some instances, the location of the first sequence read is determined by aligning the first sequence read to a constitutional genome of the subject.

At step 7206, a variant in the first sequence read corresponding to the location can be detected. The variant in the first sequence read can be a variant relative to a reference sequence at the location. As examples, the variant can be a polymorphism, microsatellite expansion, insertion, deletion, structural variation, sequence duplication, amplification, rearrangement, translocation, inversion, and/or microdeletion. The example of the variant being a single nucleotide polymorphism is shown in FIG. 68.

At step 7208, a tissue of origin of the variant can be determined using the methylation pattern of the first sequence read. The identification of the tissue of origin (tissue classification) can used any of the techniques described herein, including the techniques described in Section IV, V, and VI of the present disclosure. For example, the tissue of origin associated with the variant can be determined by comparing the methylation pattern of the first sequence read and one or more reference methylation patterns, as described in steps 5706 and 5708 of FIG. 57. Such description of the other methods equally applies to this method. For example, determining the tissue of origin includes comparing the methylation pattern to a first reference methylation patterns at the location. The first reference methylation pattern can correspond to a diseased tissue type of a disease. In some instances, the first reference methylation pattern corresponds to a particular tissue type (e.g., liver). Based on the comparison, the sequence read can be classified as being derived from one of the plurality of tissue types.

The values for the reference pattern can be binary (e.g., 0 and 1 as in FIGS. 41 or 42) or have fractions (e.g., 0.2 signifying 20% methylation index). The reference pattern that is the closest can be identified among a set of reference patterns, and the tissue classification can be determined to be the corresponding tissue type of the closest reference pattern. The closest reference pattern can be determined by taking a difference of the methylation status or index at each site relative to a reference pattern. The tissue classification can indicate that the sequence read is derived (or a level of derivation) from one of the plurality of tissue types. The tissue classification may include a probability that the sequence read is derived from one of the plurality of tissue types. The probability for more than one tissue type can be determined

In some instances, determining the tissue of origin can include inputting the location and the methylation pattern to a machine learning model. The machine-learning model can be trained using a first training set of sequence reads labeled as being from the first tissue type and a second training set of sequence reads labeled as being from one or more other tissue types. In some instances, the machine-learning model includes a convolutional neural network (CNN) and a recurrent neural network (RNN). In some instances, the first or second training set of sequence reads are obtained from one or more differentially methylated regions (DMR). Based on an output of the machine learning model, determining whether the sequence read is derived from the first tissue type.

### D. Methods for combined analysis of variants and methylation patterns for determining cancer classification

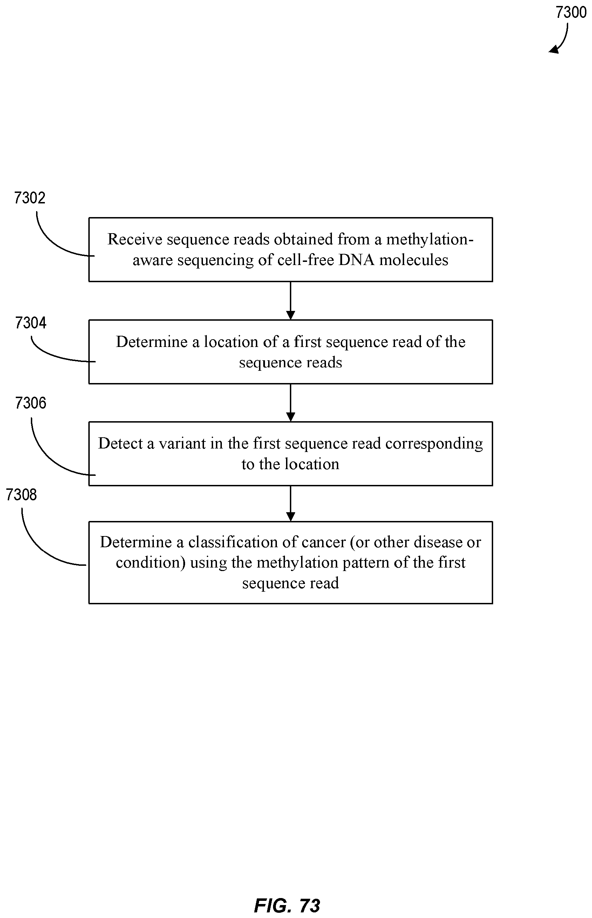

FIG. 73 shows a flowchart 7300 illustrating an example process for analyzing a biological sample of using variants and methylation patterns to determine a cancer classification based on methylation patterns of long cell-free DNA molecules, according to some embodiments. The biological sample can include DNA originating from normal cells and potentially from cells associated from cancer. In addition, at least some of the DNA is cell-free in the biological sample.

At step 7302, sequence reads obtained from a methylation-aware sequencing of cell-free DNA molecules can be received. The methylation-aware sequencing can include enzymatic treatment. In some instances, the methylation-aware sequencing does not include bisulfite treatment. In other instances, bisulfite treatment is used. Each of the sequence reads can include a methylation pattern of methylation statuses at a set of sites (e.g., CpG sites) on the sequence read. For example, a given sequence read can include at least 15 CpG sites. The methylation pattern can include a number of bases between pairs of sites of the set of sites, as well as the identity of the bases.

A single molecule methylation level in short cell-free DNA molecules may not be statistically sufficient for determining the cancer classification. To address this issue, long cell-free DNA molecules can be used. In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least 500 bp, 600 bp, 1 kbp, 2 kbp, 3 kbp, 4 kbp, 5 kbp, 6 kbp, 7 kbp, 8 kbp, 9 kbp, 10 kbp. By sequencing the long DNA molecules, the number of the set of CpG sites of a given long DNA molecule can be high (e.g., at least 5, 10, 20, 50, 100, 200, 500, or a 1,000 CpG sites). In this manner, the total proportion of sites that are methylated can be an accurate statistical determination, as opposed to fragments that just have one or two sites.

The set of sites can be various numbers. In some instances, the set of sites for each of the sequence reads can include at least an N number of sites. For example, a given sequence read can include at least 3 CpG sites. Other numbers can be contemplated, including but are not limited to at least 3, 5, 10, 20, 30, 40, 50, 60, 70, 80, 90, 100 sites. Additionally or alternatively, the sequence reads can correspond to long cell-free DNA molecules having sizes within a first size range (e.g., greater than 1000 kbps) and include at least an N number of sites (e.g., 10 CpG sites). The steps for obtaining the sequence reads and determining methylation statuses of the sequence reads are additionally described in step 5702 of FIG. 57.

At step 7304, a location of a first sequence read of the sequence reads can be determined. The location of the first sequence read can be determined by aligning the first sequence read to a reference genome. In some instances, the location of the first sequence read is determined by aligning the first sequence read to a constitutional genome of the subject. Additionally or alternatively, respective locations of the other sequence reads can also be determined, such that the cancer classification can be determined based on sequence reads from the same location of the first sequence read.

At step 7306, a variant in the first sequence read corresponding to the location can be detected. The variant in the first sequence read can be a variant relative to a reference sequence at the location. The variant can be a microsatellite expansion, insertion, deletion, structural variation, sequence duplication, amplification, rearrangement, translocation, inversion, and/or microdeletion. In some instances, the variant can be a known tumor marker, such as a microsatellite instability (e.g., a copy number aberration) or a particular sequence variant (e.g., a single nucleotide variant) that is a marker of cancer.

At step 7308, a classification of cancer (or other disease or condition) can be determined using the methylation pattern and the variant of the first sequence read. For example, the classification of cancer can be determined based on a methylation level of the methylation pattern, in which the methylation level is determined from methylation statuses of the set of sites of the first sequence read. The methylation level can be a methylation index, a methylation density, count of molecules methylated at one or more sites of the set of sites, or proportion of molecules methylated (e.g., cytosines) at one or more sites of the set of sites. In some instances, the methylation level identifies a percent methylation of CpG sites of the first sequence read, which is determined based on a count of methylated CpG sites and a total count of CpG sites of the first sequence read. For example, if a DNA molecule contained 10 CpG sites and 5 of them were determined to be methylated, the single-molecule methylation level would be 50 % (i.e. 5/10*100%).The variant of the first sequence read can be used as a first indicia to indicate whether the corresponding DNA molecule is from a tumor, and the methylation pattern of the sequence read can be used as a second indicia to indicate the DNA molecule is from a tumor. If the sequence read has the tumor marker and has a single molecule methylation level below a threshold (e.g., below 20%, 30%, 40%, 50%, or 60%), then a classification of cancer can be that cancer exists. For instance, a decrease can be a result of global hypomethylation due to cancer.

Additionally or alternatively, the single molecule methylation can be determined whether it is greater than the threshold, e.g., when a particular location in the genome (such as a CpG island) is known to be hypermethylated (e.g., greater than 40%, 50%, 60%, 70%, 80%, 90%, or 95%). In this example, if the sequence read has the tumor marker and has a single molecule methylation level above a threshold (e.g., above 40%, 50%, 60%, 70%, 80%, or 90%), then a classification of cancer can be that cancer exists.

In some instances, determining the classification of cancer includes comparing the methylation pattern to a first reference methylation pattern at the location. Thus, instead of a single molecule methylation level, the pattern of sites that are methylated or unmethylated can be used. For example, a cfDNA molecule originating from the liver with six CpG sites can have the methylation pattern as '-M-M-M-U-U-U-' where 'M' represents a methylated state and 'U'' represents an unmethylated state. However, other molecules containing the corresponding CpG sites from other tissues can have the methylations patterns as '-M-U-M-U-M-U-', '-M-U-M-M-U-U-', '-M-U-U-U-M-M-', '-M-M-U-U-M-U-', '-M-M-U-U-U-M-', '-U-M-M-M-U-U-', '-U-U-M-U-M-M-', '-U-U-M-M-M-U-', '-U-U-U-M-M-M-'. For this example, one is not able to use single molecule methylation level to differentiate the liver-derived molecule from those molecules derived from other tissues, as all molecules show identical single molecule methylation levels with a value of 0.5. In contrast, if one uses the methylation pattern across these six CpG sites, the liver-derived molecule becomes unique compared with those molecules derived from other tissues. In this situation, the combination of methylation pattern across a set of CpG sites in a molecule could serve as a 'molecular barcode' indicating the cell identity or a disease status, e.g., a disease status in a particular tissue type corresponding to the methylation pattern. The first reference methylation pattern can correspond to a particular tissue type associated with the cancer. Based on the comparison, the subject can be determined as having cancer.

The values for the reference methylation pattern can be binary (e.g., 0 and 1 as in FIGS. 41 or 42) or have fractions (e.g., 0.2 signifying 20% methylation index). For example, the reference methylation pattern that is the closest can be identified among a set of reference methylation patterns, and the disease classification can be determined to be the disease of the closest reference methylation pattern. The closest reference methylation pattern can be determined by taking a difference of the methylation status or index at each site relative to a reference methylation pattern. Additional details for identifying the closest reference methylation pattern are described in at least process 5700 of FIG. 57 and Section IV of the present disclosure.

In some instances, determining the cancer classification includes inputting the location and the methylation pattern to a machine learning model. The machine-learning model can be trained using a first training set of sequence reads labeled as being from the cancer cells and a second training set of sequence reads labeled as being from normal cells. In some instances, the machine-learning model includes a convolutional neural network (CNN) and a recurrent neural network (RNN). In some instances, the first or second training set of sequence reads are obtained from one or more differentially methylated regions (DMR). Based on an output of the machine learning model, determining whether the sequence read is derived from the cancer cells.

Additionally or alternatively, a plurality of DNA molecules can be used for determining the classification of cancer. Each DNA molecule of the plurality of DNA molecules can include the variant. The variant can again be a known tumor marker, such as a microsatellite instability (copy number aberration) or a particular sequence variant that is a marker of cancer. Based on their respective methylation patterns, the methylation level of all of the sequence reads with the tumor variant can be determined based on methylation statuses of their respective set of sites. Such a methylation level could be for just one site or across multiple sites, which may occur over a plurality of regions (e.g., across CpG islands). In some instances, the methylation level includes a proportion of methylated sites relative to a total number of sites of the sequence reads. The methylation level can be compared to a threshold to determine hypomethylation or hypermethylation for the sequence reads. If the methylation level exceeds the threshold value, cancer can be determined to exist for the subject. Examples of thresholds are provided above. The thresholds can be determined by testing methylation levels of reference samples obtained from subjects with known classifications of cancer (e.g., healthy, cancer exists). In effect, the methylation level determined from cell-free DNA molecules having variants can be used as another indicia to determine whether cancer exists in the subject.

In yet another embodiment, in another example for a plurality of DNA molecules being used, the variant can be a copy number aberration (CNA), such as a deletion or amplification. The copy number aberration can be determined in various ways, e.g., by comparing a count or reads in the region to counts in another region (e.g., to one region, an average read density across a large number of regions, to regions on another chromosome(s), or a total number of reads for entire genome). A ratio of the counts can be compared to cutoff value for classifying whether a CNA exists. For a region that has a CNA, the aggregate methylation level (e.g., a sum, an average, or a median of methylation levels determined for the sequence reads) for one or more sites of sequence reads aligned to the region can be compared to a threshold. The threshold can be determined based on methylation levels of reference samples obtained from subjects with known classifications of cancer (e.g., healthy, cancer exists). For instance, a genomic region having an amplification would have a lower methylation level in general (due to global hypomethylation), since there would be more fragments from that genomic region compared to another genomic region that does not have a CNA. As such, if the CNA corresponds to amplification of sequence reads, a classification that cancer exists for the subject can be determined if the aggregate methylation level is less than the threshold. In other instances, if the particular location is known to be hypermethylated in subjects with cancer, then it can be determined whether the methylation level is greater than a threshold. If the CNA corresponds to deletion of sequence reads for the particular region, a classification that cancer exists for the subject can be determined if the aggregate methylation level is greater than the threshold. Thus, the methylation patterns of the plurality of DNA molecules can be used as an additional indicia of whether cancer exists in the subject.

In yet another embodiment, haplotype techniques can be used for a plurality of DNA molecules. For example, an allelic ratio at one or more SNPs (heterozygous loci) can be determined. When multiple SNPs are used, the allelic ratio can be determined based on a first count of sequence reads at one haplotype and a second count of sequence reads at the other haplotype. A size of DNA fragments for different regions or different haplotypes can also be used, as will be appreciated by one skilled in the art. Then, the methylation level (e.g., an aggregate single methylation level or a level determined across DNA molecules) for the region or haplotype with the aberration can be determined and compared to a threshold. The threshold can be determined based on methylation levels of reference samples obtained from subjects with known classifications of cancer (e.g., healthy, cancer exists). If the allelic ratio at the location indicates an amplification of DNA molecules at a particular haplotype, a classification that cancer exists for the subject can be determined if the aggregate methylation level of the sequence reads corresponding to the particular haplotype is less than the threshold. In other instances, if the allelic ratio at the location indicates a deletion of DNA molecules at the particular haplotype, then a classification that cancer exists for the subject can be determined if the aggregate methylation level of the sequence reads corresponding to the particular haplotype is greater than a threshold. Thus, for a deletion, the methylation level would increase for global methylation or increase for a region that is known to have hypermethylation in the region in the tumor.

### VIII. MACHINE-LEARNING FOR DISEASE CLASSIFICATION BASED ON MULTIPLE CHARACTERISTICS OF LONG CFDNA MOLECULES

Multiple characteristics (e.g., methylation pattern, sequence motifs, sequence context) of long cell-free DNA molecules to determine a classification of a disease for a subject. In particular, a machine-learning model can be trained using long cell-free DNA molecules (e.g., sequences with sizes greater than 600 bp) obtained from training samples with known classifications of the disease.

### A. Training

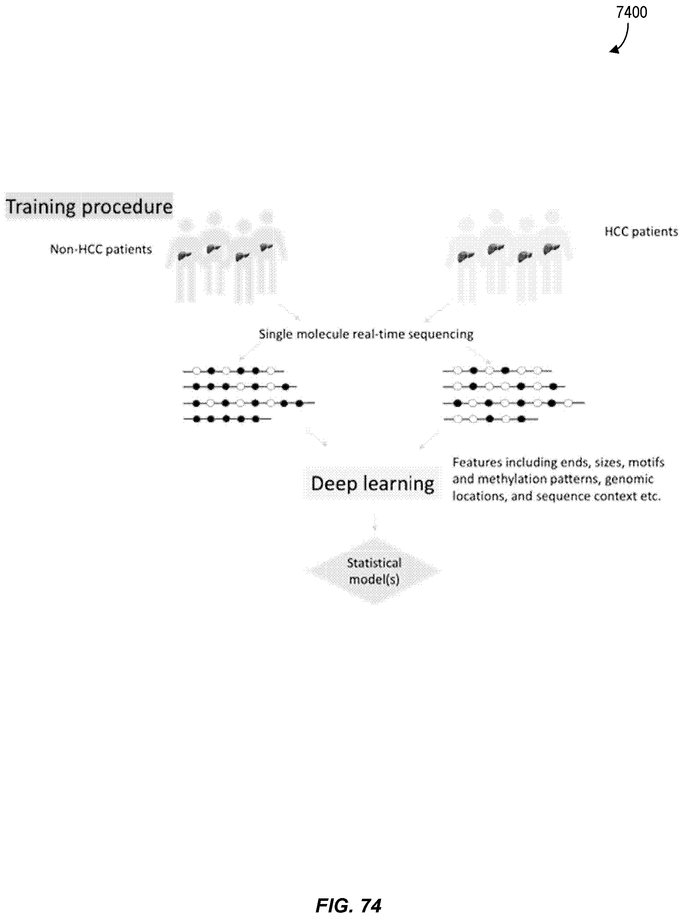

FIG. 74 shows a schematic diagram 7400 illustrating an example process for training a machine-learning model for differentiating patients with and without cancers, based on fragmentomic and epigenetic information present in plasma DNA molecules. As shown in FIG. 74, one could obtain a number of plasma DNA samples from a number of patients with and without cancers. Sequence reads of long cell-free DNA molecules can be obtained for a biological sample via single-molecule sequencing or cluster-based sequencing. The sequence reads of a given long cell-free DNA molecule can be analyzed to identify a corresponding set of features. The features of each plasma DNA molecule, including but not limited to ends, sizes, sequence context, end motifs, methylation haplotypes, jagged ends, genomic coordinates, etc. could be programmed into a data matrix. In some instances, the sequence context identifies a nucleotide sequence (e.g., a 4-mer) of at least part of the plasma DNA molecule. The sequence context can span the entire plasma DNA molecule

The data matrices from patients with and without cancers could be used for training statistical models for classifying a patient with or without cancer. Statistical models could include, but not limited to, linear regression, logistic regression, deep recurrent neural network (e.g. fully-connected recurrent neural network (RNN), Gated Recurrent Unit (GRU), long short-term memory (LSTM)), transformed-based methods (e.g. XLNet, BERT, XLM, RoBERTa), Bayes classifier, hidden Markov model (HMM), linear discriminant analysis (LDA), k-means clustering, density-based spatial clustering of applications with noise (DBSCAN), random forest algorithm, adaptive boosting(AdaBoost), eXtreme Gradient Boosting (XGBoost), and support vector machine (SVM).

### B. Classification

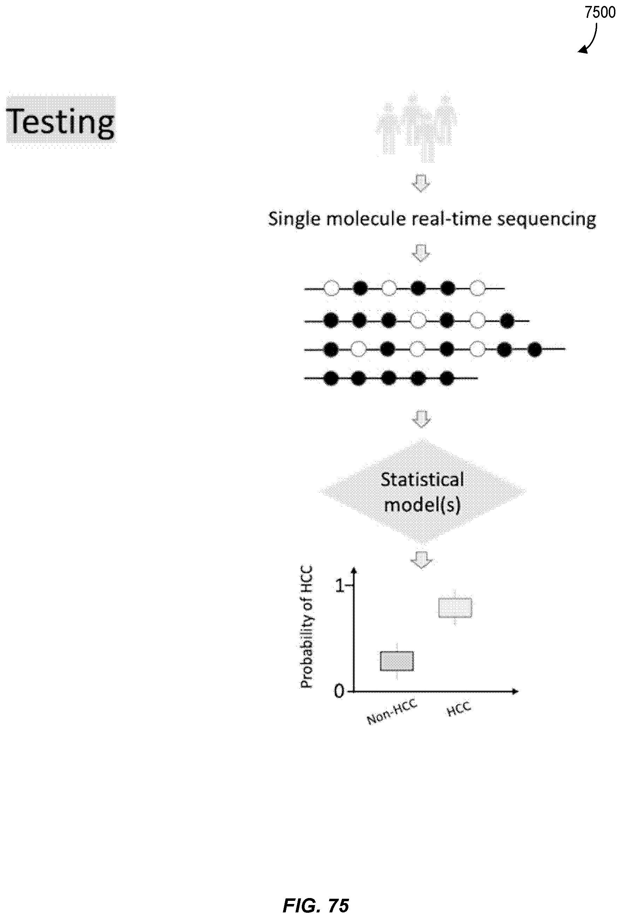

The trained model could be used for determining whether a new sample would have cancer or not. FIG. 75 shows a schematic diagram 7500 illustrating an example process for applying the trained model to cancer detection using fragmentomic and epigenetic information present in plasma DNA molecules. For example, sequence reads can be obtained from a plasma DNA sample, in which at least some of the sequence reads have a length greater than a threshold size (e.g., 600 bp). For each sequence read, one or more features are determined. The one or more features can include, for the sequence read, a location of end in a reference genome, sequence context, size, sequence motif at one or more ends, or a DNA methylation pattern. The features can be inputted into the trained machine-learning model. The machine-learning model can generate an output, which can be used to determine a classification for the sequence read. The classification can identify whether the sequence read is derived from a first tissue type or another tissue type.

The classifications of the sequence reads can be analyzed. For example, an amount of sequence reads corresponding to the first tissue type can be determined. If the amount exceeds a cutoff value, a disease classification corresponding to the first tissue type can be determined for the subject.

### C. Methods for using machine-learning models for disease classification based on multiple characteristics of long cell-free DNA molecules

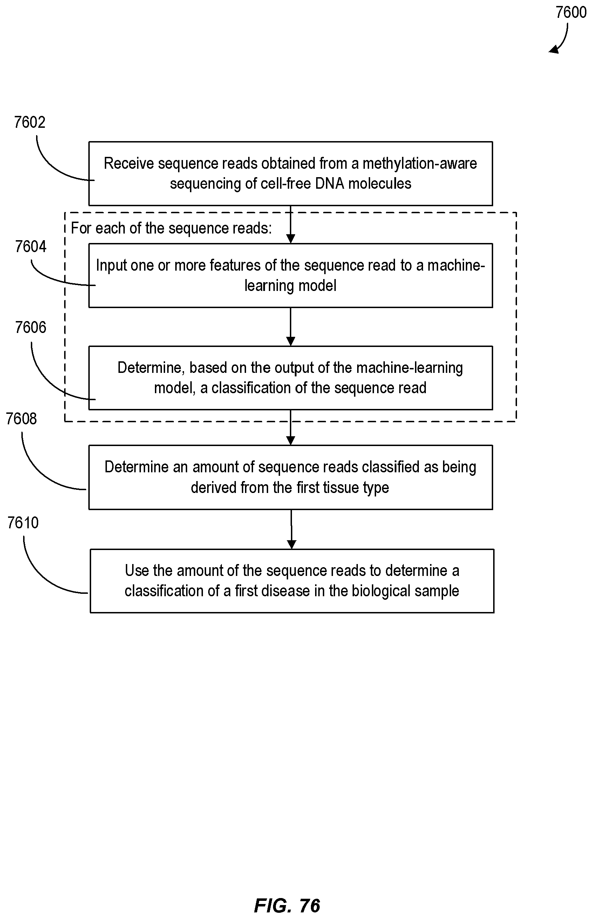

FIG. 76 shows a flowchart 7600 illustrating an example process for analyzing a biological sample of a subject using machine-learning models to determine a disease classification based on multiple characteristics of long cell-free DNA molecules, according to some embodiments. The biological sample can include DNA originating from normal cells and potentially from cells associated from a disease of a first tissue type. In addition, at least some of the DNA is cell-free in the biological sample.

At step 7602, sequence reads obtained from a methylation-aware sequencing of cell-free DNA molecules can be received. The methylation-aware sequencing can include enzymatic treatment. In some instances, the methylation-aware sequencing does not include bisulfite treatment for generating sequence reads for disease classification. By contrast, for generating the training data to train the machine-learning model, bisulfite treatment can be used for methylation-aware sequencing. Each of the sequence reads can include a methylation pattern of methylation statuses at a set of sites (e.g., CpG sites) on the sequence read. The methylation pattern can include a number of bases between pairs of sites of the set of sites, as well as the identity of the bases.

In some instances, the sequence reads correspond to long cell-free DNA molecules having sizes within a first size range, which may include a lower bound and an upper bound. As examples, the first size range can include an upper bound of at least 1,000 bases, at least 3,000 bases, or above. In some instances, the lower bound can be selected from one of at least 300 bases, at least 400 bases, at least 500 bases, at least 600 bases, or at least 800 bases.

The set of sites can be various numbers. In some instances, the set of sites for each of the sequence reads can include at least an N number of sites. For example, a given sequence read can include at least 3 CpG sites. Other numbers can be contemplated, including but are not limited to at least 3, 5, 10, 20, 30, 40, 50, 60, 70, 80, 90, 100 sites. Additionally or alternatively, the sequence reads can correspond to long cell-free DNA molecules having sizes within a first size range (e.g., greater than 500 bps) and include at least an N number of sites (e.g., 3 CpG sites). The steps for obtaining the sequence reads and determining methylation statuses of the sequence reads are additionally described in step 5702 of FIG. 57.

Steps 7604 and 7606 can be performed for each sequence read of the sequence reads received from step 7602. At step 7604, one or more features of the sequence read can be inputted to a machine-learning model. In some instances, the one or more features include at least one selected from: location of end in a reference genome, sequence context, size, sequence motif at one or more ends; and a DNA methylation pattern. For example, a feature can be a sequence context of the sequence read, in which the sequence context includes a nucleotide-base composition and/or a nucleotide-base order of the sequence read (as described in Section I.B of the present disclosure). Another feature can be a location of the end of the sequence read, in which determining the location of the end can include aligning the sequence read to the reference genome. In another example, a feature can be a DNA methylation pattern of the sequence read, in which the DNA methylation pattern includes methylation statuses at a set of sites on the sequence read (as described in Sections IV, V, and VI of the present disclosure).

The machine-learning model was trained using a first training set of sequence reads labeled as being from the first tissue type and a second training set of sequence reads labeled as being from one or more other tissue types. In some instances, the machine-learning model includes a convolutional neural network (CNN) and a recurrent neural network (RNN). In some instances, the first or second training set of sequence reads are obtained from one or more differentially methylated regions (DMR). The machine-learning model can be selected from one of convolutional neural network (CNN), linear regression, logistic regression, deep recurrent neural network (e.g., fully-connected recurrent neural network (RNN), Gated Recurrent Unit (GRU), long short-term memory, (LSTM)), transformer-based methods (e.g. XLNet, BERT, XLM, RoBERTa), Bayes's classifier, hidden Markov model (HMM), linear discriminant analysis (LDA), k-means clustering, density-based spatial clustering of applications with noise (DBSCAN), random forest algorithm, adaptive boosting (AdaBoost), eXtreme Gradient Boosting (XGBoost), support vector machine (SVM), or a composite model comprising one or more of the above machine-learning models.

The one or more other tissue types can include 1, 2, 3, 4, 5, 10, 15, 20, or more than 20 tissue types. The one or more other tissue types can include, but are not limited to, T-cells, B-cells, neutrophils, lung tissue, or liver. The one or more other tissue types can include buffy coat.

At step 7606, based on the output of the machine-learning model, a classification of the sequence read can be determined. The classification indicates that the sequence read is derived from the first tissue type. The tissue classification may include a probability that the sequence read is derived from the first tissue type. The first tissue type can be a diseased tissue type. In some instances, the first tissue type is associated with a disease.

At step 7608, an amount of sequence reads classified as being derived from the first tissue type can be determined. In some instances, a parameter representing the amount of sequence reads is determined. The parameter can include a proportion of the amount of sequence reads relative to an amount of other sequence reads that were not classified as being derived from the first tissue type.

At step 7610, the amount of the sequence reads can be used to determine a classification of a disease in the biological sample. For example, determining the classification of the disease in the biological sample includes comparing the amount to one or more cutoff values, in which the one or more cutoffs are determined using reference samples with known classifications of the disease. The disease can be cancer. In some instances, determining the classification of the disease includes determining whether vascular invasion exists. The steps for determining the classification of the disease using the amount is additionally described in step 5708 of FIG. 57.

The classification of the disease can include a classification of a severity of the disease (e.g., no disease, early stage, intermediate stage, advanced stage). For example, the classification of the disease can include a stage of cancer in accordance with BCLC stages. The classification can then select one of the stages. Accordingly, the classification can be determined from a plurality of stages of disease (e.g., one of BCLC stages for HCC). In some instances, the disease is cancer. As examples, the cancer can be hepatocellular carcinoma, lung cancer, breast cancer, gastric cancer, glioblastoma multiforme, pancreatic cancer, colorectal cancer, nasopharyngeal carcinoma, and head and neck squamous cell carcinoma.

### IX. MICROSATELLITE INSTABILITY

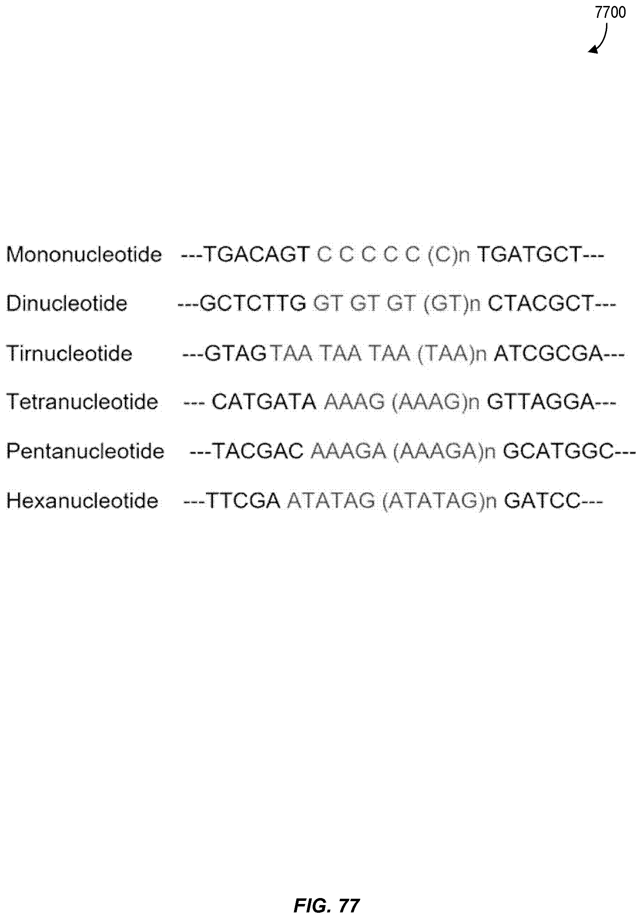

Microsatellite instability is associated with various cancers including colon, gastric, ovarian cancers, etc. Microsatellites are tandem repeats of DNA where a sequence motif of one to six nucleotides is repeated multiple times. FIG. 77 shows an example set of microsatellite sequences 7700 in DNA molecules.

These repetitive sequences can occur in thousands of regions and have a higher mutation rate than other areas of a human genome (Brinkmann et al. Am J Hum Genet. 1998;62:1408-15). Microsatellite instability (MSI) occurs likely because DNA mismatch repair (MMR) is not functioning properly, with the target microsatellite gaining or losing repeat units, resulting in a somatic change in size. The widespread instability associated with deficient MMR indicates a rapid accumulation of somatic mutations that could inactivate genes in key regulatory processes and cause tumorigenesis. Current research has identified MMR deficiency in many forms of cancer, more often in early-stage disease (Le et al. Science. 2017;357:409-413). MSI detection was initially performed in colorectal cancer either by using PCR on specific markers followed by PAGE and autoradiography (Thibodeau et al. Science. 1993;260:816-819). However, these methods were laborious, time-consuming, invasive and with low sizing accuracy.

Subsequently, MSI detection was performed in plasma of small cell lung cancer patients by using PCR on selected specific markers for the most frequent microsatellite alterations, followed by gel electrophoresis and autoradiography (Chen et al. Nat Med. 1996; 2(9):1033-5). However, these PCR-based methods restrict the application of MSI detection to a limited number of markers and cannot be applied to cancer patients harboring the other MSIs that are not targeted by PCR primers. Also, they were laborious, time-consuming, and with low sizing accuracy.

Massively parallel next-generation sequencing (NGS) (i.e. short-read sequencing) has also been proposed to detect MSI in cancer (Cortes-Ciriano et al. Nat Commun. 2017;8:15180). However, the detectable size range of microsatellites would be limited by the read length of the NGS technology, typically in the range of 50 to 150 bp. In addition, the nature of short reads by NGS and high repetitiveness of microsatellites would be prone to inaccurate alignment results, introducing the false positives when analyzing MSI.

The analysis of long plasma DNA molecules in cancer patients would provide more accurate tools to determine the presence or absence of MSI. Using long plasma DNA molecules sequenced by single molecule sequencing, one could obtain the full length of repeat as well as its flanking unique sequence information, so that the genomic locations of such repeat and the sizes of microsatellites of interest could be accurately examined. Without the ability to analyzed long cfDNA in plasma, one cannot utilise some of these markers because the polymorphic region might occasionally be longer than the conventional cfDNA (e.g. 160 bp). In some instances, other tandem repeat polymorphisms (e.g., minisatellites) can also be detected using the long cell-free DNA molecules, which may then be used to detect tumor-derived DNA. Also, in contrast to using PCR-based methods as described in literature which were restricted to a few known MSI markers, the use of long-read sequencing for MSI detection can be applied to cancer patients harboring any MSIs (e.g. on a genomewide level).

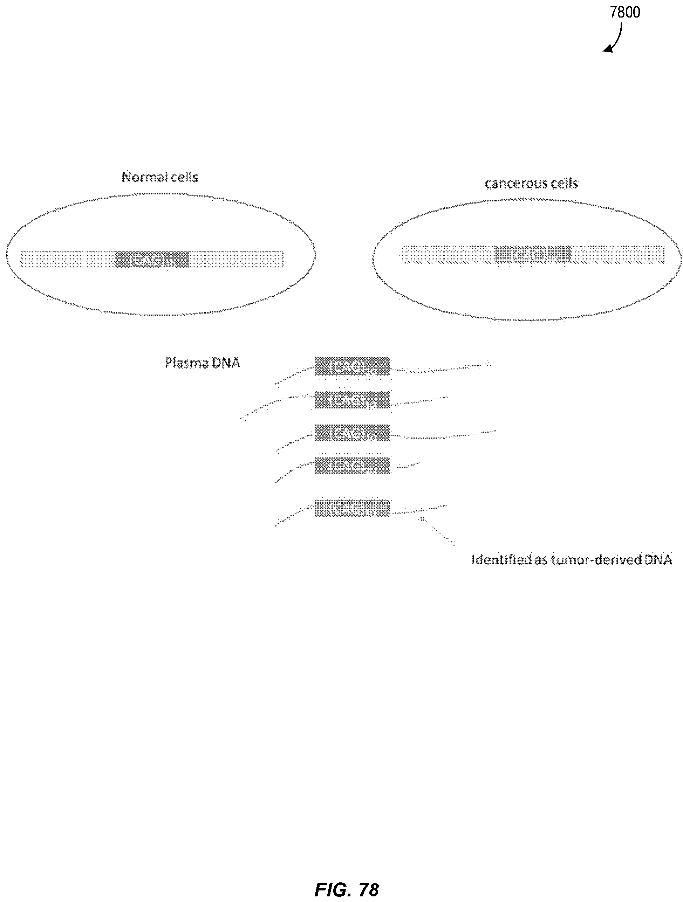

FIG. 78 illustrates an example overview 7800 of detecting tumor-derived DNA based on a cancer-specific microsatellite marker. As shown in Figure 71, in some embodiments, one could detect the tumor-derived molecules that harbored the microsatellite alteration (CAG)₃₀ uniquely present in cancerous cells but absent in normal cells. In some embodiments, the methylation haplotypes associated with microsatellite alterations could be used for determining the tumor location according to the embodiments present in this disclosure.

### X. TREATMENTS

### A. Treatment selection

Embodiments of the present disclosure can accurately predict disease relapse, thereby facilitating early intervention and selection of appropriate treatments to improve disease outcome and overall survival rates of subjects. For example, an intensified chemotherapy can be selected for subjects, in the event their corresponding samples are predictive of disease relapse. In another example, a biological sample of a subject who had completed an initial treatment can be sequenced to identify viral DNA that is predictive of disease relapse. In such example, alternative treatment regimen (e.g., a higher dose) and/or a different treatment can be selected for the subject, as the subject's cancer may have been resistant to the initial treatment.

The embodiments may also include treating the subject in response to determining a classification of relapse of the pathology. For example, if the prediction corresponds to a loco-regional failure, surgery can be selected as a possible treatment. In another example, if the prediction corresponds to a distant metastasis, chemotherapy can be additionally selected as a possible treatment. In some embodiments, the treatment includes surgery, radiation therapy, chemotherapy, immunotherapy, targeted therapy, hormone therapy, stem cell transplant, or precision medicine. Based on the determined classification of relapse, a treatment plan can be developed to decrease the risk of harm to the subject and increase overall survival rate. Embodiments may further include treating the subject according to the treatment plan.

### B. Types of treatments

Embodiments may further include treating the pathology in the patient after determining a classification for the subject. Treatment can be provided according to a determined level of pathology, the fractional concentration of clinically-relevant DNA, or a tissue of origin. For example, an identified mutation can be targeted with a particular drug or chemotherapy. The tissue of origin can be used to guide a surgery or any other form of treatment. And, the level of the pathology can be used to determine how aggressive to be with any type of treatment, which may also be determined based on the level of pathology. A pathology (e.g., cancer) may be treated by chemotherapy, drugs, diet, therapy, and/or surgery. In some embodiments, the more the value of a parameter (e.g., amount or size) exceeds the reference value, the more aggressive the treatment may be.

Treatment may include resection. As an example, for bladder cancer, treatments may include transurethral bladder tumor resection (TURBT). This procedure is used for diagnosis, staging and treatment. During TURBT, a surgeon inserts a cystoscope through the urethra into the bladder. The tumor is then removed using a tool with a small wire loop, a laser, or high-energy electricity. For patients with non-muscle invasive bladder cancer (NMIBC), TURBT may be used for treating or eliminating the cancer. Another treatment may include radical cystectomy and lymph node dissection. Radical cystectomy is the removal of the whole bladder and possibly surrounding tissues and organs. Treatment may also include urinary diversion. Urinary diversion is when a physician creates a new path for urine to pass out of the body when the bladder is removed as part of treatment.

Treatment may include chemotherapy, which is the use of drugs to destroy cancer cells, usually by keeping the cancer cells from growing and dividing. The drugs may involve, for example but are not limited to, mitomycin-C (available as a generic drug), gemcitabine (Gemzar), and thiotepa (Tepadina) for intravesical chemotherapy. The systemic chemotherapy may involve, for example but not limited to, cisplatin gemcitabine, methotrexate (Rheumatrex, Trexall), vinblastine (Velban), doxorubicin, and cisplatin.

In some embodiments, treatment may include immunotherapy. Immunotherapy may include immune checkpoint inhibitors that block a protein called PD-1. Inhibitors may include but are not limited to atezolizumab (Tecentriq), nivolumab (Opdivo), avelumab (Bavencio), durvalumab (Imfinzi), and pembrolizumab (Keytruda).

Treatment embodiments may also include targeted therapy. Targeted therapy is a treatment that targets the cancer's specific genes and/or proteins that contributes to cancer growth and survival. For example, erdafitinib is a drug given orally that is approved to treat people with locally advanced or metastatic urothelial carcinoma with FGFR3 or FGFR2 genetic mutations that has continued to grow or spread of cancer cells.

Some treatments may include radiation therapy. Radiation therapy can include the use of high-energy photons (e.g., x-rays) or other particles to destroy cancer cells. In addition to each individual treatment, combinations of these treatments described herein may be used. In some embodiments, when the value of the parameter exceeds a threshold value, which itself exceeds a reference value, a combination of the treatments may be used.

### XI. EXAMPLE SYSTEMS

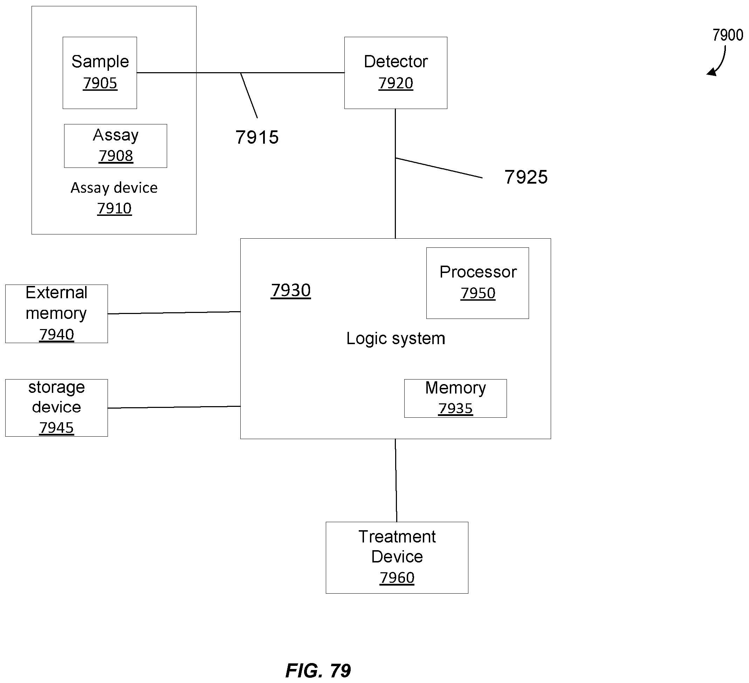

FIG. 79 illustrates a measurement system 7900 according to an embodiment of the present disclosure. The system as shown includes a sample 7905, such as cell-free DNA molecules within an assay device 7910, where an assay 7908 can be performed on sample 7905. For example, sample 7905 can be contacted with reagents of assay 7908 to provide a signal of a physical characteristic 7915. An example of an assay device can be a flow cell that includes probes and/or primers of an assay or a tube through which a droplet moves (with the droplet including the assay). Physical characteristic 7915 (e.g., a fluorescence intensity, a voltage, or a current), from the sample is detected by detector 7920. Detector 7920 can take a measurement at intervals (e.g., periodic intervals) to obtain data points that make up a data signal. In one embodiment, an analog-to-digital converter converts an analog signal from the detector into digital form at a plurality of times. Assay device 7910 and detector 7920 can form an assay system, e.g., a sequencing system that performs sequencing according to embodiments described herein. A data signal 7925 is sent from detector 7920 to logic system 7930. As an example, data signal 7925 can be used to determine sequences and/or locations in a reference genome of DNA molecules. Data signal 7925 can include various measurements made at a same time, e.g., different colors of fluorescent dyes or different electrical signals for different molecule of sample 7905, and thus data signal 7925 can correspond to multiple signals. Data signal 7925 may be stored in a local memory 7935, an external memory 7940, or a storage device 7945.

Logic system 7930 may be, or may include, a computer system, ASIC, microprocessor, graphics processing unit (GPU), etc. It may also include or be coupled with a display (e.g., monitor, LED display, etc.) and a user input device (e.g., mouse, keyboard, buttons, etc.). Logic system 7930 and the other components may be part of a stand-alone or network connected computer system, or they may be directly attached to or incorporated in a device (e.g., a sequencing device) that includes detector 7920 and/or assay device 7910. Logic system 7930 may also include software that executes in a processor 7950. Logic system 7930 may include a computer readable medium storing instructions for controlling measurement system 7900 to perform any of the methods described herein. For example, logic system 7930 can provide commands to a system that includes assay device 7910 such that sequencing or other physical operations are performed. Such physical operations can be performed in a particular order, e.g., with reagents being added and removed in a particular order. Such physical operations may be performed by a robotics system, e.g., including a robotic arm, as may be used to obtain a sample and perform an assay.

System 7900 may also include a treatment device 7960, which can provide a treatment to the subject. Treatment device 7960 can determine a treatment and/or be used to perform a treatment. Examples of such treatment can include surgery, radiation therapy, chemotherapy, immunotherapy, targeted therapy, hormone therapy, and stem cell transplant. Logic system 7930 may be connected to treatment device 7960, e.g., to provide results of a method described herein. The treatment device may receive inputs from other devices, such as an imaging device and user inputs (e.g., to control the treatment, such as controls over a robotic system).

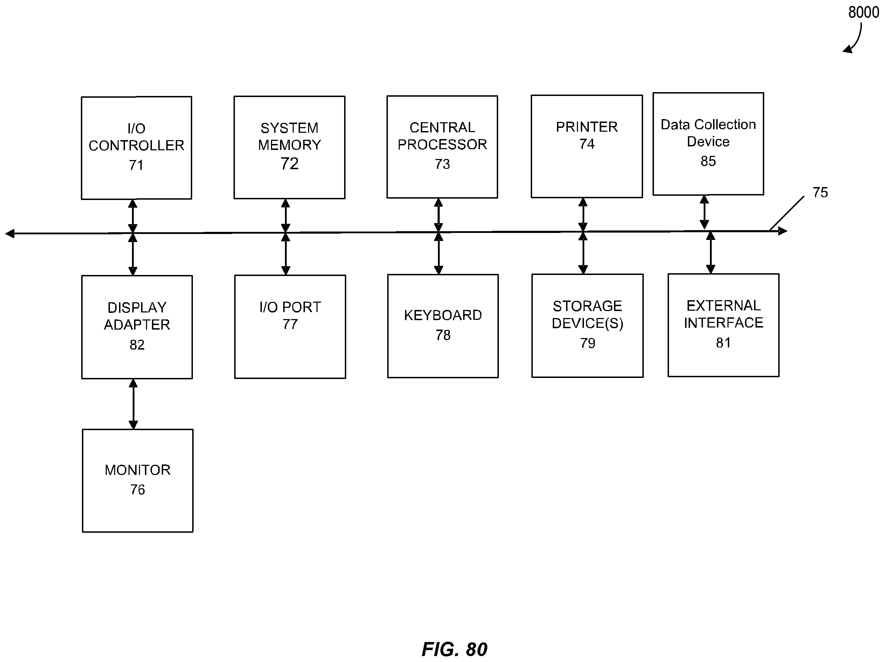

Any of the computer systems mentioned herein may utilize any suitable number of subsystems. Examples of such subsystems are shown in FIG. 80 in computer system 8000. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

The subsystems shown in FIG. 80 are interconnected via a system bus 75. Additional subsystems such as a printer 74, keyboard 78, storage device(s) 79, monitor 76 (e.g., a display screen, such as an LED), which is coupled to display adapter 82, and others are shown. Peripherals and input/output (I/O) devices, which couple to I/O controller 71, can be connected to the computer system by any number of means known in the art such as input/output (I/O) port 77 (e.g., USB, FireWire^{®}). For example, I/O port 77 or external interface 81 (e.g. Ethernet, Wi-Fi, etc.) can be used to connect computer system 8000 to a wide area network such as the Internet, a mouse input device, or a scanner. The interconnection via system bus 75 allows the central processor 73 to communicate with each subsystem and to control the execution of a plurality of instructions from system memory 72 or the storage device(s) 79 (e.g., a fixed disk, such as a hard drive, or optical disk), as well as the exchange of information between subsystems. The system memory 72 and/or the storage device(s) 79 may embody a computer readable medium. Another subsystem is a data collection device 85, such as a camera, microphone, accelerometer, and the like. Any of the data mentioned herein can be output from one component to another component and can be output to the user.

A computer system can include a plurality of the same components or subsystems, e.g., connected together by external interface 81, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software stored in a memory with a generally programmable processor in a modular or integrated manner, and thus a processor can include memory storing software instructions that configure hardware circuitry, as well as an FPGA with configuration instructions or an ASIC. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present disclosure using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk) or Blu-ray disk, flash memory, and the like. The computer readable medium may be any combination of such devices. In addition, the order of operations may be re-arranged. A process can be terminated when its operations are completed, but could have additional steps not included in a figure. A process may correspond to a method, a function, a procedure, a subroutine, a subprogram, etc. When a process corresponds to a function, its termination may correspond to a return of the function to the calling function or the main function

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Any operations performed with a processor (e.g., aligning, determining, comparing, computing, calculating) may be performed in real-time. The term *"real-time"* may refer to computing operations or processes that are completed within a certain time constraint. The time constraint may be 1 minute, 1 hour, 1 day, or 7 days. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

A recitation of "a", "an" or "the" is intended to mean "one or more" unless specifically indicated to the contrary. The use of "or" is intended to mean an "inclusive or," and not an "exclusive or" unless specifically indicated to the contrary. Reference to a "first" component does not necessarily require that a second component be provided. Moreover, reference to a "first" or a "second" component does not limit the referenced component to a particular location unless expressly stated. The term "based on" is intended to mean "based at least in part on."

When a group of substituents is disclosed herein, it is understood that all individual members of those groups and all subgroups and classes that can be formed using the substituents are disclosed separately. When a Markush group or other grouping is used herein, all individual members of the group and all combinations and subcombinations possible of the group are intended to be individually included in the disclosure. As used herein, "and/or" means that one, all, or any combination of items in a list separated by "and/or" are included in the list; for example "1, 2 and/or 3" is equivalent to "'1' or '2' or '3' or '1 and 2' or '1 and 3' or '2 and 3' or '1, 2 and 3‴.

To the extent that the terms "including", "includes", "having", "has", "with", or variants thereof are used in either the detailed description and/or the claims, such terms are intended to be inclusive in a manner similar to the term "comprising." As used herein, "consisting essentially of" does not exclude materials or steps that do not materially affect the basic and novel characteristics of the claim.

Whenever a range is given in the specification, for example, a temperature range, a time range, or a composition range, all intermediate ranges and subranges, as well as all individual values included in the ranges given are intended to be included in the disclosure.

Where a conflict exists between the instant application and a reference provided herein, the instant application shall dominate.

## Claims

1. A computer-implemented method of analyzing a biological sample of a subject, the method comprising:
receiving sequence reads obtained from a methylation-aware sequencing of a plurality of cell-free DNA molecules from the biological sample of the subject, wherein each of the sequence reads includes a methylation pattern of methylation statuses at a set of sites on the sequence read;
for each of the sequence reads:
comparing the methylation pattern to a first reference methylation pattern, wherein the first reference methylation pattern corresponds to a first tissue type of a plurality of tissue types, wherein the plurality of cell-free DNA molecules have a first size range, and wherein an upper bound of the first size range is greater than 600 bases; and
based on the comparison between the methylation pattern to the first reference methylation pattern, determining a tissue classification of the sequence read as being derived from one of the plurality of tissue types; and
determining a classification of a disease in the biological sample based on the tissue classifications of the sequences reads.

2. The method of claim 1, wherein determining the classification of the disease includes:
determining a first amount of sequence reads classified as being derived from the first tissue type; and
determining the classification of the disease in the biological sample based on the first amount.

3. The method of claim 2, wherein determining the classification of the disease in the biological sample based on the first amount includes comparing the first amount to a cutoff value.

4. The method of any one of claims 1 to 3, further comprising:
for each of the sequence reads:
comparing the methylation pattern to one or more other reference methylation patterns corresponding to one or more other tissue types;
determining one or more other amounts of sequence reads classified as being derived from one or more other tissue types; and
determining the classification of the disease in the biological sample further based on the one or more other amounts.

5. The method of any one of claims 1 to 4, wherein the first tissue type is a diseased tissue type or is associated with the disease.

6. The method of any one of claims 1 to 5, wherein the disease is cancer.

7. The method of claim 6, wherein determining the classification of the disease comprises determining whether vascular invasion exists from the cancer.

8. The method of any one of claims 1 to 7, wherein the methylation pattern includes a number of bases between pairs of sites of the set of sites.

9. The method of any one of claims 1 to 8, wherein the classification of the disease identifies a severity of the disease.

10. The method of any one of claims 1 to 9, wherein the first reference methylation pattern includes a plurality of sites of a reference tissue of first tissue type, wherein each of the plurality of sites identifies a methylation index at the site, wherein comparing the methylation pattern to the first reference methylation pattern includes:
for each site of the set of sites of the methylation pattern:
determining a similarity metric between a methylation status of the site and the methylation index of a corresponding site of the first reference methylation pattern; and
determining an aggregate value for the sequence read based on the determined similarity metrics.

11. The method of claim 10, wherein the one of the plurality of tissue types includes the first tissue type, and wherein determining the tissue classification of the sequence read as being derived from one of the plurality of tissue types includes:
determining that the aggregate value determined for the first reference methylation pattern is greater than another aggregate value determined for a second reference methylation pattern, wherein the second reference methylation pattern corresponds to other tissue types of the plurality of tissue types; and
determining the tissue classification of the sequence read as being derived from the first tissue type.

12. The method of claim 10, wherein the one of the plurality of tissue types includes one of other tissue types of the plurality of tissue types, and wherein determining the tissue classification of the sequence read as being derived from one of the plurality of tissue types includes:
determining that the aggregate value determined for the first reference methylation pattern is less than another aggregate value determined for a second reference methylation pattern, wherein the second reference methylation pattern corresponds to one of the other tissue types; and
determining the tissue classification of the sequence read as being derived from one of the other tissue types.

13. The method of any one of claims 1 to 12, wherein a plurality of reference methylation patterns include the first reference methylation pattern, and wherein each of the plurality of reference methylation patterns corresponds to a particular tissue type of the plurality of tissue types, the method further comprising:
for each of the sequence reads:
for each reference methylation pattern of the plurality of reference methylation patterns:
comparing the methylation pattern to the reference methylation pattern; and
based on the comparisons between the methylation pattern to the reference methylation patterns, determining the tissue classification of the sequence read as being derived from one of the plurality of tissue types; and
determining the classification of the disease in the biological sample based on a determination of the highest amount of sequence reads being associated with the tissue classification of the first tissue type.

14. The method of any one of claims 1 to 13, wherein the set of sites of each sequence read include at least three sites.

15. A computer program comprising a plurality of instructions that, when executed. cause a computer system to perform the method of any one of the preceding claims.

## Patentansprüche

1. Computerimplementiertes Verfahren zum Analysieren einer biologischen Probe eines Subjekts, wobei das Verfahren umfasst:
Empfangen von Sequenz-Reads, die aus einer methylierungssensitive Sequenzierung einer Mehrzahl von zellfreien DNA-Molekülen aus der biologischen Probe des Subjekts erhalten wurden, wobei jeder der Sequenz-Reads ein Methylierungsmuster von Methylierungsstatus an einem Satz von Stellen auf dem Sequenz-Read beinhaltet;
für jeden der Sequenz-Reads:
Vergleichen des Methylierungsmusters mit einem ersten Referenzmethylierungsmuster, wobei das erste Referenzmethylierungsmuster einer ersten Gewebeart einer Mehrzahl von Gewebearten entspricht, wobei die Mehrzahl von zellfreien DNA-Molekülen einen ersten Größenbereich aufweist und wobei eine obere Grenze des ersten Größenbereichs größer als 600 Basen ist; und
auf Grundlage des Vergleichs des Methylierungsmusters mit dem ersten Referenzmethylierungsmuster Bestimmen einer Gewebeklassifizierung des Sequenz-Reads als von einer der Mehrzahl von Gewebearten abgeleitet; und
Bestimmen einer Klassifizierung einer Krankheit in der biologischen Probe auf Grundlage der Gewebeklassifizierungen der Sequenz-Reads.

2. Verfahren nach Anspruch 1, wobei das Bestimmen der Klassifizierung der Krankheit beinhaltet:
Bestimmen einer ersten Menge von Sequenz-Reads, die als von der ersten Gewebeart abgeleitet klassifiziert sind; und
Bestimmen der Klassifizierung der Krankheit in der biologischen Probe auf Grundlage der ersten Menge.

3. Verfahren nach Anspruch 2, wobei das Bestimmen der Klassifizierung der Krankheit in der biologischen Probe auf Grundlage der ersten Menge das Vergleichen der ersten Menge mit einem Cut-off-Wert beinhaltet.

4. Verfahren nach einem der Ansprüche 1 bis 3, ferner umfassend:
für jeden der Sequenz-Reads:
Vergleichen des Methylierungsmusters mit einem oder mehreren anderen Referenzmethylierungsmustern, die einer oder mehreren anderen Gewebearten entsprechen;
Bestimmen einer oder mehrerer weiterer Mengen von Sequenz-Reads, die als von einer oder mehreren anderen Gewebearten abgeleitet klassifiziert werden; und
Bestimmen der Klassifizierung der Krankheit in der biologischen Probe ferner auf Grundlage der einen oder der mehreren weiteren Mengen.

5. Verfahren nach einem der Ansprüche 1 bis 4, wobei die erste Gewebeart eine erkrankte Gewebeart ist oder mit der Krankheit assoziiert ist.

6. Verfahren nach einem der Ansprüche 1 bis 5, wobei die Krankheit Krebs ist.

7. Verfahren nach Anspruch 6, wobei das Bestimmen der Klassifizierung der Krankheit das Bestimmen umfasst, ob eine vaskuläre Invasion durch den Krebs vorliegt.

8. Verfahren nach einem der Ansprüche 1 bis 7, wobei das Methylierungsmuster eine Anzahl von Basen zwischen Paaren von Stellen des Satzes von Stellen beinhaltet.

9. Verfahren nach einem der Ansprüche 1 bis 8, wobei die Klassifizierung der Krankheit einen Schweregrad der Krankheit identifiziert.

10. Verfahren nach einem der Ansprüche 1 bis 9, wobei das erste Referenzmethylierungsmuster eine Mehrzahl von Stellen eines Referenzgewebes einer ersten Gewebeart beinhaltet, wobei jede der Mehrzahl von Stellen einen Methylierungsindex an der Stelle identifiziert, wobei das Vergleichen des Methylierungsmusters mit dem ersten Referenzmethylierungsmuster beinhaltet:
für jede Stelle des Satzes von Stellen des Methylierungsmusters:
Bestimmen einer Ähnlichkeitsmetrik zwischen einem Methylierungsstatus der Stelle und dem Methylierungsindex einer entsprechenden Stelle des ersten Referenzmethylierungsmusters; und
Bestimmen eines Aggregatwerts für den Sequenz-Read auf Grundlage der bestimmten Ähnlichkeitsmetriken.

11. Verfahren nach Anspruch 10, wobei eine der Mehrzahl von Gewebearten die erste Gewebeart beinhaltet und wobei das Bestimmen der Gewebeklassifizierung des Sequenz-Reads als von einer der Mehrzahl von Gewebearten abgeleitet beinhaltet:
Bestimmen, dass der für das erste Referenzmethylierungsmuster bestimmte Aggregatwert größer ist als ein anderer Aggregatwert, der für ein zweites Referenzmethylierungsmuster bestimmt wurde, wobei das zweite Referenzmethylierungsmuster anderen Gewebearten der Mehrzahl von Gewebearten entspricht; und
Bestimmen der Gewebeklassifizierung des Sequenz-Reads als von der ersten Gewebeart abgeleitet.

12. Verfahren nach Anspruch 10, wobei die eine der Mehrzahl von Gewebearten eine der anderen Gewebearten der Mehrzahl von Gewebearten beinhaltet und wobei das Bestimmen der Gewebeklassifizierung des Sequenz-Reads als von einer der Mehrzahl von Gewebearten abgeleitet beinhaltet:
Bestimmen, dass der für das erste Referenzmethylierungsmuster bestimmte Aggregatwert kleiner ist als ein anderer Aggregatwert, der für ein zweites Referenzmethylierungsmuster bestimmt wurde, wobei das zweite Referenzmethylierungsmuster einer der anderen Gewebearten entspricht; und
Bestimmen der Gewebeklassifizierung des Sequenz-Reads als von einer der anderen Gewebearten abgeleitet.

13. Verfahren nach einem der Ansprüche 1 bis 12, wobei eine Mehrzahl von Referenzmethylierungsmustern das erste Referenzmethylierungsmuster beinhaltet und wobei jedes der Mehrzahl von Referenzmethylierungsmustern einer bestimmten Gewebeart der Mehrzahl von Gewebearten entspricht, wobei das Verfahren ferner umfasst:
für jeden der Sequenz-Reads:
für jedes Referenzmethylierungsmuster der Mehrzahl von Referenzmethylierungsmustern:
Vergleichen des Methylierungsmusters mit dem Referenzmethylierungsmuster; und
auf Grundlage der Vergleiche des Methylierungsmusters mit dem Referenzmethylierungsmuster Bestimmen der Gewebeklassifizierung des Sequenz-Reads als von einer der Mehrzahl der Gewebearten abgeleitet; und
Bestimmen der Klassifizierung der Krankheit in der biologischen Probe auf Grundlage einer Bestimmung der höchsten Menge an Sequenz-Reads, die mit der Gewebeklassifizierung der ersten Gewebeart assoziiert sind.

14. Verfahren nach einem der Ansprüche 1 bis 13, wobei der Satz von Stellen jedes Sequenz-Reads wenigstens drei Stellen beinhaltet.

15. Computerprogramm, das eine Mehrzahl von Anweisungen umfasst, die bei ihrer Ausführung bewirken, dass ein Computersystem das Verfahren nach einem der vorstehenden Ansprüche durchführt.

## Revendications

1. Procédé mis en œuvre par ordinateur pour analyser un échantillon biologique d'un sujet, le procédé comprenant :
la réception de lectures de séquences obtenues à partir d'un séquençage tenant compte de la méthylation d'une pluralité de molécules d'ADN acellulaire provenant de l'échantillon biologique du sujet, chacune des lectures de séquences comprenant un motif de méthylation des états de méthylation au niveau d'un ensemble de sites sur la lecture de séquence,
pour chacune des lectures de séquences :
la comparaison du motif de méthylation à un premier motif de méthylation de référence, le premier motif de méthylation de référence correspondant à un premier type de tissu parmi une pluralité de types de tissus, la pluralité de molécules d'ADN acellulaire présentant une première plage de tailles, et la limite supérieure de cette première plage de tailles étant supérieure à 600 bases, et
en fonction de la comparaison du motif de méthylation au premier motif de méthylation de référence, la détermination d'un classement tissulaire de la lecture de séquence comme provenant d'un type de tissu parmi la pluralité de types de tissus, et
la détermination d'un classement d'une maladie décelable dans l'échantillon biologique en fonction des classements tissulaires des lectures de séquences.

2. Procédé selon la revendication 1, dans lequel la détermination du classement de la maladie comprend :
la détermination d'une première quantité de lectures de séquences classées comme provenant du premier type de tissu, et
la détermination du classement de la maladie décelable dans l'échantillon biologique en fonction de la première quantité.

3. Procédé selon la revendication 2, dans lequel la détermination du classement de la maladie décelable dans l'échantillon biologique en fonction de la première quantité comprend la comparaison de la première quantité à une valeur seuil.

4. Procédé selon l'une quelconque des revendications 1 à 3, comprenant en outre :
pour chacune des lectures de séquences :
la comparaison du motif de méthylation à un ou plusieurs autres motifs de méthylation de référence correspondant à un ou plusieurs autres types de tissus,
la détermination d'une ou plusieurs autres quantités de lectures de séquences classées comme provenant d'un ou de plusieurs autres types de tissus, et
la détermination du classement de la maladie décelable dans l'échantillon biologique compte tenu, en outre, de la ou des autres valeurs.

5. Procédé selon l'une quelconque des revendications 1 à 4, dans lequel le premier type de tissu est un type de tissu malade ou est associé à la maladie.

6. Procédé selon l'une quelconque des revendications 1 à 5, dans lequel la maladie est un cancer.

7. Procédé selon la revendication 6, dans lequel la détermination du classement de la maladie comprend la détermination de l'existence ou non d'une invasion vasculaire due au cancer.

8. Procédé selon l'une quelconque des revendications 1 à 7, dans lequel le motif de méthylation comprend un nombre de bases entre des paires de sites de l'ensemble de sites.

9. Procédé selon l'une quelconque des revendications 1 à 8, dans lequel le classement de la maladie identifie un degré de gravité de la maladie.

10. Procédé selon l'une quelconque des revendications 1 à 9, dans lequel le premier motif de méthylation de référence comprend une pluralité de sites d'un tissu de référence d'un premier type de tissu, chaque site de la pluralité de sites identifiant un indice de méthylation au site, la comparaison du motif de méthylation au premier motif de méthylation de référence comprenant :
pour chaque site de l'ensemble de sites du motif de méthylation :
la détermination d'une mesure de similarité entre l'état de méthylation du site et l'indice de méthylation d'un site correspondant du premier motif de méthylation de référence, et
la détermination d'une valeur agrégée relative à la lecture de séquence en fonction des mesures de similarité déterminées.

11. Procédé selon la revendication 10, dans lequel le type de tissu parmi la pluralité de types de tissus comprend le premier type de tissu, et la détermination du classement tissulaire de la lecture de séquence comme provenant d'un type de tissu parmi la pluralité de types de tissus comprend :
la détermination que la valeur agrégée déterminée relativement au premier motif de méthylation de référence est supérieure à une autre valeur agrégée déterminée pour un deuxième motif de méthylation de référence, le deuxième motif de méthylation de référence correspondant à d'autres types de tissus parmi la pluralité de types de tissus, et
la détermination du classement tissulaire de la lecture de séquence comme provenant du premier type de tissu.

12. Procédé selon la revendication 10, dans lequel ledit type de tissu parmi la pluralité de types de tissus comprend un tissu parmi d'autres types de tissus de la pluralité de types de tissus, et la détermination du classement tissulaire de la lecture de séquence comme provenant d'un type de tissu parmi la pluralité de types de tissus comprenant :
la détermination que la valeur agrégée déterminée relativement au premier motif de méthylation de référence est inférieure à une autre valeur agrégée déterminée relativement à un deuxième motif de méthylation de référence, le deuxième motif de méthylation de référence correspondant à un des autres types de tissus, et
la détermination du classement tissulaire de la lecture de séquence comme provenant d'un des autres types de tissus.

13. Procédé selon l'une quelconque des revendications 1 à 12, dans lequel une pluralité de motifs de méthylation de référence comprend le premier motif de méthylation de référence, et chaque motif de la pluralité de motifs de méthylation de référence correspond à un type de tissu particulier parmi la pluralité de types de tissus, le procédé comprenant en outre :
pour chacune des lectures de séquences :
pour chaque motif de méthylation de référence parmi la pluralité de motifs de méthylation de référence :
la comparaison du motif de méthylation au motif de méthylation de référence, et
en fonction des comparaisons du motif de méthylation aux motifs de méthylation de référence, la détermination du classement tissulaire de la lecture de séquence comme provenant d'un type de tissu parmi la pluralité de types de tissus, et
la détermination du classement de la maladie décelable dans l'échantillon biologique en fonction d'une détermination de la quantité la plus élevée de lectures de séquences associées au classement tissulaire du premier type de tissu.

14. Procédé selon l'une quelconque des revendications 1 à 13, dans lequel l'ensemble de sites de chaque lecture de séquence comprend au moins trois sites.

15. Programme informatique comprenant une pluralité d'instructions qui, lorsqu'elles sont exécutées, amènent un système informatique à réaliser le procédé selon l'une quelconque des revendications précédentes.
